(19) Europäisches Patentamt
European Patent Office
Office européen des brevets

(11) **EP 4 041 908 B1**

(12) **EUROPEAN PATENT SPECIFICATION**

(45) Date of publication and mention
of the grant of the patent:
**20.09.2023 Bulletin 2023/38**

(21) Application number: **20793076.9**

(22) Date of filing: **08.10.2020**

(51) International Patent Classification (IPC):
*C12Q 1/18* [(2006.01)]     *G01N 33/543* [(2006.01)]
*G01N 33/569* [(2006.01)]

(52) Cooperative Patent Classification (CPC):
**G01N 33/56911; C12Q 1/18; G01N 33/54313;
G01N 33/56961**

(86) International application number:
**PCT/GB2020/052498**

(87) International publication number:
**WO 2021/069903 (15.04.2021 Gazette 2021/15)**

(54) **MICROORGANISM CAPTURE FROM ANTIMICROBIAL-CONTAINING SOLUTION**

EINFANGEN VON MIKROORGANISMEN AUS ANTIMIKROBIELLER LÖSUNG

CAPTURE DE MICRO-ORGANISME À PARTIR D'UNE SOLUTION CONTENANT UN AGENT ANTIMICROBIEN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2019 GB 201914538**

(43) Date of publication of application:
**17.08.2022 Bulletin 2022/33**

(73) Proprietor: **Momentum Bioscience Limited
Cardiff, Wales CF3 0EF (GB)**

(72) Inventors:
• **BENNETT, Helen Victoria
Cardiff CF3 0EF (GB)**
• **JAY, Paul
Cardiff CF3 0EF (GB)**
• **LOCKHART, Daniel
Cardiff CF3 0EF (GB)**
• **MULLEN, William
Cardiff CF3 0EF (GB)**

(74) Representative: **Boult Wade Tennant LLP
Salisbury Square House
8 Salisbury Square
London EC4Y 8AP (GB)**

(56) References cited:
**EP-A2- 1 118 676     WO-A1-2018/044966**

• **MARK CARTWRIGHT ET AL: "A Broad-Spectrum Infection Diagnostic that Detects Pathogen-Associated Molecular Patterns (PAMPs) in Whole Blood", EBIOMEDICINE, vol. 9, 1 July 2016 (2016-07-01), pages 217-227, XP55751793, ISSN: 2352-3964, DOI: 10.1016/j.ebiom.2016.06.014**
• **D. FLAYHART ET AL: "Comparison of BACTEC PLUS Blood Culture Media to BacT/Alert FA Blood Culture Media for Detection of Bacterial Pathogens in Samples Containing Therapeutic Levels of Antibiotics", JOURNAL OF CLINICAL MICROBIOLOGY, vol. 45, no. 3, 13 December 2006 (2006-12-13), pages 816-821, XP055115240, ISSN: 0095-1137, DOI: 10.1128/JCM.02064-06**

Remarks:
The complete document including Reference Table(s) and the Sequence Listing(s) can be downloaded from the EPO website

EP 4 041 908 B1

**Description**

**FIELD OF THE INVENTION**

The present invention relates generally to the field of recovering microorganisms from samples containing an antimicrobial agent. The methods of the invention therefore enable determination of the absence and presence of microbial pathogens in clinical samples such as un-purified blood, blood culture and other body fluids. Applications of the present invention include determination of the presence or absence of viable microorganisms, determination of their Gram status, species, and antimicrobial susceptibility/resistance. The present invention may also be used in monitoring the effectiveness of antimicrobial treatment of a patient.

**BACKGROUND TO THE INVENTION**

The present inventors have recognised that in samples taken from subjects suspected of carrying a microbial infection there are much greater levels of nucleated blood cells (leukocytes) than previously imagined even though the majority of samples are not in fact from infected subjects. This has led to the requirement for improved methods of separating potential microbes from blood cells, in particular leukocytes, in blood samples taken from patients screened for infection.

When blood samples are taken from patients that have already been given an antimicrobial agent the subsequent blood cultures often do not grow microorganisms, even when the patient clearly has an infection (Scerbo et al., Surg Infect (Larchmt). 2016 Jun;17(3):294-302; Sinha et al., Clin Microbiol Rev. 2018 Feb 28;31 (2), e00089-17; Scheer et al., Clin Microbiol Infect. 2019 Mar;25(3):326-331).

In order to address this problem, blood culture suppliers have provided blood culture bottles containing antibiotic-absorbing resin (e.g. BACTEC PLUS bottles from Becton Dickinson and BacT/Alert bottles from bioMerieux). Flayhart et al. (J. Clin. Microbiol. (2007), 816-821) describes testing of BACTEC PLUS and BacT/Alert bottles under simulated conditions. In the presence of ceftriaxone, neither system was able to recover *Streptococcus pneumoniae.*

Chung et al. (Eur J Clin Microbiol Infect Dis. (2019), 38(12):2229-2233) also describes testing BacT/Alert bottles and BACTEC Plus bottles under simulated conditions. Both systems exhibited low to zero detection rates in the presence of certain antimicrobial agents. For example, *E. coli* or *K. pneumoniea* in the presence of cefepime; *E. coli* in the presence of cefotaxmine; and *E. coli, K. pneumoniea or P.aeruginosa in the presence of meropenem.* Thus, neither system can universally recover microorganisms from a sample containing an antimicrobial agent.

In WO2009/007719, NAD- dependent ligases are described as a useful indicator of the presence of a micro-organism in a sample. Ligases are enzymes which catalyze ligation of nucleic acid molecules. The ligation reaction requires either ATP or NAD+ as co-factor depending upon the ligase concerned.

WO2011/130584 describes a method for detection of viable microorganisms based on detection of DNA or RNA polymerases in which a sample is contacted with a nucleic acid substrate that acts as a substrate for microbial polymerase, incubated under conditions suitable for polymerase activity from intact microorganisms and any resulting nucleic acid product is determined using a nucleic acid amplification technique such as quantitative polymerase chain reaction. Such assays have been termed "ETGA assays", where ETGA stands for Enzymatic Template Generation and Amplification. A problem with ETGA assays for viable microorganisms in crude samples is the presence of contaminating polymerase activity outside the microorganisms arising from host (e.g. human) cells and dead microorganisms. The ETGA assay is unable to distinguish microorganism polymerase activity from that of the host or from dead microorganisms.

WO2010/119270 describes a method for removing DNA ligase activity outside intact microorganisms. WO2011/070507 describes the selective lysis of animal cells using a non-ionic detergent and a buffer. WO2017/182775 describes a method of detecting the absence or presence of a microorganism in a sample that may also contain non-microorganism cells comprising the selective lysis of non-microorganism cells, filtering the lysate and detecting the absence or presence of microorganisms retained within or upon the filter.

The use of magnetic beads coated with specific binding moieties such as antibodies is known for capture of targeted species. The specificity of these products is defined by the specificity of the antibody or other binding ligand, which is generally chosen for a particular purpose to be highly specific to allow the isolation of a particular microorganism.

WO03/102184 describes methods, compositions and kits for concentrating or separating cells (e.g. bacteria) using flocculating agents, such as polyamines or cationic detergents, to form complexes with cells causing them to aggregate. The separation of the aggregated cells can be effected with a solid phase which is capable of binding the cells, such as magnetic beads.

WO01/53525 describes a method of isolating cells (e.g. microorganisms) from a sample which method comprises binding the cells to a solid support by means of a carbohydrate ligand immobilised on the solid support. A kit for performing such a method is sold by DiaSorin Molecular ("Bugs'n Beads™" kit).

Other kits for isolating microorganisms include ApoH-Technologies Peps6 magnetic beads. The beads are coated with the synthetic molecule, Peps6, which is derived from the Apolipoprotein H protein (ApoH), also known as β-2 glycoprotein.

Cartwright et al. (EBioMedicine (2016) 9:217-227) describes capturing Pathogen-Associated Molecular Patterns (PAMPs) using magnetic beads coated with Mannose Binding Lectin. PAMPs are, according to Cartwright, carbohydrate cell wall materials and outer membrane vesicles that are released by various types of live and dead pathogens. Cartwright states that the release of PAMPs by pathogens is increased when they are killed by antibiotics. The Cartwright assay does not aim to recover viable microorganisms from a sample.

WO2018/044966, EP1118676, CN109929763, CN109741896 describe using beads to capture microorganisms from samples which do not contain an antimicrobial agent.

## DESCRIPTION OF THE INVENTION

The invention relates to the recovery of viable microorganisms from a sample comprising microorganism cells and an antimicrobial agent by capturing microorganisms with coated particles to form particle-microorganism complexes and separating the particle-microorganism complexes from the antimicrobial agent. The inventors have discovered that coated particles are capable of universal binding to microorganisms and thus can recover a range of types of microorganisms, such as bacteria and fungi, without knowing which type is present in the sample a *priori*. Pan-microorganism recovery according to the invention is effective to remove the growth-inhibiting effect of the antimicrobial agent. This provides an alternative and, as demonstrated in comparative experiments reported herein, more effective way to address the issue currently addressed in clinical practice by using blood culture bottles containing antibiotic-absorbing resin. As demonstrated herein, the recovered microorganisms remain viable and rapidly recover to demonstrate growth. The inventors have surprisingly found that this recovery can be achieved with coated particles lacking complex moieties, such as antibodies, which bind to (specific) microorganisms in a targeted manner.

In addition to rescuing the microorganisms from an antimicrobial agent, the present invention also allows microorganisms to be rescued from inhibitors of later analysis such as blood cell remnants, haemoglobin and leukocyte DNA which can severely affect techniques such as molecular detection and identification. Removal of platelets can also be useful in some techniques for determining antimicrobial susceptibility.

The present invention also results in significant concentration of the microorganisms (for example, the microorganisms in a 5 mL blood sample can be concentrated into tens of $\mu$L), particularly when compared with blood culture bottles containing antibiotic-absorbing resin (e.g. BACTEC PLUS bottles from Becton Dickinson and BacT/Alert bottles from bioMerieux). This is advantageous for downstream applications which require a higher concentration of microorganisms. Various tests, including molecular tests, may be performed to detect and/or characterise recovered viable microorganisms and a more concentrated sample is generally required to perform, or is advantageous in the context of, such assays. Suitable assays are discussed herein, including EGTA assays, PCR or sequencing assays and antimicrobial susceptibility testing (AST), particularly multi-well AST which is performed in relatively low volumes. Providing a suitably concentrated sample initially improves processing times as a further concentration step may be avoided in downstream testing of the microorganisms.

## METHODS

The invention provides a method of recovering viable microorganisms from a sample comprising microorganism cells and an antimicrobial agent, the method comprising: a) incubating the sample with coated particles to form particle-microorganism complexes; and b) separating the particle-microorganism complexes from the antimicrobial agent; thereby recovering viable microorganisms from the sample; and c) incubating and/or culturing the viable microorganisms recovered in step b), wherein the sample is from a subject who has been treated with the antimicrobial agent, wherein the microorganisms in the sample are or are thought to be susceptible to the antimicrobial agent and wherein the sample comprises a bodily fluid.

The term "viable microorganisms" refers to microorganisms which are not dead and applies to all aspects of the disclosure. Thus, they are microorganisms that have not been killed by the antimicrobial agent. Viable microorganisms may be capable of metabolic recovery from exposure to the antimicrobial agent and/or growth under suitable conditions (once recovered from the sample containing the antimicrobial agent, as described herein). By "growth" is meant both an increase in volume/size and the ability to proliferate, in particular the ability to proliferate (through synthesis, DNA replication and cell division).

In recovering viable microorganisms from the sample, the concentration of antimicrobial agent to which the recovered viable microorganisms are exposed may be substantially reduced. In recovering viable microorganisms from the sample, the concentration of viable microorganisms may be increased relative to the sample. The increased concentration of viable microorganisms may assist with downstream processes such as characterisation of the viable microorganisms.

The step of "incubating the sample" refers to contacting the sample with the coated particles under conditions conducive to the formation of particle-microorganism complexes. In some embodiments, the step of incubating the sample with coated particles comprises contacting the coated particles with the sample for a fixed period of time (e.g. 30 minutes) at a specified temperature (e.g. 37 °C). The incubation may be performed with or without shaking (e.g. by a platform shaker, orbital shaker or shaking incubator set at 500-1000 rpm). The incubation may be performed in the absence of a fixative agent. A fixative agent may be a cross-linking or non-cross-linking fixative agent. Cross-linking fixatives function by making chemical bonds between with the microorganisms in the sample. Non-cross-linking fixatives do not chemically alter the microorganisms in the sample; rather they simply precipitate them.

The method of the invention comprises incubating and/or culturing the recovered viable microorganisms. The viable microorganisms may be incubated and/or cultured whilst they remain attached to the coated particles (i.e. in complex).

According to the methods of the invention, culturing the recovered viable microorganisms may comprise increasing the number of viable microorganisms. In the methods, incubating the recovered viable microorganisms may be considered a distinct phase and thus may not comprise increasing the number of viable microorganisms. Microorganisms exposed to antimicrobial agent may not be in position to grow for a period following recovery. Thus, incubating the recovered viable microorganisms may comprise allowing the organisms to undergo metabolic recovery following exposure to antimicrobial agent. For some downstream characterisation methods, it is not necessary for the microorganisms to grow; however metabolic recovery may be important. Thus, some methods may not involve a culture step following recovery. However, following metabolic recovery, the viable microorganisms may enter a growth phase. Accordingly, the methods of the invention may comprise both incubation and culture, to enable metabolic recovery followed by growth.

Accordingly, the invention provides a method of incubating and/or culturing viable microorganisms recovered from a sample comprising microorganism cells and an antimicrobial agent, the method comprising: a) incubating the sample with coated particles to form particle-microorganism complexes; b) separating the particle-microorganism complexes from the antimicrobial agent, thereby recovering viable microorganisms from the sample; and c) incubating and/or culturing the recovered viable microorganisms, wherein the sample is from a subject who has been treated with the antimicrobial agent, wherein the microorganisms in the sample are or are thought to be susceptible to the antimicrobial agent and wherein the sample comprises a bodily fluid.

The methods of the invention may further comprise detecting and/or characterising the recovered viable microorganisms. "Detecting" refers to determining, or confirming, by any suitable means, whether there were indeed viable microorganisms in the sample that have been recovered. "Characterising" goes further than detecting the presence or absence of micoorganisms and provides additional information about the recovered viable microorganisms. Characterising may comprise determining whether the sample contains bacteria and/or fungi. This is particularly relevant in clinical samples, especially those taken from subjects suspected of having an infection (e.g. patients with potential sepsis). Characterising may additionally (which may be sequentially, i.e. first determine whether there is a microorganism (such as bacteria or fungi) present and, if so, perform an additional characterising step) or alternatively comprise identifying the genus or species of microorganism recovered from the sample. Characterising may comprise determining the Gram status of the microorganism (i.e. whether the bacterium is gram negative or gram positive). These types of characterisation may be particularly useful for determining which type of antimicrobial agent is most suitable for the subject from whom the sample was taken. Characterising may comprise determining antimicrobial susceptibility and/or resistance of the microorganisms. Any suitable method may be employed for these further steps; examples of such methods are discussed further herein.

Thus, the invention provides a method of detecting the absence or presence of a viable microorganism in a sample comprising an antimicrobial agent and suspected of containing a microorganism, the method comprising: a) incubating the sample with coated particles to form particle-microorganism complexes (if the microorganism is present in the sample; if it is not present the complexes cannot form); b) separating particle-microorganism complexes from the antimicrobial agent, thereby recovering viable microorganisms from the sample; and c) incubating and/or culturing the viable microorganisms ; and d) detecting the absence or presence of viable microorganisms, wherein the sample is from a subject who has been treated with the antimicrobial agent, wherein the microorganisms in the sample are or are thought to be susceptible to the antimicrobial agent and wherein the sample comprises a bodily fluid.

Similarly, the invention provides a method of detecting the absence or presence of a viable microorganism in a sample comprising an antimicrobial agent and suspected of containing a microorganism, the method comprising: a) incubating the sample with coated particles to form particle-microorganism complexes (if the microorganism is present in the sample; if it is not present the complexes cannot form); b) separating particle-microorganism complexes from the antimicrobial agent, thereby recovering viable microorganisms from the sample; c) incubating and/or culturing the recovered viable microorganisms; and d) detecting the absence or presence of viable microorganisms, wherein the sample is from a subject who has been treated with the antimicrobial agent, wherein the microorganisms in the sample are or are thought to be susceptible to the antimicrobial agent and wherein the sample comprises a bodily fluid.

The subject has been treated with an antimicrobial agent. This is then the source of antimicrobial agent in the sample. The sample is typically a clinical sample as discussed herein. The subject is typically a human subject, often a human subject suspected of suffering from an infection (which may be a bacterial or fungal infection).

In these methods, the method may further comprise characterising the microorganism responsible for the infection. Any suitable method of characterisation may be employed, as discussed herein (which discussion applies *mutatis mutandis*).

As already mentioned, the present invention is particularly applicable to clinical samples. Such samples typically contain non-microorganism cells. Indeed, they may contain predominantly non-microorganism cells (i.e. there are fewer, typically significantly fewer, microorganism cells than non-microorganism cells in the sample). Thus, the finding of the present inventors that coated particles can remove microorganisms from samples containing both an antimicrobial agent and non-microorganism cells is particularly advantageous in the present invention.

The invention therefore provides a method of recovering viable microorganisms from a sample comprising microorganism cells, non-microorganism cells and an antimicrobial agent, the method comprising: a) incubating the sample with coated particles to form particle-microorganism complexes; and b) separating the particle-microorganism complexes from the antimicrobial agent and the non-microorganism cells; thereby recovering viable microorganisms from the sample, c) incubating and/or culturing the recovered viable microorganisms, wherein the sample is from a subject who has been treated with the antimicrobial agent, wherein the microorganisms in the sample are or are thought to be susceptible to the antimicrobial agent and wherein the sample comprises a bodily fluid.

In these methods, the sample comprises non-microorganism cells and step b) separates the particle-microorganism complexes from the antimicrobial agent and the non-microorganism cells. The non-microorganism cells may comprise blood cells. The blood cells may comprise red (erythrocytes) and/or white blood cells (leukocytes). For the avoidance of doubt, the non-microorganism cells may be lysed before separation takes place. Thus, separation of particle-microorganism complexes from non-microorganism cells encompasses separating particle-microorganism complexes from non-microorganism cell lysates. However, separation may also be performed when the non-microorganism cells remain intact.

Exposure of microorganisms to an antimicrobial agent is intended to kill or prevent growth of the microorganisms. This presents a challenge when attempting to recover viable microorganisms. Thus, the present methods may rely upon conditions intended to preserve the viable microorganisms that remain in the sample following exposure to an antimicrobial agent. Those microorganisms may be more susceptible to cell death and lysis. Thus, certain agents that might be useful to preferentially lyse non-microorganism cells over microorganisms in a sample may not be suitable in certain embodiments of the invention. Such agents include detergents, in particular at higher concentrations. Thus, the methods of the invention (step b)) are may be performed in the absence of a detergent.

In some embodiments, step b) may be performed in the presence of sodium polyanethol sulfonate. As shown herein, this reagent is useful for recovery of microorganisms from samples containing non-microorganism cells.

In further embodiments, step b) may additionally or alternatively be performed in the presence of a reagent (such as a detergent) that selectively lyses non-microorganism cells in the sample whilst retaining intact microorganisms present in the sample. Suitable reagents are known in the art and discussed further herein.

In view of the increased sensitivity of the antimicrobial agent-exposed microorganisms to lysis, in some embodiments no lysis of non-microorganism cells is performed to facilitate separation of the microorganisms from the non-microorganisms in the sample. Because the coated particles preferentially bind microorganisms there is no absolute requirement to lyse non-microorganisms in the sample. However, it may be advantageous to perform one or more washing steps to remove non-microorganism cells (typically intact non-microorganism cells) and/or cell lysate from the particle-microorganism complexes. Thus, step b) of the methods of the invention may comprise washing the separated particle-microorganism complexes. Any suitable wash solution may be employed, examples are discussed herein. In some embodiments, the separated particle-microorganism complexes are washed with a solution that does not contain detergent. In some embodiments, the wash buffer comprises Tris and/or sodium chloride. In some embodiments, the wash buffer has a pH from about 7 to 9, such as from 7.5 to 8.5. In some embodiments, the wash buffer contains a salt such as a metal halide salt. A preferred example is sodium chloride. The salt concentration may be from about 50 to 150 mM. Preferably, the wash buffer comprises phosphate buffered saline (PBS), Tris or Tricine.

In other embodiments, the separated particle-microorganism complexes may be washed with a solution containing detergent. The solution may be a weak detergent solution to reduce the risk of lysing microorganisms. The solution may comprise detergent at a concentration that does not substantially lyse the microorganisms. Suitable examples include polyethylene glycol sorbitan monolaurate (Tween 20), for example at 1 to 5% w/v, preferably around 1%. The reagent may include a saponin, for example at 1 to 5% w/v, preferably around 1%. The microorganisms may be exposed to the detergent solution for a period of time that does not substantially lyse the microorganisms. The solution may selectively lyse at least a proportion of non-microorganisms in the sample.

The reagent that selectively lyses non-microorganism cells in the sample whilst retaining intact microorganisms present in the sample may comprise one or more enzymes. The one or more enzymes may comprise a proteinase and/or a DNAse. Suitable enzymes are discussed herein.

However, as already mentioned, when dealing with microorganisms which may be fragile following exposure to an antimicrobial agent, it is preferable that step a) and any washes performed in step b) are performed in the absence of a detergent. The entire methods may be performed in the absence of detergent in some embodiments. In other embodiments, the methods may be performed in the absence of detergent except for detecting and/or characterising the recovered viable microorganisms. In this embodiment, the contents of the microorganism cells may need to be released for analysis. A suitable lysis reagent may comprise detergent. Such detergent may be at high concentration to ensure complete lysis of the microorganisms.

In the methods, step b) typically involves some form of physical separation of the particle-microorganism complexes from the remainder of the sample. This may comprise use of any suitable means of separation. For example, separation may be achieved using a magnetic field. Use of a magnetic field requires that the particles are magnetic particles and attracts the particle-microorganism complexes. Alternatively, centrifugation or other separation methods such as filtration may be employed. Step (b) may comprise or further comprise removing the non-microorganism cells from the particle-microorganism complexes by aspiration.

For the avoidance of doubt, washing steps may follow the physical separation step or steps. Thus, the method may further comprise (as part of step b)) washing the separated particle-microorganism complexes to remove antimicrobial agent and/or non-microorganism cells or lysate from the particle-microorganism complexes.

More generally, step b) may further comprise removing the non-microorganism cells from the particle-microorganism complexes.

Where selective lysis is employed, in order to remove non-microorganism cells from the sample (and retain viable microorganisms), it may be performed at any suitable stage of the methods. It may thus be performed as an initial processing step, before the sample is incubated with the coated particles. Accordingly, in some embodiments, step a) may be preceded by selectively lysing non-microorganism cells in the sample whilst retaining intact microorganisms present in the sample.

Selectively lysing non-microorganism cells in the sample whilst retaining intact any microorganisms present in the sample may comprise osmotic lysis or adding a detergent. The detergent may be a weak detergent that causes lysis of non-microorganism cells (such as blood cells) does not substantially lyse the microorganisms. Suitable examples include polyethylene glycol sorbitan monolaurate (Tween 20), for example at 1 to 5% w/v, preferably around 1%. The reagent may include a saponin, for example at 1 to 5% w/v, preferably around 1%.

In the methods, step a) is typically performed in aqueous solution. Step a) may be performed in the presence of a buffer. The buffer may comprise any suitable buffering agent including, but not limited to any one or more of phosphate buffered saline (PBS), TAPS ([Tris(hydroxymethyl)methylamino]propanesulfonic acid), Bicine (2-(Bis(2-hydroxyethyl)amino)acetic acid), Tris (Tris(hydroxymethyl)aminomethane), Tricine (3-[N-Tris(hydroxymethyl)methylamino]-2-hydroxypropanesulfonic acid), TAPSO (3-[N-Tris(hydroxymethyl)methylamino]-2-hydroxypropanesulfonic acid), HEPES (4-(2-hydroxyethyl)-1-piperazineethanesulfonic acid), TES (2-[[1,3-dihydroxy-2-(hydroxymethyl)propan-2-yl]amino]ethanesulfonic acid), MOPS (3-(N-morpholino)propanesulfonic acid), PIPES (Piperazine-N,N'-bis(2-ethanesulfonic acid)), Cacodylate (Dimethylarsenic acid), MES (2-(N-morpholino)ethanesulfonic acid), Bis-tris (2-[Bis(2-hydroxyethyl)amino]-2-(hydroxymethyl)propane-1,3-diol), maleate, phosphate, glycine, citrate, glycylglycine, formate, succinate, acetate, propionate, piperazine, histidine, ethanolamine, imidazole, borate, carbonate. The buffer may have a pH between 7.4 and 8.5.

Preferably, the buffer comprises phosphate buffered saline (PBS), Tris or Tricine. In the methods, step a) may be performed in the presence of sodium chloride. The sodium chloride may be present at a concentration of between 50 and 500 mM. Preferably, sodium chloride may be present at a concentration around 150 mM.

In the methods, the reagent that selectively lyses non-microorganism cells in the sample whilst retaining intact microorganisms present in the sample may be a detergent, in particular a weak detergent. In the methods, the detergent may be non-ionic. In the methods, the detergent may not be conjugated to the particles capable of forming complexes with microorganisms. Thus, typically the detergent forms part of a solution to which the particles are added and does not form part of the particles themselves.

As already introduced, the methods of the invention may involve detecting and/or characterising the recovered viable microorganisms. In some embodiments, detecting the absence or presence of a microorganism may comprise (i) detecting an enzymatic activity of the microorganism, (ii) detecting a nucleic acid or polypeptide from the microorganism (iii) detecting the microorganism directly by cytometry or microscopy, or (iv) detecting the microorganism following cell culture.

The detection of the absence or presence of microorganisms in the particle-microorganism complexes according to all relevant aspects of the invention can be performed according to any desired method. The method may involve detecting the simple absence or presence of the one or more microorganisms. It may involve quantification of the microorganisms, if present. It may also involve characterisation of the nature of the microorganism in some embodiments. Thus, detection of bacteria and/or fungi may be performed. Discrimination of Gram positive versus Gram negative bacteria may also be performed. Identification of species and antimicrobial susceptibility and/or resistance of the organisms may also be performed.

Detection and/or characterisation may occur after the removal (or recovery) of the microorganisms from the particle-microorganism complexes. Recovered microorganisms may be lysed prior to detection. Recovery may be of the intact microorganisms or of a lysate following lysis of the microorganisms (as discussed in further detail herein).

Preferably, detection occurs without prior removal (or recovery) of the microorganisms from the particle-microorganism complexes. This embodiment is particularly useful in applying the invention to magnetic bead-processing instrumentation (i.e. where the particles are magnetic, which represents a particularly useful implementation of the methods of the invention).

Detection of nucleic acid molecules associated with microorganisms is known in the art and may be performed at the DNA or RNA level. It can be performed by any suitable method, such as amplification (e.g. PCR) or sequencing (in particular next generation sequencing). Such methods may take advantage of sequence divergence between microorganisms and non-microorganisms, such as human, DNA and RNA. Such methods may involve lysing the microorganisms (e.g. present in the form of particle-microorganism complexes) in order to release the nucleic acid component.

Direct detection of microorganisms is also known. This may involve cytometric analysis, for example by flow cytometry. It may involve use of microscopy, for example to visualise the microorganisms recovered from particle-microorganism complexes or to visualise microorganisms in particle-microorganism complexes.

Microorganism detection may also be performed following cell culture, in order to expand the number of microorganisms. Thus, the microorganisms initially captured within particle-microorganism complexes can be cultured for a set period of time, prior to detection. Culture methods may permit direct detection of microorganisms in the original sample. Alternatively, the microorganisms may be incubated for a shorter period (than when cultured), to allow metabolic recovery from exposure to the antimicrobial agent, prior to detection (but without expansion).

However, in preferred embodiments, the detection of the absence or presence of recovered microorganisms may comprise detecting an enzymatic activity associated with the microorganism. Suitable enzymatic activities are typically nucleic acid modifying activities and are discussed in greater detail herein.

Accordingly, in the methods, the step of detecting the microorganisms may comprise the steps of: (i) lysing the microorganisms in the particle-microorganism complexes; (ii) incubating the lysate with a nucleic acid molecule which acts as a substrate for nucleic acid modifying activity of the microorganisms; and (iii) specifically determining the absence or presence of a modified nucleic acid molecule resulting from the action of the nucleic acid modifying enzyme on the substrate nucleic acid molecule to indicate the absence or presence of the microorganism. Suitable substrates are discussed in further detail herein. By "substrate" is meant a nucleic acid molecule which is acted on by the microorganism-derived enzyme. Typically, such nucleic acid molecules are oligonucleotide substrates. They are synthetic nucleic acid molecules.

Lysis of microorganisms in the particle-microorganism complexes permits detection of nucleic acid molecules or enzymes within the microorganisms, such as nucleic acid modifying enzymes. Lysis may be achieved by addition of a lysis mixture. The lysis mixture is generally useful in the methods of the invention. The lysis mixture may include a specific mixture of components to ensure efficient lysis of microorganisms without adversely affecting nucleic acid molecules and/or enzyme activity, such as nucleic acid modifying activity, within the cells. The components may be selected from carrier/serum proteins such as BSA, surfactants/detergents, metal halide salts, buffers, chelators etc. In its basic form, the lysis mixture of the invention may include the following components:

1. A surfactant/detergent
2. Serum protein such as albumin (e.g. BSA)
3. Buffer
4. Nucleotides, such as dNTPs
5. Nucleic acid molecule (acting as a substrate in the assays of the invention).

A suitable lysis mixture is set forth below:

L1: 252 mL in 360 mL LM

    1.46% (w/v) BSA
    0.15% Triton X100
    0.15% Tween 20

L2: 36 mL in 360 mL LM

    100 mM Ammonium sulphate

20 mM Magnesium sulphate heptahydrate
100 mM Potassium chloride
200 mM Tris-HCl [pH 8.0]

L3: 36 mL in 360 mL LM

0.1 μM PTO-AS oligo
0.1 μM PTO-S1 oligo
20 mM Tris-HCl [pH 8.5]
10 mM Potassium chloride
10 μM EDTA
10 mM dNTPs: 3.6 mL in 360 mL LM
PTO-IPC stock: ~180 μL in 360 mL LM
H2O: ~32.4 mL in 360 mL LM

By "PTO-AS oligo" is meant an antisense oligonucleotide comprising phosphorothioate nucleotides. By "PTO-S1 oligo" is meant a sense oligonucleotide comprising phosphorothioate nucleotides. The two oligonucleotides hybridise to one another to form the substrate nucleic acid molecule.

By "PTO-IPC" is meant an IPC molecule comprising phosphorothioate nucleotides.

Suitable substrate and IPC molecules are discussed in further detail herein.

Exemplary amounts and concentrations of each component are listed but may be modified as would be readily appreciated by one skilled in the art.

Lysis may also require disruption of the cells. For example, the cells may be disrupted using the lysis mixture in combination with physical and/or enzymatic means. Typically, however, the methods in which the cells are lysed avoid use of physical disruption. In some embodiments, physical disruption employs a disruptor. The disruptor may incorporate beads such as glass beads to lyse the cells. Suitable apparatus are commercially available and include the Disruptor Genie manufactured by Scientific Industries, Inc. Sonication may be utilised, for example applying an ultra sonic horn. Enzymatic disruption may require use of one or more agents selected from lysostaphin, lysozyme and/or lyticase in some embodiments.

Once the microorganisms, if present in the sample, are lysed, the released nucleic acid and/or enzymes may be detected to indicate whether viable microorganisms were present in (and recovered from) the sample. In some embodiments, the lysate is incubated with a nucleic acid molecule which acts as a substrate for nucleic acid modifying activity (of the microorganisms). The absence or presence of a modified nucleic acid molecule resulting from the action of the nucleic acid modifying enzyme on the substrate nucleic acid molecule is then determined to indicate the absence or presence of the microorganism. The nucleic acid substrate molecule is designed according to the nucleic acid modifying activity that is to be detected. One skilled in the art is well able to design suitable substrate nucleic acid molecules. Although the initial sample may contain non-microorganism sources of nucleic acid modifying activity, the methods of the invention prevent this contaminating activity acting on the substrate nucleic acid molecules.

According to the methods of the invention typical nucleic acid modifying activity that may be detected comprises polymerase and/or ligase activity. The nucleic acid modifying enzyme may comprise a DNA or RNA polymerase. In preferred embodiments, the DNA polymerase is a DNA-dependent DNA polymerase. In some embodiments, the DNA polymerase is DNA polymerase I. The nucleic acid modifying enzyme may comprise a ligase, optionally wherein the nucleic acid modifying enzyme is an NAD-dependent ligase (to indicate the presence of bacteria) and/or an ATP-dependent ligase (to indicate the presence of fungi or bacteria). NAD-dependent ligases are only found in (eu)bacteria and thus, detecting such activity may provide an additional level of specificity. This is discussed further in WO2009/007719 and WO2010/119270. Other nucleic acid modifying activities relevant to viability may alternatively be measured such as phosphatase, kinase and/or nuclease activity.

As already discussed, the methods may comprise washing the separated particle-microorganism complexes to remove non-microorganism cells or lysate. The step of washing may remove inhibitors of the subsequent analysis e.g. PCR inhibitors. The step of washing are typically performed under conditions that do not dissociate the particle-microorganism complexes.

In some embodiments, the action of the nucleic acid modifying activity on the "substrate" nucleic acid molecule produces an extended nucleic acid molecule. This may be by strand extension (polymerase activity) and/or by ligation of two nucleic acid molecules (ligase activity). In some embodiments, a substrate that can be acted upon by either polymerase or ligase is utilised since either activity is indicative of the presence of a microorganism in the sample. In some embodiments, the relevant activity can be distinguished in terms of the novel nucleic acid molecule that is produced.

The substrate may be a template for the nucleic acid modifying activity of the microorganisms. For example, the substrate may be a template for a DNA or RNA polymerase, preferably a DNA-dependent DNA polymerase, optionally wherein the DNA polymerase is DNA polymerase I.

Suitable nucleic acid molecules which acts as a substrate for nucleic acid modifying activity of the microorganisms are described in WO2011/130584, WO2010/119270 and WO2009/007719. In the case of phosphatase activity, suitable nucleic acid molecules are disclosed in WO2006/123154.

In specific embodiments, the (substrate) nucleic acid molecule used in the methods of the invention is at least partially double stranded and comprises uracil residues in the complementary strand and the step of specifically determining the absence or presence of the modified nucleic acid molecule comprises adding Uracil DNA Glycosylase (UDG) to the sample in order to degrade the uracil residues in the complementary strand.

In certain embodiments, the (substrate) nucleic acid molecule comprises or is a DNA molecule, typically a DNA oligonucleotide. In certain embodiments, the (substrate) nucleic acid molecule comprises DNA and is partially double-stranded.

In some embodiments, the (substrate) nucleic acid molecule comprises a nucleic acid consisting of a sense oligonucleotide (DNA) strand and an antisense oligonucleotide (DNA) strand, wherein the two strands overlap to form a double stranded region and a single stranded portion of the antisense oligonucleotide strand acts as a template with the sense oligonucleotide strand of the double stranded region acting as a primer to create an extension product in the presence of polymerase activity;

In certain embodiments, the first strand of the partially double stranded (substrate) nucleic acid molecule comprises (or consists of) synthetic nucleotides (e.g. phosphorothioate nucleotides) and the second (complementary) strand comprises (or consists of) uracil residues and, optionally, synthetic nucleotides (e.g. phosphorothioate nucleotides). Preferably, the double stranded region encompasses the 3' end regions of the first and second (complementary) strands. In some embodiments, the second (complementary) strand comprises a base (e.g. dideoxyCytidine) at its 3' end that blocks DNA polymerase-mediated extension of the second strand. Such partially double stranded (substrate) nucleic acid molecules are described, for example, in Zweitzig et al., 2012 (Characterization of a novel DNA polymerase activity assay enabling sensitive, quantitative and universal detection of viable microbes. Nucleic Acids Research 40, 14, e109, 1-12. Preferably, the double stranded region is at least 5, at least 10, at least 15, at least 20 or at least 25 nucleotides; optionally, the double stranded region is no more than 50 nucleotides. The first strand may be extended during an incubation step, as described

herein, using unprotected (or standard) dNTPs by the polymerase activity of a microorganism in the sample to form an extended first strand that comprises unprotected (or standard) nucleotides. This step relies upon using the second strand as template (upstream of the region of complementarity between the first and second strands). Following the incubation step, the second (complementary) strand may be degraded by adding Uracil DNA Glycosylase (UDG) to the sample leaving the extended first strand as a single stranded molecule comprising synthetic nucleotides and unprotected nucleotides. Following degradation of the second strand, the extended first strand of the (substrate) nucleic acid molecule may be detected in an amplification step. The inventors have found that the use of a partially double stranded (substrate) nucleic acid molecule as described above improves the detection of a microorganism in the sample.

In some embodiments, the substrate nucleic acid molecule is pre-modified so as to protect it from nuclease activity i.e. the nucleic acid molecule is modified so as to protect it from nuclease activity before it is added to the assay. The inventors have determined that protection of the substrate nucleic acid molecule from nuclease activity is advantageous in the context of the assays of the invention. More specifically, incorporation of protected nucleic acid molecules into the methods of the invention improves sensitivity of detection. Any suitable means may be employed in order to protect the nucleic acid molecule from nuclease activity. Non-limiting examples include incorporation of methylation into the nucleic acid molecule, end modification such as protection of the 3' and/or 5' ends and incorporation of synthetic nucleotides. In specific embodiments, the synthetic nucleotides comprise phosphorothioate nucleotides and/or locked nucleic acid nucleotides. Preferably, the synthetic nucleotides are phosphorothioate nucleotides. In certain embodiments, the synthetic nucleotides replace at least one up to all of the nucleotides in the nucleic acid molecule.

The (substrate) nucleic acid molecules may include any natural nucleic acid and natural or synthetic analogues that are capable of being acted upon by nucleic acid modifying activity in order to generate a (novel detectable) nucleic acid molecule. The substrate may be extended and/or ligated in specific embodiments. Combinations of nucleic acid substrate molecules may be employed to permit detection of polymerase and ligase activity in some embodiments.

The nucleic acid substrate may be present in excess, and in particular in large molar excess, over the nucleic acid modifying activity (provided by the microorganisms) in the sample. Because a novel extended or ligated nucleic acid molecule is detected, only the presence of this molecule in the sample is essential for the detection methods to work effectively. Thus, it is not detrimental to the methods of the invention if other nucleic acid molecules are present in the sample such as from the microorganisms to be detected or from mammalian or other sources which may be found in the sample to be tested for example.

The inventors have previously investigated the use of an internal positive control (IPC) molecule in the context of their methods. Thus, according to all aspects, the invention may rely upon inclusion of an IPC molecule. In some embodiments, the IPC is included with the substrate nucleic acid molecule so that the IPC is exposed to identical conditions. In some embodiments, the IPC molecule is pre-modified so as to protect it from nuclease activity i.e. the nucleic acid molecule is modified so as to protect it from nuclease activity before it is added to the assay. The inventors have determined that protection of the IPC molecule from nuclease activity is advantageous in the context of the assays of the invention. Any suitable means may be employed in order to protect the nucleic acid molecule from nuclease activity. Non-limiting examples include incorporation of methylation into the nucleic acid molecule, end modification such as protection of the 3' and/or 5' ends and incorporation of synthetic nucleotides. In specific embodiments, the synthetic nucleotides comprise phosphorothioate nucleotides and/or locked nucleic acid nucleotides. Preferably, the synthetic nucleotides are phosphorothioate nucleotides. In certain embodiments, the synthetic nucleotides replace at least one up to all of the nucleotides in the IPC molecule. Preferably, the substrate and IPC molecules are modified in the same manner as it is advantageous for them to behave similarly in the assays of the invention.

In some embodiments, the internal positive control (IPC) nucleic acid molecule comprises identical primer binding sites to the substrate nucleic acid molecule such that there is competition for primer binding in a nucleic acid amplification reaction containing both the nucleic acid molecule and the IPC.

In all methods of the invention specifically determining the absence or presence of the modified nucleic acid molecule may comprise, consist essentially of or consist of a nucleic acid amplification step. This serves to make the methods of the invention maximally sensitive. Such amplification techniques are well known in the art, and include methods such as PCR, NASBA (Compton, 1991), 3SR (Fahy et al., 1991), Rolling circle replication, Transcription Mediated Amplification (TMA), strand displacement amplification (SDA) Clinical Chemistry 45: 777-784, 1999, the DNA oligomer self-assembly processes described in US6261846, ligase chain reaction (LCR) (Barringer et al., 1990), selective amplification of target polynucleotide sequences (US 6410276), arbitrarily primed PCR (WO 90/06995), consensus sequence primed PCR (US 4437975), invader technology, strand displacement technology and nick displacement amplification (WO 2004/067726). The list above is not intended to be exhaustive. Any nucleic acid amplification technique may be used provided the appropriate nucleic acid product is specifically amplified.

Similarly, sequencing based methodologies may be employed in some embodiments to include any of the range of next generation sequencing platforms, such as sequencing by synthesis of clonally amplified sequences (Illumina), pyrosequencing, 454 sequencing (Roche), nanopore sequencing (e.g. Oxford Nanopore), ion torrent (ThermoFisher) and single molecule real-time (SMRT) sequencing (Pacific Biosystems). The fact that a novel nucleic acid molecule is generated means that a sequencing approach can confirm the presence or otherwise of the modified nucleic acid molecule and also provide quantification of that molecule.

Amplification is achieved with the use of amplification primers specific for the sequence of the modified nucleic acid molecule which is to be detected. In order to provide specificity for the nucleic acid molecules primer binding sites corresponding to a suitable region of the sequence may be selected. The skilled reader will appreciate that the nucleic acid molecules may also include sequences other than primer binding sites which are required for detection of the novel nucleic acid molecule produced by the modifying activity in the sample, for example RNA Polymerase binding sites or promoter sequences may be required for isothermal amplification technologies, such as NASBA, 3SR and TMA.

One or more primer binding sites may bridge the ligation/extension boundary of the substrate nucleic acid molecule such that an amplification product is only generated if ligation/extension has occurred, for example. Alternatively, primers may bind either side of the ligation/extension boundary and direct amplification across the boundary such that an amplification product is only generated (exponentially) if the ligated/extended nucleic acid molecule is formed. Primers and the substrate nucleic acid molecule(s) may be designed to avoid non-specific amplification (e.g. of genomic DNA in the sample).

Primers may incorporate synthetic nucleotide analogues as appropriate or may be RNA or PNA based for example, or mixtures thereof. The primers may be labelled, such as with fluorescent labels and/or FRET pairs, depending upon the mode of detection employed.

Probes may be utilised, again which may be labelled, as desired. The detection method may require use of nucleotide probes in addition to primers, or as an alternative to primers. For example, a branched DNA assay, which does not require use of primers, may be employed in some embodiments.

In certain aspects, the methods of the invention are carried out using nucleic acid amplification techniques in order to detect the modified nucleic acid molecule produced as a direct result of the action of nucleic acid-modifying activity on the substrate nucleic acid molecule which indicates the presence of a micro-organism in the sample. In certain embodiments the technique used is selected from PCR, NASBA, 3SR, TMA, SDA and DNA oligomer self-assembly.

Detection of the amplification products may be by routine methods, such as, for example, gel electrophoresis but in some embodiments is carried out using real-time or end-point detection methods.

A number of techniques for real-time or end-point detection of the products of an amplification reaction are known in the art. These include use of intercalating fluorescent dyes such as SYBR Green I (Sambrook and Russell, Molecular Cloning - A Laboratory Manual, Third edition), which allows the yield of amplified DNA to be estimated based upon the amount of fluorescence produced. Many of the real-time detection methods produce a fluorescent read-out that may be continuously monitored; specific examples including molecular beacons and fluorescent resonance energy transfer probes. Real-time and end-point techniques are advantageous because they keep the reaction in a "single tube". This means there is no need for downstream analysis in order to obtain results, leading to more rapidly obtained results. Furthermore keeping the reaction in a "single tube" environment reduces the risk of cross contamination and allows a quantitative output from the methods of the invention. This may be particularly important in the context of the present invention where health and safety concerns may be of paramount importance (such as in detecting potential microbial infection in a patient samples for example).

Real-time and end-point quantitation of PCR reactions may be accomplished using the TaqMan® system (Applied Biosystems), see Holland et al; Detection of specific polymerase chain reaction product by utilising the 5'-3' exonuclease activity of Thermus aquaticus DNA polymerase; Proc. Natl. Acad. Sci. USA 88, 7276-7280 (1991), Gelmini et al. Quantitative polymerase chain reaction-based homogeneous assay with flurogenic probes to measure C-Erb-2 oncogene amplification. Clin. Chem. 43, 752-758 (1997) and Livak et al. Towards fully automated genome wide polymorphism screening. Nat. Genet. 9, 341-342 (19995). This type of probe may be generically referred to as a hydrolytic probe. Suitable hydrolytic/Taqman probes for use in real time or end point detection are also provided. The probe may be suitably labelled, for example using the labels detailed below.

In the Molecular Beacon system, see Tyagi & Kramer. Molecular beacons - probes that fluoresce upon hybridization. Nat. Biotechnol. 14, 303-308 (1996) and Tyagi et al. Multicolor molecular beacons for allele discrimination. Nat. Biotechnol. 16, 49-53 (1998), the beacons are hairpin-shaped probes with an internally quenched fluorophore whose fluorescence is restored when bound to its target. These probes may be referred to as hairpin probes.

A further real-time fluorescence based system which may be incorporated in the methods of the invention is the Scorpion system, see Detection of PCR products using self-probing amplicons and fluorescence by Whitcombe et al. Nature Biotechnology 17, 804 - 807 (01 Aug 1999). Additional real-time or end-point detection techniques which are well known to those skilled in the art and which are commercially available include Lightcycler® technology, Amplifluour® primer technology, DzyNA primers (Todd et al., Clinical Chemistry 46:5, 625-630 (2000)), or the Plexor™ qPCR and qRT-PCR Systems.

Thus, in further aspects of the invention the products of nucleic acid amplification are detected using real-time or end point techniques. In specific embodiments of the invention the real-time technique consists of using any one of hydrolytic probes (the Taqman® system), FRET probes (Lightcycler® system), hairpin primers (Amplifluour® system), hairpin probes (the Molecular beacons system), hairpin probes incorporated into a primer (the Scorpion® probe system), primers incorporating the complementary sequence of a DNAzyme and a cleavable fluorescent DNAzyme substrate (DzYNA), Plexor qPCR and oligonucleotide blocking systems.

Amplification products may be quantified to give an approximation of the microbial nucleic acid modifying activity in the sample and thus the level of microorganisms in the sample. Thus, "absence or presence" is intended to encompass quantification of the levels of microorganisms in the sample.

The inventors have further discovered that the optimal temperature for measuring nucleic acid modifying activity of the microorganisms may not be the same as the optimal temperature for lysis of microorganisms. Thus, in some embodiments, lysis of the microorganisms is performed at a lower temperature than the step of incubating the lysate with a nucleic acid molecule that acts as a substrate for nucleic acid modifying activity of the microorganisms. As already discussed, in some embodiments of the invention, the substrate nucleic acid molecule is included in the lysis reagent used to lyse the microorganisms. Such embodiments are consistent with the differing temperature preferences. Thus, even though the substrate nucleic acid molecule may be included in the lysis reagent, the initial lower temperature does not adversely affect the subsequent incubation at higher temperature, at which the substrate is modified by the nucleic acid modifying activity released from the microorganisms. Accordingly, in some embodiments the method involves a step of lysis of the microorganisms in which the lysis reagent contains a nucleic acid molecule which acts as a substrate for nucleic acid modifying activity of the microorganisms. This step is performed at a lower temperature than the subsequent step of incubating the lysate with the substrate nucleic acid molecule to enable the activity of the enzymes released from the microorganisms. Thus, the substrate is exposed to the initial lower temperature, followed by a higher temperature under which enzyme activity is enhanced.

In some embodiments, the step of incubating the lysate with a nucleic acid molecule that acts as a substrate for nucleic acid modifying activity of the microorganisms is performed at a temperature of at least around 30°C. The temperature may be optionally between around 30°C and 40°C or between around 32°C and 37°C, such as around 37°C.

In additional or alternative embodiments, the step of lysis of the microorganisms is performed at a temperature of no more than around 30°C, optionally between around 15°C and 30°C or between around 18°C and 25°C, such as around 18, 19, 20, 21, 22, 23, 24 or 25°C. In some embodiments, all steps prior to incubating the lysate with a nucleic acid molecule that acts as a substrate for nucleic acid modifying activity of the microorganisms are performed at a temperature of no more than around 30°C. The temperature may optionally be between around 15°C and 30°C or between around 18°C and 25°C, such as around 18, 19, 20, 21, 22, 23, 24 or 25°C.

Such a method may incorporate any one or more up to all of the embodiments described in relation to the various aspects of the invention.

In some embodiments, the method is further characterised in that the step of incubating the lysate with a substrate nucleic acid molecule is performed at a temperature of at least around 30°C, optionally between around 30°C and 40°C or between around 32°C and 37°C, such as around 37°C.

In additional or alternative embodiments, each of steps prior to incubating the lysate with a substrate nucleic acid molecule is performed at a temperature of no more than around 30°C, optionally between around 15°C and 30°C or between around 18°C and 25°C, such as around 18, 19, 20, 21, 22, 23, 24 or 25°C.

As introduced above, prior to the step of incubating the sample with magnetic particles to form particle-microorganism complexes (i.e. prior to step (a)), the method may comprise selectively lysing non-microorganism cells in the sample whilst retaining intact microorganisms present in the sample.

The step of selectively lysing non-microorganism cells in the sample whilst retaining intact microorganisms present in the sample may comprise adding a combination of a detergent and one or more enzymes to the sample. The one or more enzymes may comprise a proteinase and/or a DNAse, optionally wherein the proteinase is proteinase K.

The step of selective lysis of non-microorganism cells in the sample whilst retaining intact any microorganisms present in the sample may prevent enzymatic activity from non-microorganism cells, such as leukocytes, falsely indicating the presence of microorganisms in the sample. Such selective lysis can be achieved by any suitable means as discussed further herein. Any suitable reagent that lyses non-microorganisms, in particular mammalian cells, present in the sample but does not lyse microorganisms in the sample may be utilised. The reagent may include a surfactant or detergent in some embodiments, such as a non-ionic detergent. Suitable examples include polyethylene glycol sorbitan monolaurate (Tween 20), for example at 1 to 5% w/v, preferably around 1%. The reagent may include a saponin, for example at 1 to 5% w/v, preferably around 1%. The reagent may include a metal halide salt, such as sodium chloride, for example at 8.5g/l. The reagent may include a mixture of all three components. The sample may be mixed with the reagent under suitable conditions to ensure lysis of non-microorganism cells, in particular mammalian cells, if present in the sample but no (or insignificant) lysis of microorganisms if present in the sample. The sample may be exposed to the reagent for a period of between around 5 and 30 minutes, such as 5, 10, 15, 20, 25 or 30 minutes. This step may be performed at any suitable temperature, for example between 15 and 30 degrees Celsius or at room temperature. The selective lysis of non-microorganism cells may lyse substantially all the non-microorganism cells present. The selective lysis of non-microorganism cells may also lyse some, but critically not all, microorganism cells present in the sample, particularly if the microorganisms are fragile and/or have been exposed to a high concentration of antibiotic.

In some embodiments, according to all aspects of the invention, selective lysis of non-microorganism cells in the sample whilst retaining intact any microorganisms present in the sample comprises adding a combination of a detergent and one or more enzymes to the sample. Without wishing to be bound by any particular theory, the detergent selectively permeabilises non-microorganism cell membranes, whereas the microorganisms are protected by virtue of their cell wall. The enzymes are useful for breaking down released intracellular material and other cellular debris and may contribute to preventing carryover of released enzymatic activity. In some embodiments, the one or more enzymes comprise a proteinase and/or a nuclease. Suitable proteinases include proteinase K. Suitable nucleases include DNAses. In one embodiment, the reagent used to selectively lyse non-microorganism cells comprises a combination of triton X-100 and proteinase K. More specifically the lysis reagent may comprise 0.25% Triton X-100 and 4.8 μg/mL Proteinase K.

It is important to inactivate any relevant enzymatic activity released if the non-microorganism cells are lysed. The inventors have devised methods in which high pH conditions are utilised to ensure effective inactivation of the enzymatic activity. The microbial cells typically remain intact, at least during some of the treatment, and intracellular enzymatic activity is not significantly adversely affected by the high pH treatment. In addition, the inventors have previously shown that microbial enzymes are more resistant to the high pH treatment in any case.

Accordingly, after the step of selective lysis of non-microorganism cells in the sample whilst retaining intact any microorganisms present in the sample, the method may comprise exposing the lysate to high pH conditions. The duration of exposure to the high pH conditions is typically less than 20 minutes and may be not more than 10, 9, 8, 7, 6 or 5 minutes and may be around 5, 6, 7, 8, 9 or 10 minutes. In some embodiments the treatment is carried out for between around 2 and 15 minutes, such as around 5 minutes. By "around" in this context is meant plus or minus 30 seconds.

Any suitable reagent may be in order to provide high pH conditions. In particular embodiments, the high pH conditions comprise contacting the sample with an alkali or a buffer. In particular embodiments, NaOH or $Na_2CO_3$ is used. In specific embodiments, the concentration of the NaOH or $Na_2CO_3$ is around 5mM or greater. The buffer may have a pKa value above 9. Examples of suitable buffers include borate, carbonate and pyrophosphate buffers.

The high pH conditions typically inhibit the activity of nucleic acid modifying enzymes including ATP-dependent ligase and polymerases from non-microorganism sources such as mammalian cells, but do not inhibit the activity of the microbial ligases or polymerases. This is primarily due to the differential lysis conditions employed in the methods to ensure that only the non-microorganism enzymes are exposed to the high pH conditions. However, it may also be due to the greater resistance of microbial enzymes to these conditions. "High pH" is generally a pH of at least around 10, such as around 10, 11, 12, 13 or 14. "Low pH" is generally a pH of less than or equal to around 4, such as around 4, 3, 2, or 1. By "around" in this context is meant 0.5 of a pH unit either side of the stated value. Altering the pH of the sample may be achieved using any suitable means, as would be readily appreciated by one skilled in the art. Microbial enzymes such as polymerases and ligases may be resistant to extremes of pH, whereas corresponding mammalian enzymes may be inactivated under the same pH conditions. This assists with the selective detection of microbial enzymatic activity in a sample containing both mammalian cells and microbial cells. In specific embodiments, the conditions that inhibit the activity of non-microorganism nucleic acid modifying activity, such as ATP-dependent ligase, from mammalian cells but which do not inhibit the activity of the microorganism source of nucleic acid modifying activity, such as microbial ligases, comprise treating the sample with sodium hydroxide (NaOH) or sodium carbonate (Na2CO3). Such agents can readily be used, as shown herein, to increase the pH of the sample to high pH thus inactivating non-microorganism enzymatic activity whilst leaving the microbial (fungal and bacterial) enzymes active. Suitable concentrations and volumes of the appropriate agent can be applied by a skilled person. In certain embodiments, however, the NaOH is at least around 5mM NaOH. In some embodiments, the alkali concentration is no more than 10mM, such as 5, 6, 7, 8, 9 or 10mM.

In further embodiments, the pH is around 12 to inactivate mammalian nucleic acid modifying activity (such as polymerase and/or ATP-dependent ligase activity), but not microbial nucleic acid modifying activity (such as polymerase and/or ligase activity). In specific embodiments, pH conditions may be increased to at least around 11, or at least 11.2. This treatment may, after a certain period of time, result in lysis of microorganisms in the sample and thus lead to nucleic acid modifying activity (e.g. polymerase and/or ligase) release into the sample. Thus, in some embodiments, the lysis of microorganisms is achieved by high pH treatment. This permits detection of nucleic acid modifying activity (e.g. polymerases and/or ligases) in the sample, originating from the microorganism, without the need for a separate cell lysis step. Under these conditions, mammalian ligases (such as blood ATP-dependent ligases) are inactivated. However, typically the methods include a separate step for lysing microorganisms in the sample, as discussed in greater detail herein.

In some embodiments, the treatment under high pH conditions is stopped by adding a reagent to lower the pH. This is done before the microorganisms are lysed. Suitable reagents include a buffer and/or an acid. Thus, the pH may be reduced by adding a neutralisation buffer. In specific embodiments, the buffer comprises a Tris-HCl buffer (e.g. pH 7.2 or 8). Other suitable agents for lowering the pH include acids such as hydrochloric acid (HCl) and sulphuric acid ($H_2SO_4$). These (and other) acids may be incorporated into a buffer as would be readily appreciated by one skilled in the art. One specific reagent useful for treating the sample after the pH has been elevated comprises a combination of Ammonium sulphate, Magnesium sulphate heptahydrate, Potassium chloride and Tris-HCl. More specifically, the reagent may comprise 10 mM Ammonium sulphate, 2 mM Magnesium sulphate heptahydrate, 10 mM Potassium chloride and 20 mM Tris-HCl [pH 8.0].

The methods of the invention may be used to complement any already available diagnostic techniques, potentially as a method of confirming an initial diagnosis. Alternatively, the methods may be used as a preliminary diagnosis method in their own right, since the methods provide a quick and convenient means of diagnosis. Furthermore, due to their inherent sensitivity, the methods of the invention require only a minimal sample, thus preventing unnecessary invasive surgery. Also, a large but non-concentrated sample may also be tested effectively according to the methods of the invention.

The methods of the invention may involve identifying the nature of the infection, once the positive presence of a microorganism has been detected in the sample. Any suitable method may be employed for this further identification step.

## COMPOSITIONS AND KITS (not according to the invention)

Compositions and kits described herein are not encompassed by the wording of the claims but are considered useful for understanding the invention. In the course of performing the methods of the invention new compositions (combinations of components) are created.

Accordingly, described herein is a composition comprising: a) a sample containing an antimicrobial agent and viable microorganisms; and b) coated particles capable of forming complexes with the viable microorganisms in the sample.

Similarly, described herein is a composition comprising a sample containing an antimicrobial agent and viable microorganisms complexed with coated particles.

In the compositions, the sample is as defined herein. Thus, the sample that contains viable microorganism cells may further comprise non-microorganism cells. The non-microorganism cells may comprise blood cells. The blood cells may comprise red and/or white blood cells.

Also described herein is a kit for performing any of the methods described herein.

Described herein is a kit comprising: a) a vessel containing coated particles capable of forming complexes with viable microorganisms; b) a vessel containing a medium suitable for incubating and/or culturing the viable microorganisms recovered using the coated particles and that does not contain an antimicrobial agent and/or an agent capable of binding an antimicrobial agent; and/or c) a vessel containing a wash buffer for washing coated particles recovered from a sample, which wash buffer does not lyse viable microorganisms.

In the kits, components a), b) and c) may be in separate vessels. The vessels described herein may be any suitable container for the relevant reagent as would be understood by one skilled in the art. Typically, the vessels contain sufficient quantity or concentration of the reagent to perform a method of the invention once. Thus, the kit may be a single use kit. The vessel containing coated particles capable of forming complexes with viable microorganisms is a container of material and volume suitable for containing the coated particles. The container may also be of material and volume suitable for incubating the sample with the coated particles to form particle-microorganism complexes. This means the sample can be simply added to the vessel containing the coated particles in order to begin the method. The vessel containing medium is a container of material and volume suitable for containing the medium. The container may also be of material and volume to additionally contain the recovered viable microorganisms, in particular in the form of particle-microorganism complexes. Thus, the medium containing vessel may be suitable for incubating or culturing the particle-microorganism complexes. Alternatively, the medium may be added to another vessel (typically of larger volume) that already contains the particle-microorganism complexes. The vessel containing the wash buffer is a container of material and volume suitable for containing the wash buffer. Typically, the wash buffer is used to wash particle microorganism complexes contained in a separate vessel. Thus, the wash buffer may be provided in a vessel designed only to contain the wash buffer. Where multiple (e.g. 2 or 3) washes are needed the kit may contain multiple individual vessels each containing the appropriate amount of wash buffer for a single wash. Alternatively, a single vessel containing sufficient wash buffer for multiple (e.g. 2 or 3) washes may be provided.

Suitable wash buffers are described herein. In the kits, the wash buffer may be detergent free. The wash buffer may comprise Tris and/or sodium chloride (suitable concentrations are described in the examples herein). Typically, the wash buffer is used to remove non-microorganism cells in a wash step and thus the wash buffer does not lyse the non-microorganism cells.

All further means included in the kits may be contained in one or more vessels as appropriate.

The kit may further include means for detecting and/or characterising the recovered viable microorganisms. Any suitable means may be employed and they may represent the complete set of reagents needed for detecting and/or characterising the viable microorganisms.

The detection means may comprise, or is, a nucleic acid molecule which acts as a substrate for nucleic acid modifying activity of the microorganisms. Suitable substrate molecules, in particle DNA oligonucleotide substrate molecules, are described herein which discussion applies *mutatis mutandis.*

The detection means may comprise or further comprise reagents for nucleic acid amplification. The reagents for nucleic acid amplification may comprise a primer pair and/or at least one probe. Those primers and/or probes may hybridise with a microorganism nucleic acid molecule. They may therefore allow detection of microorganisms in the sample by detecting the amplified microorganism nucleic acid molecule. Alternatively, the primers or probes hybridise to a nucleic acid molecule which acts as a substrate for nucleic acid modifying activity of the microorganisms. Such nucleic acid molecules are described in further detail herein.

The kit may comprise: a) coated particles capable of (selectively) forming complexes with microorganisms (i.e. particle-microorganism complexes); and (b) detection means for detecting the absence or presence of microorganisms in the particle-microorganism complexes. The detection means may comprise a nucleic acid molecule which acts as a substrate for nucleic acid modifying activity of the microorganisms. The nucleic acid molecule may be at least partially double stranded and may, optionally, comprise uracil residues in the complementary strand. The complementary strand may comprise a base (e.g. dideoxyCytidine) at its 3' end that blocks DNA polymerase-mediated extension of the second strand. The nucleic acid molecule may be any of the nucleic acid molecules described herein.

Accordingly, the kits may further comprise: a) a nucleic acid molecule which acts as a substrate for nucleic acid modifying activity of the viable microorganisms; or b) primers and/or a probe which hybridize specifically to a nucleic acid (extracted) from the viable microorganism.

Also described herein is a kit comprising: a) a vessel containing coated particles capable of forming complexes with viable microorganisms and b) a nucleic acid molecule which acts as a substrate for nucleic acid modifying activity of the viable microorganisms; or primers and/or a probe which hybridize specifically to a nucleic acid from the viable microorganism.

The discussion below applies *mutatis mutandis* to the related methods.

According to all aspects of the invention, the nucleic acid from the viable microorganism may be a DNA molecule, e.g. a gene, or a RNA molecule, such as a mRNA or miRNA for example. Preferred targets are genes, such as ILV3 as discussed herein. Other preferred targets are pathogenicity genes, such as those encoding Shiga toxin (e.g. in Enterohaemorrhagic *E.coli* infection).

According to all aspects of the invention, nucleic acid molecules which act as a substrate for nucleic acid modifying activity are described elsewhere herein. Suitable substrates are discussed in further detail herein. By "substrate" is meant a nucleic acid molecule which is acted on (directly) by the microorganism-derived enzyme. Typically, such nucleic acid molecules are oligonucleotide substrates. They are synthetic nucleic acid molecules.

According to all aspects of the invention, the primers and/or a probe which hybridize specifically to a nucleic acid from the viable microorganism may permit determination of whether a bacteria or a fungus is present in the sample and/or whether the bacteria or fungus has antimicrobial resistance genes. The kit may comprise a plurality of primer pairs and/or a plurality of probes for the detection and/or characterisation of the microorganism. The plurality of primer pairs and/or a plurality of probes may permit the detection and/or characterisation of bacteria and fungi. The primers and/or a probe may permit identification of the genus and/or species of the microorganism present in the sample.

According to all aspects of the invention, by "hybridising specifically", or equivalent language, is meant that the primers hybridise to the targeted nucleic acid but do not hybridise (or cross-react) with other nucleic acids. Primers and/or probes may hybridise to particular subregions within a gene. The primers and/or a probe may hybridize specifically to the same gene in multiple species in the same genus to permit identification of the genus of the microorganism. Alternatively, the primers and/or a probe may hybridize specifically to a gene in a single species of microorganism to permit identification of the species of microorganism.

According to all aspects of the invention, as described in WO2018189502, the ILV3 gene is particularly useful in detecting whether a fungus or yeast is present in a sample due to the lack of sequence identity with the human genome.

According to all aspects of the invention, for the detection or characterisation of fungus/yeast, primers may comprise:

a. a forward and reverse primer hybridizing specifically to the ILV3 gene of the following *Candida* species

i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*

and optionally comprises, consists essentially of or consists of the nucleotide sequence of SEQ ID NO: 1 and SEQ ID NO: 2 respectively;

b. a forward and reverse primer hybridizing specifically to the ILV3 gene of the following *Aspergillus* species

i. *Aspergillus fumigatus*
ii. *Aspergillus niger*
iii. *Aspergillus flavus*

and optionally comprises, consists essentially of or consists of the nucleotide sequence of SEQ ID NO: 70 and 71 or SEQ ID NO: 73 and 74 respectively;

c. a forward and reverse primer hybridizing specifically to the ILV3 gene of *Candida albicans* and optionally comprises, consists essentially of or consists of the nucleotide sequence of SEQ ID NO: 4 and 5 or SEQ ID NO: 6 and 7 respectively;

d. a forward and reverse primer hybridizing specifically to the ILV3 gene of *Candida dubliniensis* and optionally comprises, consists essentially of or consists of the nucleotide sequence of SEQ ID NO: 8 and 9, SEQ ID NO: 10 and 11 or SEQ ID NO: 12 and 13 respectively;

e. a forward and reverse primer hybridizing specifically to the ILV3 gene of *Candida tropicalis* and optionally comprises, consists essentially of or consists of the nucleotide sequence of SEQ ID NO: 14 and 15 or SEQ ID NO: 16 and 17 respectively;

f. a forward and reverse primer hybridizing specifically to the ILV3 gene of *Candida parapsilosis* and optionally comprises, consists essentially of or consists of the nucleotide sequence of SEQ ID NO: 18 and 19 or SEQ ID NO: 20 and 21 respectively;

g. a forward and reverse primer hybridizing specifically to the ILV3 gene of *Candida glabrata* and optionally comprises, consists essentially of or consists of the nucleotide sequence of SEQ ID NO: 22 and 23, SEQ ID NO: 24 and 25, SEQ ID NO: 26 and 27 or SEQ ID NO: 28 and 29 respectively;

h. a forward and reverse primer hybridizing specifically to the ILV3 gene of *Candida krusei* and optionally comprises, consists essentially of or consists of the nucleotide sequence of SEQ ID NO: 30 and 31, SEQ ID NO: 32 and 33, SEQ ID NO: 34 and 35, SEQ ID NO: 36 and 37 or SEQ ID NO: 38 and 39 respectively;

i. a forward and reverse primer hybridizing specifically to the ILV3 gene of *Candida guilliermondii* and optionally comprises, consists essentially of or consists of the nucleotide sequence of SEQ ID NO: 40 and 41, SEQ ID NO:

42 and 43, SEQ ID NO: 44 and 45 or SEQ ID NO: 46 and 47 respectively;

j. a forward and reverse primer hybridizing specifically to the ILV3 gene of *Candida auris* and optionally comprises, consists essentially of or consists of the nucleotide sequence of SEQ ID NO: 48 and 49, SEQ ID NO: 50 and 51, SEQ ID NO: 52 and 53, SEQ ID NO: 54 and 55, SEQ ID NO: 56 and 57, SEQ ID NO: 58 and 59, SEQ ID NO: 60 and 61, SEQ ID NO: 62 and 63, SEQ ID NO: 64 and 65, SEQ ID NO: 66 and 67 or SEQ ID NO: 68 and 69 respectively;

k. a forward and reverse primer hybridizing specifically to the ILV3 gene of *Aspergillus fumigatus* and optionally comprises, consists essentially of or consists of the nucleotide sequence of SEQ ID NO: 76 and 77, SEQ ID NO: 79 and 80, SEQ ID NO: 82 and 80 or SEQ ID NO: 83 and 84 respectively;

l. a forward and reverse primer hybridizing specifically to the ILV3 gene of *Aspergillus niger* and optionally comprises, consists essentially of or consists of the nucleotide sequence of SEQ ID NO: 87 and 86;

m. a forward and reverse primer hybridizing specifically to the ILV3 gene of *Aspergillus flavus* and optionally comprises, consists essentially of or consists of the nucleotide sequence of SEQ ID NO: 90 and 89 or SEQ ID NO: 90 and 92 respectively; and/or

n. a forward and reverse primer hybridizing specifically to the ILV3 gene of *Cryptococcus neoformans* and optionally comprises, consists essentially of or consists of the nucleotide sequence of SEQ ID NO: 93 and 94, SEQ ID NO: 96 and 97, SEQ ID NO: 99 and 100, SEQ ID NO: 102 and 103 or SEQ ID NO: 105 and 106 respectively.

According to all aspects of the invention, for the detection or characterisation of fungus/yeast, the probe may comprise:

a. a probe that hybridizes specifically to the ILV3 gene of the following *Candida* species

i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*

and optionally comprises, consists essentially of or consists of the nucleotide sequence of SEQ ID NO: 3

b. a probe that hybridizes specifically to the ILV3 gene of *Candida albicans* and optionally comprises, consists essentially of or consists of the nucleotide sequence of SEQ ID NO: 116 or 117

c. a probe that hybridizes specifically to the ILV3 gene of *Candida dubliniensis* and optionally comprises, consists essentially of or consists of the nucleotide sequence of SEQ ID NO: 118, 119 or 120

d. a probe that hybridizes specifically to the ILV3 gene of *Candida tropicalis* and optionally comprises, consists essentially of or consists of the nucleotide sequence of SEQ ID NO: 121

e. a probe that hybridizes specifically to the ILV3 gene of *Candida parapsilosis* and optionally comprises, consists essentially of or consists of the nucleotide sequence of SEQ ID NO: 122 or 123

f. a probe that hybridizes specifically to the ILV3 gene of *Candida glabrata* and optionally comprises, consists essentially of or consists of the nucleotide sequence of SEQ ID NO: 124, 125, 126 or 127

g. a probe that hybridizes specifically to the ILV3 gene of *Candida krusei* and optionally comprises, consists essentially of or consists of the nucleotide sequence of SEQ ID NO: 128, 129, 130, 131 or 132

h. a probe that hybridizes specifically to the ILV3 gene of *Candida guilliermondii* and optionally comprises, consists essentially of or consists of the nucleotide sequence of SEQ ID NO: 133, 134 or 135

i. a probe that hybridizes specifically to the ILV3 gene of *Candida auris* and optionally comprises, consists essentially of or consists of the nucleotide sequence of SEQ ID NO: 136, 137, 138, 139, 140, 141, 142, 143, 144, 145 or 146

j. a probe that hybridizes specifically to the ILV3 gene of the following *Aspergillus* species

i. *Aspergillus fumigatus*
ii. *Aspergillus niger*
iii. *Aspergillus flavus*

and optionally comprises, consists essentially of or consists of the nucleotide sequence of SEQ ID NO: 72 or 75

k. a probe that hybridizes specifically to the ILV3 gene of *Aspergillus fumigatus* and optionally comprises, consists essentially of or consists of the nucleotide sequence of SEQ ID NO: 78, 81 or 85

l. a probe that hybridizes specifically to the ILV3 gene of *Aspergillus niger* comprises, consists essentially of or consists of the nucleotide sequence of SEQ ID NO: 88

m. a probe that hybridizes specifically to the ILV3 gene of *Aspergillus flavus* and optionally comprises, consists essentially of or consists of the nucleotide sequence of SEQ ID NO: 91; and/or

n. a probe that hybridizes specifically to the ILV3 gene of *Cryptococcus neoformans* and optionally comprises, consists essentially of or consists of the nucleotide sequence of SEQ ID NO: 95, 98, 101, 104 or 107.

According to all aspects of the invention, for the detection and/or characterisation of bacteria, the primers and probes may hybridize specifically to specific parts of the 16S region of bacterial DNA. The primers may amplify specific parts of the 16S region of bacterial DNA. The primers PLK1 (5-TACGGGAGGCAGCAGT-3 - SEQ ID NO: 108) and PLK2 (5-TATTACCGC GGCTGCT-3 - SEQ ID NO: 109) are highly conserved in different groups of eubacteria. A 187-bp fragment is synthesized by these primers. PLK2 may be labelled with fluorescein internally. A probe may distinguish Gram-negative from Gram-positive bacteria. The fluorescence dye-labelled hybridization probes ISN2 (5-CCGCAGAATAAG CACCGGCTAACTCCGT-3 - SEQ ID NO: 110) and ISP2 (5-CCT AAC CAG AAA GCC ACG GCT AAC TAC GTG-3 - SEQ ID NO: 111) emit light at different wavelengths (640 and 705 nm) and can be used for detection and Gram stain differentiation of bacterial DNA by a fluorescence signal. Other suitable primers may comprise the nucleotide sequence CAACGCGAAGAACCTTACC (SEQ ID NO: 112) and ACGTCATCCCCACCTTCC (SEQ ID NO: 113). A suitable Gram-positive probe comprises the nucleotide sequence 5'-FAM-ACGACAACCATGCACCACCTG-TAMRA-3' (SEQ ID NO: 114). A suitable Gram-negative probe comprises the nucleotide sequence 5'-HEX-ACGACAGCCATGCAGCACCT-TAMRA'3(SEQ ID NO: 115). Although these probes are differently labelled to permit differential detection, it will be appreciated by the skilled person that alternative approaches as described herein may be adopted to facilitate detection.

Accordingly, the kit may comprise a) a vessel containing coated particles capable of forming complexes with viable microorganisms and b) a nucleic acid molecule which acts as a substrate for nucleic acid modifying activity of the viable microorganisms; or primers and/or a probe which hybridize specifically to the ILV3 gene in yeast and/or 16S rRNA gene of bacteria.

The kit may further comprise a reagent that selectively lyses non-microorganism cells in the sample whilst retaining intact microorganisms present in the sample.

The kits may further comprise i) sodium polyanethol sulfonate; and ii) at least one reagent that selectively lyses non-microorganism cells in the sample whilst retaining intact microorganisms present in the sample. Suitable reagents are known in the art and discussed further herein. As shown herein, sodium polyanethol sulfonate is useful for recovery of microorganisms from samples containing non-microorganism cells.

The kits may further comprise i) sodium polyanethol sulfonate; and ii) a detergent. A detergent is an example of a reagent that selectively lyses non-microorganism cells in the sample whilst retaining intact microorganisms present in the sample.

In view of the increased sensitivity of the antimicrobial agent-exposed microorganisms to lysis, in some embodiments the kit may not comprise a reagent that selectively lyses non-microorganism cells. Because the coated particles preferentially bind microorganisms there is no absolute requirement to lyse non-microorganisms in the sample. However, it may be advantageous to perform one or more washing steps to remove non-microorganism cells (typically intact non-microorganism cells) and/or cell lysate from the particle-microorganism complexes. Any suitable wash solution may be employed, examples are discussed herein.

The kit may comprise: a) particles capable of forming complexes with microorganisms; b) sodium polyanethol sulfonate; c) at least one reagent that selectively lyses non-microorganism cells in the sample whilst retaining intact microorganisms present in the sample; and d) detection means for detecting the absence or presence of microorganisms in the particle-microorganism complexes, wherein the detection means comprises a nucleic acid molecule which acts as a substrate for nucleic acid modifying activity of the microorganisms, and wherein the nucleic acid molecule is at least partially double stranded and comprises uracil residues in the complementary strand.

The (substrate) nucleic acid molecule may be designed on the basis that the nucleic acid modifying enzyme comprises a DNA or RNA polymerase, preferably a DNA-dependent DNA polymerase. In some embodiments, the DNA polymerase is DNA polymerase I. In additional or alternative embodiments, the nucleic acid modifying enzyme comprises a ligase, such as an ATP- or NAD-dependent ligase.

The detection means may further comprise reagents for nucleic acid amplification, optionally wherein the reagents for nucleic acid amplification comprise a primer pair and/or at least one probe that hybridises with the nucleic acid molecule.

The kit may further comprise a reagent capable of lysing microorganisms in the particle-microorganism complexes, optionally wherein the reagent capable of lysing microorganisms in the particle-microorganism complexes comprises the nucleic acid molecule which acts as a substrate for nucleic acid modifying activity of the microorganisms. Suitable examples include polyethylene glycol sorbitan monolaurate (Tween 20), for example at 1 to 5% w/v, preferably around 1%. The reagent may include a saponin, for example at 1 to 5% w/v, preferably around 1%.

The kit may further comprise a reagent that selectively lyses non-microorganism cells in the sample whilst retaining intact microorganisms present in the sample.

The reagent that selectively lyses non-microorganism cells in the sample whilst retaining intact microorganisms present in the sample may comprise one or more enzymes, wherein the one or more enzymes optionally comprise a proteinase and/or a DNAse. Suitable detergents and enzymes are discussed herein.

The kit may further comprise a high pH reagent e.g. a base or a buffer. This may, for example, be NaOH, e.g. 5mM NaOH. Other suitable reagents are described herein.

The kit may further comprise a neutralisation buffer. The neutralisation buffer may be capable of restoring the pH of the sample following the high pH treatment. Suitable reagents are described herein.

The nucleic acid modifying enzyme may comprise: (a) a DNA or RNA polymerase, optionally wherein the DNA polymerase is a DNA-dependent DNA polymerase such as DNA polymerase I; and/or (b) a ligase, optionally wherein the ligase is an ATP- and/or NAD-dependent ligase.

## ADDITIONAL FEATURES APPLICABLE TO ALL ASPECTS

As already mentioned, the present invention is particularly applicable to clinical samples, in particular samples taken from a subject that is suspected to be suffering from a microorganism infection. Thus, according to all aspects of the invention, the microorganism that may be recovered from the sample (and detected and/or characterised) may be a pathogenic microorganism, such as a pathogenic bacterium or fungus/yeast. The bacterium or fungus/yeast may be any bacterium or fungus/yeast which is capable of causing infection or disease in a subject, preferably a human subject. In one embodiment, the bacteria comprises or consists essentially of or consists of any one or more of Staphylococcus species, including *Staphylococcus epidermidis* and *Staphylococcus aureus* (and preferably methicillin resistant strains), Enterococcus species, Streptococcus species including *Streptococcus pneumonia*, Pseudomonas species, Klebsiella species, Mycobacterium species, in particular *Mycobacterium tuberculosis*, Vibrio species, in particular *Vibrio cholerae*, Salmonella and/or *Escherichia coli* etc. The bacteria may comprise, consist essentially of or consist of Clostridium species and in particular C. *difficile* in certain embodiments. C. *difficile* is the major cause of antibiotic-associated diarrhoea and colitis, a healthcare associated intestinal infection that mostly affects elderly patients with other underlying diseases. The bacteria may comprise, consist essentially of or consist of Pseudomonas species, in particular *Pseudomonas aeruginosa.* The bacteria may comprise, consist essentially of or consist of Klebsiella species, in particular, *Klebsiella pneumonia.* In one embodiment, the fungus/yeast comprises or consists essentially of any one or more *Candida, Aspergillus, Cryptococcus, Histoplasma, Pneumocystis* and/or *Stachybotrys* species.

The microorganism may be detected through its enzymatic activity, as described herein. Thus, the methods provide an indication of viable microorganisms recovered from the antibiotic-containing sample. After a period of time, if the microorganisms are not viable, the enzymatic activity would be lost from the sample. This represents an advantage of using enzymatic activity as an indicator of microorganisms in the sample over use of nucleic acid molecules, in particular DNA, which may persist for much longer in some embodiments.

The methods of the invention may involve identifying the nature of the infection, once the positive presence of a microorganism has been detected in the sample. Any suitable method may be employed for this further identification step, as discussed herein.

"Antimicrobial agent" is defined herein to encompass any agent that kills or inhibits the growth of a microorganism. The antimicrobial agent may be an antibiotic or antifungal agent. The antimicrobial may be any antimicrobial agent that is routinely used in the treatment of bloodstream infections. The antimicrobial used to treat the subject will be selected by the caregiver based on a clinical assessment of the subject. This information may be used in performing the present invention when considering which microorganism is considered most likely to be present in the sample. It may contribute towards selection of suitable conditions, e.g. suitable selective lysis conditions. For example, if the sample is likely to contain a more robust microorganism, detergents may be used for selective lysis. On the other hand, if the sample is likely to contain a more sensitive microorganism, no detergent may be employed and wash steps may be used instead. The antimicrobial agent may be broad-spectrum, which can kill or inhibit a range of microorganisms. Non-limiting examples of antibiotic agents include: penicillin, meropenem, flucloxacillin, ampicillin, oxacillin, piperacillin-tazobactam, vancomycin, teicoplanin, daptomycin, tigecycline, quinupristin/dalfopristin, gentamicin, amikacin, linezolid, azithromycin, clarithromycin, ciprofloxacin, levofloxacin, sparfloxacin, gatifloxacin, garenoxacin, gemifloxacin, moxifloxacin, doxycycline, TMP-SMX, polymyxin B, cefotaxime, cefotetan, cefamandole, cefuroxime, ceftizoxime, ceftazidime, cefixime, cefoperazone, cefepime, cefazolin, cefoxitin and ceftriaxone. Non-limiting examples of antifungal agents include: flucytosine, fluconazole, itraconazole, voriconazole, posaconazole, etoconazole, griseofulvin, amphotericin B, caspofungin, micafungin and anidulafungin. Administered amounts of these agents are determined according to standard of care procedures depending on the particular agent. This contributes to the levels of antimicrobial agent present in the sample. The antimicrobial may be present in the sample at a concentration of at least or more than 0.05, 0.5, 5, 10, 25, 50, 75 or 100 $\mu$g/mL. The antimicrobial may be present in the sample at a concentration of no more than or less than 100, 250, 500 or 1000 μg/mL. The antimicrobial may be present in the sample at a concentration of no more than or less than 100 μg/mL. The antimicrobial may typically be present in the sample at a concentration of between 0.5 and 250 μg/mL. The antimicrobial may be present in the sample at a concentration of between 0.5 and 100 μg/mL.

As explained herein, the invention is applied in the clinical setting in which a subject suspected of a microbial infection is treated with an antimicrobial agent before a body fluid (especially blood) sample can be taken. Thus, the antimicrobial agent given to a subject as treatment may be (and should be) the only source of antimicrobial agent in the sample. The antimicrobial agent has thus typically been selected by a care-giver (e.g. a doctor or nurse) and administered based on the current clinical symptoms demonstrated by the subject. In the invention, the microorganism in the sample is (or is thought to be) susceptible to the antimicrobial agent.

A "sample" in the context of the present invention is one which contains, or is suspected to contain, a microorganism, such as a fungus (e.g. a yeast) and/or a bacterium and also contains an antimicrobial agent. Typically, the sample is a liquid sample. Thus the sample may comprise, consist essentially of or consist of a clinical sample, such as a body fluid sample. A preferred sample type is blood, to include whole blood, plasma, serum and blood containing samples, such as a blood culture or blood broth. In some embodiments, the sample comprises blood, cerebral spinal fluid (CSF), joint fluid, urine or bronchoalveolar lavage (BAL). The sample may be a clinical sample taken from a subject that is receiving, or has received, treatment using the antimicrobial agent. The sample may comprise a sample from a patient suspected of suffering from, or being screened for, an infection. The sample may be any suitable volume such as 0.2 to 10 ml, or 1 to 10 ml.

The sample being used will depend on various factors, such as availability, convenience and the condition that is being tested for. Typical samples which may be used, but which are not intended to limit the invention, include whole blood, serum, plasma, platelet, joint fluid and urine samples etc. taken from a patient, most preferably a human patient. The patient may be suspected of suffering from, or being screened for, a bloodstream infection. The patient may be a hospitalised patient. The sample may be taken from a subject comprising more than 5, 10 or 15 million white blood cells (WBC) per ml of blood.

In addition to an antimicrobial agent, the sample may further comprise one or more inhibitors of later analysis. The one or more inhibitors of later analysis may be selected from: blood cell remnants, haemoglobin, leukocyte DNA and platelets.

The present invention, thus, also separates the microorganisms from the one or more inhibitors of microbial growth.

For the avoidance of doubt, the methods of the invention represent *in vitro* methods. They are carried out on a sample removed from a subject. The methods of the invention are carried out beginning with a sample that has already been isolated from the patient in a separate procedure. The methods will most preferably be carried out on a sample from a human, but the methods of the invention may have utility for many animals. Antimicrobial agents are frequently used in veterinary practice and agriculture.

The sample may comprise non-microorganism cells at a concentration of between 20,000 and 5 million cells per millilitre. The sample may comprise non-microorganism cells at a concentration of at least around 100,000 cells per millilitre. Preferably, the sample may comprise non-microorganism cells at concentration of at least around 20,000 cells per millilitre.

The particles used in the present invention are coated. The coating is typically a polymeric coating. Alternatively, the coated particles may lack a polymeric coating. The coated particles may be coated with molecules such as citrate, starch, mannose binding lectin or poly-L-lysine. The molecules coating the coated particles may be adsorbed onto the surface of the particles or form an amalgam with the particles. The coated particles may be completely or partially coated.

Coated particles are thus distinguished from uncoated particles. Uncoated particles include particles consisting of a metal or a metallic compound (e.g. metal oxide) with no other molecules adsorbed on the surface (e.g. citrate) or amalgamated with the metal or metallic compound. The coated particles are able to bind and form particle-microorganism complexes with a range of different microorganisms. They are thus "pan-microorganism" or "universal" in their microorganism specificity. The coated particles, which are preferably magnetic particles, may bind to the microorganisms by non-specific binding (in contrast to use of target-specific ligands such as antibodies etc.). The coated particles typically have a greater affinity for the microorganisms than for the non-microorganism cells.

The coated particles act by removing microorganisms from the sample, whilst the antimicrobial agent does not form (a significant) part of the formed complexes. Thus, the coated particles do not substantially bind to an antimicrobial agent.

The coated particles typically comprise a polymeric outer surface. The polymeric surface may be a regular outer polymeric surface, in which the surface of the particle is (relatively) uniformly coated (within usual manufacturing tolerances). The polymeric surface may comprise a carbon-based polymer. The polymeric surface may comprise derivatives and copolymers of polyimides, poly(ethylene glycol), polyvinyl alcohol, polyethyleneimine, and polyvinylamine, polyacrylates, polyacrylamides, polyamides, polyesters, polycarbonates, polyvinyls, polystyrenes, and any combination thereof. Preferably, the polymeric surface comprises polystyrene and/or poly(styrene/divinyl benzene) or polyacrylamide.

Most preferably, the polymeric surface comprises polystyrene and/or poly(styrene/divinyl benzene).

The polymeric surface may comprise derivatives and copolymers of polydimethylsiloxane, polyimide, polyethylene terephthalate, polymethylmethacrylate, polyurethane, polyvinylchloride, polystyrene polysulfone, polycarbonate, polymethylpentene, polypropylene, polyvinylidine fluoride, polysilicon, polytetrafluoroethylene, polysulfone, acrylonitrile butadiene styrene, polyacrylonitrile, polybutadiene, poly(butylene terephthalate), poly(ether sulfone), poly(ether ether ketones), poly(ethylene glycol), styrene-acrylonitrile resin, poly(trimethylene terephthalate), polyvinyl butyral, polyvinylidenedifluoride, poly(vinyl pyrrolidone), and any combination thereof. In some embodiments, the polymeric surface is not silicon based.

In specific embodiments according to all aspects of the invention, the coated particles may comprise, on the outer surface, any one or more of: i) carboxylic acid groups; ii) amino groups, iii) hydrophobic groups; and iv) streptavidin. These functionalities have been shown by the inventors to be useful in universal viable microorganism recovery. Such functionalities typically form part of the polymer coating and are not supplied by a separate targeting ligand. Thus, the i) carboxylic acid groups, ii) amino groups; or iii) hydrophobic groups may not be part of a polypeptide or peptidomimetic in some embodiments. Similarly, the i) carboxylic acid groups, ii) amino groups; or iii) hydrophobic groups may not be part of a nucleoside, nucleotide or nucleic acid. Also similarly, the i) carboxylic acid groups; ii) amino groups or iii) hydrophobic groups may not be part of a lipid, steroid, hormone or cofactor.

The coated particles may be magnetic and indeed are typically magnetic. Magnetic beads are paramagnetic and are attracted to an externally applied magnetic field. Magnetic beads are well known and commercially available. The coated particles may be superparamagnetic. The coated particles may comprise a metal or metallic compound. The coated particles may comprise iron oxide. The iron oxide may comprise magnetite and/or maghemite. The iron oxide may not comprise a 1:1, 2:1, 3:1 or 4:1 ratio of $Fe^{2+}$ and $Fe^{3+}$. Preferably, the iron oxide is encapsulated by a polymer coating. The coated particles may be an amalgam of iron oxide and polymer. The coated particles may be partially encapsulated by an outer polymeric surface, although this is less preferred. The coated particles may comprise a core and a polymer coating. The core may be magnetic. The coated particles may comprise iron oxide coated with citrate, starch, mannose binding lectin or poly-L-lysine.

The coated particles may have a diameter of between 0.05 and 20 $\mu$m, or between 0.05 and 1 $\mu$m, such as between 0.1 and 0.5 $\mu$m, or between 0.2 and 0.3 $\mu$m. Preferably, the coated particles may have a diameter of between 0.2 and 0.3 $\mu$m. The coated particles may have a diameter of between 0.1 and 3 $\mu$m or 0.1 and 2 $\mu$m. More preferably, the coated particles have a diameter of between 0.1 and 1.0 $\mu$m.

The coated particles may comprise:

a) a polymeric coating (such as polystyrene, poly(styrene/divinyl benzene) or polyacrylamide);
b) a polymeric coating (such as polystyrene, poly(styrene/divinyl benzene) or polyacrylamide) with one of the following additional groups on the surface: carboxylic acids, amines or streptavidin; or
c) citrate, mannose binding lectin, starch or poly-L-lysine molecules adhered to the surface.

The coated particles may be magnetic and comprise:

a) a polymeric coating (such as polystyrene, poly(styrene/divinyl benzene) or polyacrylamide);
b) a polymeric coating (such as polystyrene, poly(styrene/divinyl benzene) or polyacrylamide) with one of the following additional groups on the surface: carboxylic acids, amines or streptavidin; or
c) citrate, mannose binding lectin, starch or poly-L-lysine molecules adhered to the surface.

The coated particles may comprise a metal (such as iron) or metallic compound (such as iron oxide) and:

a) a polymeric coating (such as polystyrene, poly(styrene/divinyl benzene) or polyacrylamide);
b) a polymeric coating (such as polystyrene, poly(styrene/divinyl benzene) or polyacrylamide) with one of the following additional groups on the surface: carboxylic acids, amines or streptavidin; or
c) citrate, mannose binding lectin, starch or poly-L-lysine molecules adhered to the surface.

The coated particles thus have an outer polymeric surface. In some embodiments, the outer surface of the coated particles may not be coated with any of (i) an antibody, (ii) a carbohydrate, (iii) a peptide derived from Apolipoprotein H protein, (iv) a Mannose Binding Lectin protein, (v) a polyamine or (vi) a cationic detergent. The Mannose Binding Lectin (MBL) protein may be a genetically engineered protein based on MBL. For example, it may be a genetically engineered protein comprising the pathogen-binding portion of MBL fused to an Fc region of an immunoglobulin (i.e. FcMBL).

The outer surface of the coated particles may not be coated with any of (i) an antibody, (ii) a carbohydrate or (iii) an innate immune system protein in some embodiments.

The outer surface of the coated particles may not be coated with any of (i) an antibody, (ii) a carbohydrate, (iii) a peptide derived from Apolipoprotein H protein, (iv) Mannose Binding Lectin, or (v) a flocculating agent (e.g. a flocculating agent as defined in WO 03/102184) in some embodiments.

The outer surface of the coated particles may not be coated with any of (i) an antibody, (ii) a carbohydrate, (iii) an innate immune system protein or (iv) a flocculating agent (e.g. a flocculating agent as defined in WO 03/102184) in some embodiments.

The outer surface of the coated particles may not be coated with polylysine or polylysine like moieties in some embodiments.

The antibody may be a fragment or derivative of an antibody that retains antigen-specific binding function. Such fragments and derivatives include Fab fragments, ScFv, single domain antibodies, nanoantibodies, heavy chain antibodies etc.

The carbohydrate may be a monosaccharide, oligosaccharide (e.g. a disaccharide or a trisaccharide), a polysaccharide and/or a derivative thereof.

The outer surface of the coated particles may not be coated with a ligand. The outer surface of the coated particles may not be coated with a non-specific ligand (e.g. a non-specific ligand as described in WO01/53525). The outer surface of the coated particles may not be coated with a non-proteinaceous ligand (e.g. a non-proteinaceous ligand as described in WO01/53525).

The outer surface of the coated particles may be carboxylated.

The outer surface of the coated particles may be coated with streptavidin. The outer surface of the coated particles may be coated with streptavidin and not coated with a ligand.

According to all relevant aspects and embodiments of the invention, the term "sodium polyanethol sulfonate" is intended to encompass all functionally equivalent derivatives and salt forms thereof (e.g. potassium polyanethol sulfonate, magnesium polyanethol sulfonate, sodium amylo sulphate, etc.)

As used herein, "coating" or "coated" generally refers to a layer of molecules or material formed on an outermost or exposed layer of a particle. Given the coating may be partial, the coating does not have to comprise a continuous layer of molecules on an outermost or exposed layer of a particle.

Throughout this disclosure, the term "particles" and "beads" may be used interchangeably.

## BRIEF DESCRIPTION OF THE FIGURES

Figure 1 is an image of agar plates at T=0 hours (left) and T=2 hours (right) both of which show a central zone of no-growth due to inhibition by antibiotic from the sample (see Example 1).

Figure 2 is an image of blood samples and shows the extent of blood lysis for each sample-set: E-BUF, UREA, Tris+NaCl, freezing (left to right) (see Example 9).

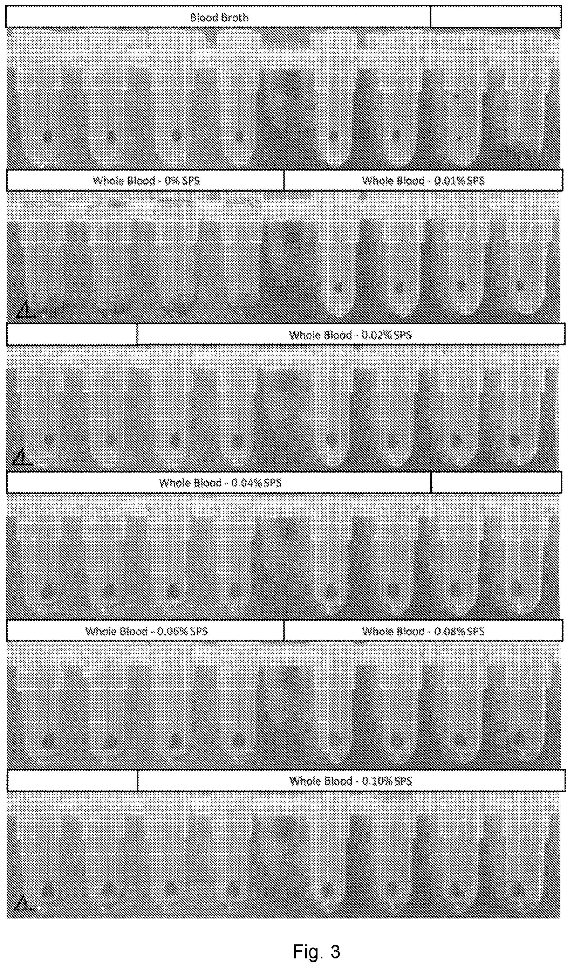

Figure 3 is an image of final sample outputs prior to PCR set-up. The image provides a visual demonstration of the benefit of SPS for sample processing with magnetic beads in blood: SPS appears to enable more thorough removal of blood components as indicated by less red eluates in the presence of SPS. Note, that the BacTec PLUS aerobic broth used for the Blood Broth sample-set also contains SPS (see Example 10).

The invention will be understood with respect to the following non-limiting examples:

## EXPERIMENTAL SECTION

**Abbreviations & definitions:**

| | |
|---|---|
| 5th% | Fifth Percentile threshold calculation to determine 5% FPR (formula = PERCENTILE.INC(array,0.05)) |
| ABX | Antibiotic or Antimicrobial |
| BO | Broth Only |
| BB | Blood Broth |
| CBA | Columbia blood agar |
| Cfu | Colony Forming Unit |
| COL | Columbia base agar |

| | |
|---|---|
| Confirm | A PCR multiplex assay targeting microbial DNA according to gram status (Gram Negative, Gram Positive or Candida) |
| CPD | Citrate Phosphate Dextrose |
| Ct | Cycle Threshold Value |
| CV | Critical Value (cfu): theoretical limit of detection based on cfu value and $\Delta$Ct using formula: sample cfu $\div$ $2^{\Delta Ct}$ |
| D1.. | Dilution point (10-fold series) |
| Dil | Dilution |
| DMBB | Detergent-free Microbial Binding Buffer |
| DWB | Detergent-free Wash Buffer |
| E-Buff | Blood lysis buffer |
| E*cfu | Extrapolated cfu value using dilution point with highest countable TVC in a dilution series |
| EC | *Escherichia coli* |
| ETGA | Enzyme Template Generation and Amplification |
| FA+ | Aerobic blood culture bottles with Resin, FA plus BioMerieuxGMBB Gentle Microbial Binding Buffer |
| IPC | Internal Process Control: PCR template present in LM to demonstrate correct sample processing and verify PCR amplification in ETGA negative samples |
| LAWN | Confluent microbial growth |
| LM | Microbial Lysis Mix containing a mixture of detergents and microbial lytic enzymes |
| MM | Master Mix |
| NoCt | No amplification above threshold fluorescence after 50 cycles |
| NSC | No Spike Control |
| O/n | Overnight |
| PC | Polymerase-spike Control |
| PCR | Polymerase Chain Reaction |
| Pt | Positivity threshold calculated from NSC/NC results |
| qPCR | Quantitative Polymerase Chain Reaction |
| RT | Room temperature (+19 to +20°C) |
| SA | Standard aerobic blood culture bottle, SA BioMerieux |
| SAB | Sabouraud dextrose agar |
| s/n | Supernatant |
| SPS | Sodium polyanethol sulfonate |
| TNTC | Too Numerous To Count |
| TVC | Total Viable Count |
| WB | Wash Buffer (containing Tris-HCl + Sodium Chloride + Igepal + Sodium Deoxycholate + Tergitol, unless otherwise stated); or Whole Blood where stated. |
| $\Delta$Ct | Difference between two Ct values (typically NSC Ct - positive sample Ct) |

## Example 1

**Aim**

To assess whether micro-organisms in clinical blood containing antibiotics can be 'rescued' and removed before being inoculated into a fresh medium for incubation. A brief investigation into whether the rescued organisms grow more efficiently compared to organisms that remain in antibiotic-containing blood.

Specifically, we aim to show that when S. *aureus* is inoculated into blood containing an inhibitory concentration of a lytic antibiotic, piperacillin, and left to incubate for two hours, the organisms can be grown by capturing them on magnetic capture beads and transferring them to a blood culture bottle with no antibiotic.

**Materials**

**Buffer compositions**

| Buffer | Volume | Reagent | Cat # |
|---|---|---|---|
| Detergent-free Microbrial Binding | 25.0 mL 8.8 g | 2M Tris-HCl [pH 8.0] NaCl | Sigma T3069 Sigma S7653 |

(continued)

| Buffer | Volume | Reagent | Cat # |
|---|---|---|---|
| Buffer - DMBB | Make up to 100 mL | Water, molecular grade | Sigma W4502 |
| Detergent-free Wash Buffer - DWB | 10 mL 90 mL | DMBB Water, molecular grade | As above Sigma W4502 |

**Method**

Test organism: *S. aureus*
Antibiotic: Piperacillin 96ug/mL blood
Human blood: from Cambridge Bioscience, Research Donors, London UK.

An overnight culture of test organism was prepared in blood/broth medium (50:50 blood:broth medium from a BioMerieux SA culture bottle).

Ten-fold serial dilutions of overnight culture was prepared in blood/broth as required. Antibiotic (abx) was added to whole human blood (in citrate-phosphate-dextrose, CPD, an anticoagulant) at required level.

Whole blood was used as the no-antibiotic control.

Test dilution(s) of organism was added to blood+antibiotic or blood only at 10 $\mu$L per mL of blood (sufficient prepared for experiment requirements).

100 $\mu$L plate count sample was taken (T0 sample).

The inoculated samples were incubated at 37 °C (static) for 2-hour.

100 $\mu$L plate count sample was taken (T2 sample).

For the control, 5 mL of spiked blood+abx or blood only was added to blood culture bottles (BioMerieux SA culture bottle) using a vial adapter (West Pharmaceutical Services, Inc. PA, US) and incubated in the automated blood culture cabinet (adapter removed).

For the 'rescue' test, 5 mL of spiked blood+abx or blood only was treated with Momentum Capture Beads (Streptavidin-coated, ~300nm beads, Bio estapor, Merk Cat# BE-M 08/0.3) as below:

- Momentum Capture Beads diluted in Detergent-free Microbial Binding Buffer (DMBB - Tris+NaCl) - 50 $\mu$L beads:700 $\mu$L DMBB per clean-up

- 750 $\mu$L of diluted beads added to 50 mL conical centrifuge tube

- 5 mL of broth medium added to tube

- 5 mL of spiked blood+abx or blood only added to tube

- Incubate on ITL TherMix (Integrated Technologies Ltd. Kent, UK), 32.5 °C / 30 min /1000rpm - ramped speed protocol

- Magnetise on V&P magnet (V&P scientific Inc, CA, US) for 5 min then remove supernatant

- Resuspend beads in 1 mL Detergent-free Wash Buffer (DWB - Tris+NaCl) off magnet and transfer to 2 mL Eppendorf flip-top tube on DynaMag-2 magnet (Thermo Fisher Scientific, Life Technologies Ltd. Paisley, UK)

- Magnetise for 5 min then remove supernatant

- Resuspend beads in 1 mL DWB off magnet then replace on DynaMag-2 magnet

- Magnetise for 5 min then remove supernatant

- Resuspend beads in 1 mL DWB, add to a fresh, unused blood culture bottle (BioMerieux SA culture bottle) containing standard blood culture growth medium using a vial adapter, and start incubation (adapter removed).

- Blood culture bottles are incubated in the automated blood culture cabinet (BacT/ALERT BioMerieux) which records time to positivity. The "Flip" time is when a bottle turns positive as determined by the automated blood culture cabinet.

- "Flip" time of each bottle is recorded.

- Plate counts (Total Viable Count TVC) are recorded.

**Results**

As can be seen in Table 1, recovered organisms from samples 4b and 5b flipped positive in culture at between 10 and 15 hours when the control blood culture remained negative at 120 hours (5 days). Only the rescued organisms continued growth to positivity where the organisms in the control blood culture were killed or inhibited by the presence of the antibiotic. Growth occurred in both samples containing no antibiotic, where one was treated as the control and one was processed with the rescue procedure.

Table 1:  Results showing recovery of *S. aureus* from antibiotic-containing blood

| Sample ID | Sample type | Organism | Org spike dil | Antibiotic: Piperacillin 96ug/mL blood | Date incubated | Flip time in Days | Flip time in hours/mins | TVC T0 | TVC T2 |
|---|---|---|---|---|---|---|---|---|---|
| 4a | Control | S. aureus | 10 -3 | Y | 09/08/2019 | - | Negative at 120h | 98 | 2 |
| 4b | Bead rescue | S. aureus | 10 -3 | Y | 09/08/2019 | 0.57 | 13h 40m | | |
| 5a | Control | S. aureus | 10 -4 | Y | 09/08/2019 | - | Negative at 120h | 15 | 0 |
| 5b | Bead rescue | S. aureus | 10 -4 | Y | 09/08/2019 | 0.62 | 14h 52m | | |
| 6a | Control | S. aureus | 10 -4 | N | 09/08/2019 | 0.34 | 8h 9m | 331 | >1000, |
| 6b | Bead rescue | S. aureus | 10 -4 | N | 09/08/2019 | 0.45 | 10h 48m | | <3000 TNTC |

In conclusion, we have demonstrated that organisms can be 'rescued' and removed from a blood sample containing antibiotics, inoculated into fresh medium and incubated until they grow to positivity. In the experiment discussed here, organisms that were not rescued and that remained in the antibiotic-containing sample did not grow and remained negative at day 5.
The following examples demonstrate the ability of a variety of coated particles to form particle-microorganism complexes in different samples and under different conditions and thereby recover viable microorganisms. Different downstream detection and characterisation methods are also exemplified.

**Example 2**

In a manual format, two bead types (Merck Bio-Estapor (streptavidin-conjugated) 300 nm beads (Product - BE-M08/03; "Bio-Estapor") and ademtech Bio- Adembeads Streptavidin Plus 200 nm beads (Product number 03222; "Bio-Ademtech")) were compared to ApoH Technologies Peps6 beads (Reference - MP20006; "ApoH Peps6") .
In experiment 1A, an aliquot of Bio-Estapor beads (25uL) and an aliquot of ApoH Pep6 beads (10uL) were compared for binding. The higher volume of Bio-Estapor reflected the lower number of beads per mL in the material provided compared to the ApoH material.
Three organisms were tested: *E. coli* (Gram negative bacterium), *S. epidermidis* (Gram positive bacterium) and *C.albicans* (yeast). 0.5mL of organism suspension was exposed to the beads in 0.5mL "TTGB" microbial binding buffer, provided in the ApoH Peps6 kit ("Peps6 Captobac", Reference MP10031-50T).
After allowing the organism to bind for 30 min, the sample of beads was separated from the liquid supernatant by applying a magnetic field to concentrate the beads and removing the supernatant with a pipette. The beads were gently washed with three aliquots of wash buffer (50mM Tris pH 8, 1% v/v Igepal CA-630, 150mM NaCl, 0.25% v/v Tergitol 15-S-9) and the retained supernatant and the washed beads were analysed for viable organisms by two methods; colony counts on an Agar Petri dish and detection of microbial DNA by the enzymatic template generation and amplification (ETGA) test (as described in Zweitzig et al., 2012. Characterization of a novel DNA polymerase activity assay enabling sensitive, quantitative and universal detection of viable microbes. Nucleic Acids Research 40, 14, e109, 1-12; and in WO2011/130584, WO2013/103744 and WO2016/005768).
The plate counts in Table 1A show that with the Bio-Estapor beads and *E.coli,* the great majority of growth is found from the beads (33 CFU) vs the supernatant (2 CFU) and this is similar to the result from ApoH Peps6. No growth was found with S. *epidermidis* and this organism did not appear to grow in the original broth. *C. albicans* showed approximately 10% of the CFU in the supernatant and 90% bound, for both Bio-Estapor and ApoH Peps6. These results indicate that the Bio-Estapor beads appear to bind organisms at an equivalent rate to the commercially available organism-binding beads Peps6 under the conditions of the test. The sensitive ETGA test supports the results but indicates that *S. epidermidis* may bind to Bio-Estapor better than Peps6 as shown by the lower Cq value.

Table 1A

| Experiment 1 A<br><br>Estapor beads at 25 µL, ApoH at 10 µL (+ve ctl) and no beads (-ve ctl) vs E. coli, S. epidermidis & C. albicans dilutions in blood broth (Manual protocol). |
| --- |

| E.c. ONC dif[n] 2 | | | 1.01E+04 cfu/mL | | E. coli |
| --- | --- | --- | --- | --- | --- |
| S. e. ONC dif[n] 2 | | | No growth cfu/ml | | Staph epidermidis |
| C. a. ONC dif[n] 1 | | | 3.60E+05 cfu/ml | | Candida albicans |
| | | | | Plate count | ETGA Results |
| Tube | Organism | ApoH | Estapor | CFU | Cq |
| Bio-Estapor beads | | | | | |
| Bio-Estapor in binding buffer (supernatant) | E. coli | | 25 µL | 2 | not tested |
| Bio-Estapor in binding buffer (bound) | | | | 33 | 32.54 |
| Bio-Estapor in binding buffer (supernatant) | S. epidermidis | | 25µL | 0 | not tested |
| Bio-Estapor in binding buffer (bound) | | | | 0 | 37.63 |
| Bio-Estapor in binding buffer (supernatant) | C. albicans | | 25 µL | 34 | not tested |
| Bio-Estapor in binding buffer (bound) | | | | 246 | 33.34 |
| ApoH Peps6 beads | | | | | |
| ApoH Peps6 in binding buffer (supernatant) | E. coli | 10 µL | | 1 | not tested |
| ApoH Peps6 in binding buffer (bound) | | | | 38 | 33.18 |
| ApoH Peps6 in binding buffer (supernatant) | S. epidermidis | 10 µL | | 0 | not tested |
| ApoH Peps6 in binding buffer (bound) | | | | 0 | 40.94 |
| ApoH Peps6 in binding buffer (supernatant) | C. albicans | 10 µL | | 35 | not tested |
| ApoH Peps6 in binding buffer (bound) | | | | ≈316 | 34.01 |
| No beads | | | | | |
| No beads | E. coli | | | 43 | not tested |
| No beads | S. epidermidis | | | 0 | not tested |
| No beads | c. albicans | | | ≈316 | not tested |
| No bugs / no beads | | | | | |
| No beads no microbes | | | | 0 | not tested |

Experiment 1B demonstrates *E. coli* binding under similar conditions to Experiment 1A although in 1B the wash steps were omitted. Experiment 1B shows that another bead, Bio-Ademtech, also binds organisms although at a lower level (see Table 1B). Here the plate counts indicate that approximately one third of the viable counts have bound to the bead. The more sensitive ETGA DNA polymerase assay indicates that half of the organisms remain on the beads, as the Cq for the beads and the supernatant are approximately equal.

Table 1B

| Experiment 1B | | | | | |
|---|---|---|---|---|---|
| AdemTech beads and ApoH (+ve ctl) and no beads & no beads/no bugs controls. | | | | | - E. |

| ONC dil$^n$ 2 | | 1.26E+04 cfu/mL | | | |
|---|---|---|---|---|---|
| | | | | Plate count | ETGA Resuts |
| Tube | AdemTech | ApoH | Estapor | CFU | Cq |
| AdemTech beads | | | | | |
| Ademtech beads supernatant | 100µL | | | 78 | 34.09 |
| Ademtech beads bound | 100µL | | | 44 | 34.02 |
| ApoH Peps6 (5µL in 95µL) | | | | | |
| ApoH Peps6 supernatant | | 5µL | | 0 | 40.16 |
| ApoH Pep6 bound | | 5µL | | 36 | 32.8 |
| No beads | | | | | |
| No beads | | | | 45 | 34.22 |
| No bugs / no beads | | | | | |
| No beads/no organisms | | | | 0 | Excluded |

## Example 3

In Experiment 3, ApoH Peps6, Bio-Estapor and Estapor beads with a carboxylated surface (Product MI-030/40; "Estapor COOH") were compared. The number of organisms remaining in the supernatant after binding of *E.coli* to the beads for 30 min was measured using a fluorescent ATP assay (BacTiter-Glo Microbial Cell Viability Assay; Promega Corporation, G8230). Although this is an indirect test in that it does not directly detect the presence of organisms on the bead, it is a useful comparative test for the ligand-based beads (ApoH Peps6) and the non-ligand beads (Bio-Estapor and Estapor COOH). After binding of 1mL of $10^4$ CFU/mL E coli for 30 mL from a phosphate saline buffer, an aliquot of the supernatant was assayed for ATP as a measure of organism content using the BacTiter-Glo assay. The results in Table 2 show that the reduction in levels of organisms in the supernatant for Peps6 beads, Bio-Estapor and Estapor COOH were 33%, 27% and 24% respectively when measured using this technique.

Table 2

| Bead Supplier (Type) | Date of assay | Time | Sample (10^4 CFU (ml E.coli) (RFU) | Baseline (No E.coli) (RFU) | Baseline corrected (RFU) | % Binding Based on Depletion of Supernatant | Signal to noise |
|---|---|---|---|---|---|---|---|
| | | | | | | | |
| ApoH (Peps6) | 06/03/2018 | am | 1896 | 345 | 1551 | 32% | 5.50 |
| ApoH (Peps6) | 06/03/2018 | am | 1856 | 345 | 1511 | 34% | 5.38 |
| ApoH (Peps6) | 06/03/2018 | pm | 2549 | 378 | 2171 | 31% | 6.74 |
| ApoH (Peps6) | 06/03/2018 | pm | 2398 | 378 | 2020 | 35% | 6.34 |
| | | | | | **Average** | **33%** | |
| | | | | | **Std Dev** | **2.1%** | |
| | | | | | **%CV** | **6.4%** | |
| | | | | | | | |
| BioEstapor (Sav) | 06/03/2018 | am | 2067 | 345 | 1722 | 24% | 5.99 |

(continued)

| Bead Supplier (Type) | Date of assay | Time | Sample (10^4 CFU (ml E.coli) (RFU) | Baseline (No E.coli) (RFU) | Baseline corrected (RFU) | % Binding Based on Depletion of Supernatant | Signal to noise |
|---|---|---|---|---|---|---|---|
| BioEstapor (Sav) | 06/03/2018 | am | 2138 | 345 | 1793 | 21% | 6.20 |
| BioEstapor (Sav) | 06/03/2018 | pm | 2447 | 378 | 2069 | 34% | 6.47 |
| BioEstapor (Sav) | 06/03/2018 | pm | 2618 | 378 | 2240 | 28% | 6.93 |
| | | | | | Average | 27% | |
| | | | | | Std Dev | 5.4% | |
| | | | | | %CV | 20.1% | |
| Estapor (COOH) | 06/03/2018 | am | 2045 | 345 | 1700 | 25% | 5.93 |
| Estapor (COOH) | 06/03/2018 | am | 2500 | 345 | 2155 | 5% | 7.25 |
| Estapor (COOH) | 06/03/2018 | pm | 2416 | 378 | 2038 | 35% | 6.39 |
| Estapor (COOH) | 06/03/2018 | pm | 2520 | 378 | 2142 | 32% | 6.67 |
| | | | | | Average | 24% | |
| | | | | | Std Dev | 13.2% | |
| | | | | | %CV | 54.3% | |
| No Beads | 06/03/2018 | am | 2578 | 345 | 2233 | N/A | 7.47 |
| No Beads | 06/03/2018 | am | 2668 | 345 | 2323 | N/A | 7.73 |
| No Beads | 06/03/2018 | pm | 3594 | 378 | 3216 | N/A | 9.51 |
| No Beads | 06/03/2018 | pm | 3418 | 378 | 3040 | N/A | 9.04 |

## Example 4

Example 4 shows results from testing *E.coli* (EC), *S. aureus* (SA) and *C. albicans* (CA) in dilution series performed by automating the method for magnetic separation described in Example 2. The assay used Bio-Estapor 300nm diameter beads as the capture medium with a binding buffer of TTGB containing 0.25% Tergitol. As 10-fold dilutions of each of the three organisms were made, so a continuous change in the Ct was recorded allowing a dose response curve to be constructed.

Table 3

| LOB Result | Ct | DF |
|---|---|---|
| ETGA FAM | 38.51 | |
| ETGA IPC | 31.22 | |
| Confirm FAM? | | 0.13 |
| Confirm HEX | | 2.13 |
| Confirm Cy5 | | 1.69 |

| Bugs: EC, SA, CA | |
|---|---|
| ETGA threshold | 4.338 |
| IPC threshold | 0.911 |

| Sample | TVCs | ETGA Ct | IPC Ct | ETGA result | Gr-Neg Ct (315) | df | Gr-Pos Ct (318) | df | Candida Ct (161) | df | Confirm Result | Confirm |
|---|---|---|---|---|---|---|---|---|---|---|---|---|
| EC.e-2 | 640,000 CFU/mL | 15.62 | 32.33 | | 18.89 | 10.29 | No Ct | 0.57 | No Ct | 0.08 | Gr-Neg | Gr-Neg |
| EC.e-3 | 64,000 CFU/mL | 20.18 | 32.20 | | 21.82 | 9.25 | No Ct | 0.69 | No Ct | 0.10 | Gr-Neg | Gr-Neg |
| EC.e-4 | 6,400 CFU/mL | 25.39 | 31.79 | | 24.00 | 8.11 | 35.20 | 1.30 | No Ct | 0.15 | Gr-Neg | Gr-Neg |
| EC.e-5 | 640 CFU/mL | 29.15 | 31.57 | | 29.56 | 3.64 | 32.22 | 3.23 | No Ct | 0.13 | ND | Gr-Neg |
| EC.e-6 | 64 CFU/mL | 34.03 | 31.59 | | 32.23 | 2.23 | 32.07 | 2.70 | No Ct | 0.03 | ND | Gr-Neg |
| EC.e-7 | | 37.32 | 31.62 | | 35.62 | 0.79 | 33.75 | 2.36 | No Ct | 0.09 | N/A | N/A |
| EC.e-8 | | 37.77 | 31.99 | | 34.55 | 1.05 | 33.52 | 2.37 | No Ct | 0.13 | N/A | N/A |
| SA.e-2 | 180,000 CFU/mL | 17.49 | 32.40 | | 33.89 | 0.04 | 16.83 | 28.51 | No Ct | 0.05 | Gr-Pos | Gr-Pos |
| SA.e-3 | 18,000 CFU/mL | 21.34 | 32.20 | | No Ct | 0.08 | 20.18 | 16.59 | No Ct | 0.02 | Gr-Pos | Gr-Pos |
| SA.e-4 | 1,800 CFU/mL | 24.75 | 31.38 | | No Ct | 0.73 | 25.37 | 16.26 | No Ct | 0.08 | Gr-Pos | Gr-Pos |
| SA.e-5 | 180 CFU/mL | 28.63 | 31.45 | | No Ct | 0.76 | 28.26 | 12.15 | No Ct | 0.15 | Gr-Pos | Gr-Pos |
| SA.e-6 | 18 CFU/mL | 35.21 | 31.95 | | No Ct | 0.54 | 32.92 | 2.05 | No Ct | 0.02 | ND | N/A |
| SA.e-7 | | 37.34 | 31.99 | | 34.15 | 0.39 | 33.45 | 1.95 | No Ct | 0.07 | N/A | N/A |
| SA.e-8 | | 37.27 | 31.91 | | 36.02 | 0.27 | 32.84 | 2.24 | No Ct | 0.01 | N/A | N/A |
| CA.e-0 | 293,000 CFU/mL | 23.99 | 32.99 | | No Ct | 0.52 | 32.31 | 3.36 | 21.50 | 7.66 | ND | Candida |
| CA.e-1 | 29,300 CFU/mL | 27.97 | 31.73 | | No Ct | 0.75 | 33.36 | 1.72 | 24.87 | 6.14 | Candida | Candida |
| CA.e-2 | 2,900 CFU/mL | 31.85 | 31.69 | | 34.44 | 0.50 | 31.73 | 4.44 | 29.33 | 2.90 | ND | Candida |
| CA.e-3 | 290 CFU/mL | 35.56 | 31.65 | | No Ct | 0.32 | 32.53 | 3.54 | 35.14 | 0.47 | ND | Candida |
| CA.e-4 | 29 CFU/mL | 36.94 | 31.52 | | No Ct | 0.29 | 34.12 | 1.50 | No Ct | 0.02 | N/A | N/A |
| CA.e-5 | 3 CFU/mL | 35.03 | 32.03 | | No Ct | 0.11 | 33.94 | 1.42 | No Ct | -0.02 | N/A | N/A |
| CA.e-6 | 0 CFU/mL | 36.96 | 31.88 | | 36.07 | 0.33 | 33.99 | 1.01 | No Ct | 0.02 | N/A | N/A |
| NSC 1 | 0 CFU/mL | 37.35 | 32.02 | | No Ct | 1.22 | 32.45 | 3.07 | No Ct | 0.10 | Gr-Pos | N/A |
| NSC 2 | 0 CFU/mL | 37.20 | 32.01 | | No Ct | 0.01 | 32.21 | 3.85 | No Ct | 0.08 | Gr-Pos | N/A |
| NSC 3 | 0 CFU/mL | 37.10 | 31.82 | | No Ct | 0.52 | 31.47 | 4.24 | No Ct | -0.02 | Gr-Pos | N/A |
| NSC Av | | 37.22 | 31.98 | | | | | | | | | |

The following examples demonstrate the universal microbial capture of microorganisms by magnetic beads in Momentum's Magnitor test. The Magnitor test consists of two microbial detection read-outs:

- **ETGA:** detection of microbial polymerase from intact microbial cells
- **Confirm:** detection of microbial DNA according to gram status (Gram Negative, Gram Positive, or Candida)

**Key findings:**

- Magnetic beads capture bacteria and fungi from simple buffers and a variety of complex biological specimen types
- Microbial capture occurs using a variety of different bead sizes (0.2 to 1.5 $\mu$m diameter beads) and surface coatings (e.g. carboxylated, hydrophobic, aminated etc)

Certain binding buffer components can improve microbial detection in the Magnitor assay, for example detergent-based lysis of blood.

**Example 5: Detection of microorganisms is dependent on capture by magnetic beads**

**Aim:**

Microbial binding performance was assessed for *E. coli, S. aureus* and *C. albicans* in a simple Tris+NaCl buffer (pH buffered with physiological salt conc. to prevent microbial osmotic shock that may occur in water only). A 'no bead control' sample-set was also included in this experiment to demonstrate that detection is dependent on the presence of magnetic beads for microbial capture.

**Magnetic bead preparation:**

Estapor beads (Merck, Cat# M1-30/40) washed 3 x 1 mL in 1X Tris+NaCl buffer: 40 $\mu$L beads resuspended in a final volume of 400 $\mu$L 1X Tris+NaCl buffer (1% solid content)

**Protocol:**

- Microorganism overnight liquid cultures (o/n) set-up as standard in BacTec PLUS aerobic broth (inoculation of 3 mL broth from agar plate). The following day (approx. 16 hours later) 1.88 $\mu$L *E. coli and S. aureus* liquid culture added to 3 mL broth, and 18.75 $\mu$L *C. albicans* liquid culture added to 3 mL broth; and 2-hour outgrowth performed at 37°C, 500 rpm.
- Following 2-hour outgrowth, microorganism precultures diluted (DF10) in 1X Tris+NaCl buffer (50 mM Tris-HCl [pH8.0] + 150 mM NaCl) to create four dilution points per microorganism.
- 100 $\mu$L TVCs performed for each microbial dilution

Manual simulation of Magnitor performed using DynaMag-2 magnet and manual liquid transfers:

- 1 mL samples added to 2 mL tubes containing 15 $\mu$L prewashed beads - Note, 112 $\mu$L 1X Tris+NaCl buffer not added to tube with beads (as per standard protocol), because all microbial samples were diluted in the same 1X buffer.
- 30 mins shaking (1000 rpm) @ 37°C
- 5 mins magnetisation on DynaMag-2
- All s/n removed
- 1 mL Wash Buffer (WB) added and tubes mixed for 2 mins @ RT (1000 rpm)
- 3 mins magnetisation on Dynmag-2
- All s/n removed
- 50 $\mu$L Lysis Mix (LM) added to tubes off magnet (5 $\mu$L Polymerase Control (PC) added to each PC sample tube)
- ETGA reaction performed: 5 mins at 1000 rpm, then 55 mins at 800 rpm @26°C
- Manual qPCR set-up for ETGA and Confirm (10 $\mu$L reactions)

**Results:**

TVCs (COL/SAB agar plates)

|  | Colonies | *E cfu/mL |
|---|---|---|
| *E. coli D1* | TNTC | 216,000 |
| *E. coli D2* | TNTC | 21,600 |
| *E. coli D3* | *216 | 2,160 |
| *E. coli D4* | 26 | 216 |
| *S. aureus D1* | *671 | 6,710 |
| *S. aureus D2* | 121 | 671 |
| *S. aureus D3* | 33 | 67 |
| *S. aureus D4* | 0 | 7 |
| *C. albicans D1* | *51 | 510 |
| *C. albicans D2* | 6 | 51 |
| *C. albicans D3* | 8 | 5 |
| *C. albicans D4* | 0 | 1 |
| NSC | 0 | - |

*E cfu/mL values derived from highest countable TVC plate*

ETGA Ct

|  | *E. coli* | | *S. aureus* | | *C. albicans* | |
|---|---|---|---|---|---|---|
|  | (+) BEADS | (-) BEADS | (+) BEADS | (-) BEADS | (+) BEADS | (-) BEADS |
| D1 | 12.64 | 26.25 | 18.99 | 29.29 | 26.57 | 30.61 |
| D2 | 18.09 | 29.02 | 24.28 | 32.40 | 30.08 | 33.62 |
| D3 | 22.75 | 32.11 | 28.61 | 32.70 | 33.27 | 33.12 |
| D4 | 30.52 | 31.01 | 30.85 | 31.56 | 30.80 | 31.65 |
| NSC1 | 35.26 | 32.22 | 35.73 | 31.37 | 34.69 | 31.31 |
| NSC2 | 35.67 | 33.13 | 35.61 | 35.13 | 35.29 | 32.40 |
| NSC3 | 34.92 | 32.38 | 35.28 | 31.54 | 34.31 | 31.77 |
| PC | 32.10 | 30.79 | 31.40 | 29.64 | 31.69 | 29.34 |
| Average NSC | 35.28 | 32.58 | 35.54 | 32.68 | 34.76 | 31.83 |

Internal Process Control (IPC) Ct

|  | *E. coli* | | *S. aureus* | | *C. albicans* | |
|---|---|---|---|---|---|---|
|  | (+) BEADS | (-) BEADS | (+) BEADS | (-) BEADS | (+) BEADS | (-) BEADS |
| D1 | 37.36 | 31.51 | 32.61 | 32.20 | 32.32 | 31.95 |
| D2 | 32.85 | 32.11 | 32.26 | 32.38 | 32.10 | 32.26 |
| D3 | 32.44 | 32.10 | 31.98 | 32.19 | 31.82 | 33.10 |
| D4 | 32.15 | 32.01 | 32.10 | 32.15 | 31.68 | 32.15 |
| NSC1 | 32.20 | 32.15 | 32.26 | 31.95 | 32.12 | 32.05 |

(continued)

|  | E. coli | | S. aureus | | C. albicans | |
|---|---|---|---|---|---|---|
|  | (+) BEADS | (-) BEADS | (+) BEADS | (-) BEADS | (+) BEADS | (-) BEADS |
| NSC2 | 32.24 | 32.40 | 32.13 | 33.38 | 32.09 | 32.20 |
| NSC3 | 32.17 | 32.33 | 32.18 | 32.39 | 32.26 | 32.20 |
| PC | 32.30 | 32.62 | 32.18 | 32.51 | 32.50 | 32.24 |

ETGA ACt (averageNSC)

|  | E. coli | | S. aureus | | C. albicans | |
|---|---|---|---|---|---|---|
|  | (+) BEADS | (-) BEADS | (+) BEADS | (-) BEADS | (+) BEADS | (-) BEADS |
| D1 | 22.64 | 6.33 | 16.55 | 3.39 | 8.19 | 1.22 |
| D2 | 17.19 | 3.56 | 11.26 | N/A | 4.69 | N/A |
| D3 | 12.54 | 0.47 | 6.93 | N/A | 1.49 | N/A |
| D4 | 4.77 | 1.57 | 4.69 | N/A | 3.97 | N/A |
| NSC1 | N/A | N/A | N/A | N/A | N/A | N/A |
| NSC2 | N/A | N/A | N/A | N/A | N/A | N/A |
| NSC3 | N/A | N/A | N/A | N/A | N/A | N/A |
| PC | 3.18 | 1.79 | 4.14 | 3.04 | 3.08 | 2.48 |

Critical Values based on average NSC (cfu/mL)

|  | E. coli | | S. aureus | | C. albicans | |
|---|---|---|---|---|---|---|
|  | (+) BEADS | (-) BEADS | (+) BEADS | (-) BEADS | (+) BEADS | (-) BEADS |
| D1 | 0.03 | 2692.80 | 0.07 | 641.54 | 1.74 | 218.60 |
| D2 | 0.14 | 1836.42 | 0.27 | N/A | 1.98 | N/A |
| D3 | 0.36 | 1557.76 | 0.55 | N/A | 1.81 | N/A |
| D4 | 7.93 | 72.82 | 0.26 | N/A | 0.03 | N/A |
| NSC1 | N/A | N/A | N/A | N/A | N/A | N/A |
| NSC2 | N/A | N/A | N/A | N/A | N/A | N/A |
| NSC3 | N/A | N/A | N/A | N/A | N/A | N/A |
| PC | N/A | N/A | N/A | N/A | N/A | N/A |

Confirm Ct

|  | E. coli (GrNeg) | | S. aureus (GrPos) | | C. albicans (Candida) | |
|---|---|---|---|---|---|---|
|  | (+) BEADS | (-) BEADS | (+) BEADS | (-) BEADS | (+) BEADS | (-) BEADS |
| D1 | 24.26 | 42,44 | 28.13 | NoCt | 35.08 | NoCt |
| D2 | 27.83 | 32.08 | 31.65 | NoCt | 39.30 | 43.21 |
| D3 | 33.17 | NoCt | 35.79 | NoCt | NoCt | NoCt |
| D4 | NoCt | NoCt | 36.71 | NoCt | NoCt | NoCt |
| NSC1 | NoCt | NoCt | NoCt | 41.51 | NoCt | 48.84 |

(continued)

| | E. coli (GrNeg) | | S. aureus (GrPos) | | C. albicans (Candida) | |
| --- | --- | --- | --- | --- | --- | --- |
| | (+) BEADS | (-) BEADS | (+) BEADS | (-) BEADS | (+) BEADS | (-) BEADS |
| NSC2 | 40.57 | NoCt | NoCt | NoCt | NoCt | NoCt |
| NSC3 | NoCt | NoCt | NoCt | 40.49 | NoCt | NoCt |
| PC | NoCt | NoCt | 43.24 | NoCt | NoCt | NoCt |
| Positivity threshold (Pt) $\leq$ 40 Ct | | | | | | |

**Analysis:**

- Magnitor results for '(+) BEADS' samples show a very strong cell-density specific ETGA and Confirm signal for all three microorganism species, demonstrating bead-specific binding of a broad range of microorganism groups (GrNeg, GrPos, Candida).
- Note, that Candida results could have followed a better cell density trend, but the liquid culture was quite particulate which may have affected the quality of serial dilutions

Some evidence of microbial cell carryover in the '(-) BEADS' controls, but this is to be expected with only a single wash step.

**Example 6: Microbial capture from blood by magnetic beads occurs in simple and complex blood lysis buffers, and allows microbial detection comparable to capture by centrifugation**

**Aim:**

To compare two different blood lysis buffers in two different diluent formats for development of a simple and fast 'Rapid Magnitor' test (no wash step included in protocol).

**Test Conditions:**

- 2X EBB      1 mL 2X EBB + 1 mL Specimen
- 10X EBB      112 $\mu$L 10X EBB + 1 mL Specimen
- 2X B-BUF      1 mL 2X B-BUF + 1 mL Specimen
- 10X B-BUF      112 $\mu$L 10X B-BUF + 1 mL Specimen

- 10X EBB: 500 mM Tris-HCl [pH 8.0] + 2.5% Tergitol
- 10X B-BUF: 500 mM Tris-HCl [pH 8.0] + 1.5 M Sodium Chloride + 10% Igepal + 5% Sodium Deoxycholate + 2.5% Tergitol

Sample set-up:

- *E. coli* o/n liquid culture 1E-3 dilution spiked into blood broth (6.25 $\mu$L o/n per mL: 244 $\mu$L o/n + 39 mL blood broth) and 60 minute out-growth performed in shaking incubator @ 37°C, 500 rpm
- Following 2-hour outgrowth, samples produced by adding 1 mL specimen to 2 mL tube containing buffer (and 15 $\mu$L BioEstapor beads (Merck, Cat# BE-M 08/0.3) for Mag Beads sample-set): triplicate *E. coli* (EC) and No Spike Control (NSC) samples per test condition
- 100 $\mu$L TVCs performed for NSC and *E. coli* (including dilutions of specimen to ensure countable plates)

**Protocol:**

Samples set up as above, and progressed immediately to Spin or Mag Beads protocol

Spin Protocol

- Samples centrifuged for 3 minutes at 9000 xg (tube hinges facing outwards for pellet traceability)
- Supernatants removed
- 50 μL LM added to samples (approx. 10x pipette mixes to resuspend pellets)
- Samples placed in shaking incubator at 900 rpm for 5 minutes and then 800 rpm for 55 minutes (26°C)
- Centrifuge samples at 17000 xg for 1 minute before qPCR set-up

Mag Beads Protocol

- Samples placed in shaking incubator at 900 rpm for 30 minutes (37°C)
- Samples placed on DynaMag-2 magnetic rack for 5 minutes and then supernatants removed
- 50 μL LM added to samples (approx. 10x pipette mixes to resuspend pellets)
- Samples placed in shaking incubator at 900 rpm for 5 minutes and then 800 rpm for 55 minutes (26°C)
- Magnetise samples for 3 minutes before qPCR set-up
  Manual qPCR set-up (10 μL reactions) for ETGA mastermix only

**Results:**

**cfu calculation**

Sample and dilutions of sample plated on COL plates (100 μL)

|  | **TVC** | ***cfu/mL** |
|---|---|---|
| *E. coli* 1E-3 | TNTC | 68500 |
| *E. coli* 1E-4 | 685 | 6850 |
| *E. coli* 1E-5 | 89 | 685 |
| NSC | 0 | 0 |

Sample source

**ETGA Ct**

|  |  | **Centrifugation** | | | | **Magnetic Beads** | | | |
|---|---|---|---|---|---|---|---|---|---|
|  | cfu/sample | 2XEBB | 10XEBB | 2XB-BUF | 10XB-BUF | 2XEB8 | 10XEB8 | 2XB-BUF | 10X8-BUF |
| ***E. coli* 1** | 68,500 | 15.89 | 16.94 | 21.43 | 22.83 | 15.10 | 17.12 | 20.78 | 22.61 |
| ***E. coli* 2** | 68,500 | 15.63 | 16.87 | 22.72 | 21.82 | 15.35 | 17.50 | 21.07 | 23.21 |
| ***E. coli* 3** | 68,500 | 16.05 | 16.85 | 20.04 | 22.20 | 15.63 | 17.73 | 21.77 | 23.20 |
| **NSC1** | - | 23.72 | 25.48 | 47.72 | NoCt | 29,52 | 34.45 | 45.73 | 44.89 |
| **NSC2** | - | 24.51 | 25.01 | 43.55 | 46.85 | 30.02 | 34.30 | 46.30 | 44.26 |
| **NSC3** | - | 24.16 | 25.74 | NoCt | 44.62 | 30.08 | 34.21 | 48.06 | 45.06 |

**Summary data**

| | cfu/ sample | Centrifugation | | | | Magnetic Beads | | | |
|---|---|---|---|---|---|---|---|---|---|
| | | 2XEBB | 10XEBB | 2XB-BUF | 10XB-8UF | 2XEBB | 10XEBB | 2XB-BUF | 10XB-BUF |
| Ave. *E. coil* ETGA Ct | 68,500 | 15.86 | 16.89 | 21.40 | 22.28 | 15.38 | 17.45 | 21.21 | 23.01 |
| Ave.NSC ETGA Ct | - | 24.13 | 25.41 | 45.64 | 45.73 | 29.88 | 34.32 | 46.70 | 44.74 |
| Ave. ETGA △Ct | 68,500 | 8.27 | 8.52 | 24.24 | 23.45 | 14.49 | 16.87 | 25.49 | 21.73 |

**Analysis:**

- Microbial binding by magnetic beads occurs in:

  ○ Simple and complex blood lysis buffers (EBB = Tris-HCl + Tergitol; B-BUF = Tris-HCl + Sodium Chloride + Igepal + Sodium Deoxycholate + Tergitol), but blood-derived test signal varies depending on blood lysis buffer components
  ○ Diluted (2X buffer: 1-part blood lysis buffer to 1-part specimen) and concentrated (10X buffer: 1-part blood lysis buffer to 9 parts specimen) sample formats

Furthermore, microbial detection signal for microbial capture by magnetic beads is comparable to capture by centrifugation.

**Example 7: Microbial capture from blood by magnetic beads is not dependent on blood lysis, but downstream microbial detection is improved when microbial binding occurs in lysed blood**

**Aim:**

Given the recent discovery that multiple bead types/sizes produce similar Magnitor results for microbial serial dilutions and NSCs, it was thought that a component within Momentum's binding buffer might be mediating/facilitating this observed universal microbial binding character. To investigate this possibility, a dilution series of *E. coli* was performed comparing the standard binding buffer (B-BUF) with a detergent-free B-BUF consisting of just Tris-HCl [pH8.0] + NaCl, to test whether the detergents in general are important for microbial binding. Sample-sets were prepared for blood-broth, broth-only and in 1X binding buffer only to compare results for different specimen types.

**Preparation:**

100 mL 10X Binding Buffers prepared fresh:

- **B-BUF:** 500 mM Tris-HCl [pH 8.0] + 1.5 M Sodium Chloride + 10% Igepal + 5% Sodium Deoxycholate + 2.5% Tergitol
- **Tris+NaCl:** 500 mM Tris-HCl [pH 8.0] + 1.5 M Sodium Chloride

Estapor beads (Merck, Cat# M1-30/40) washed 3 x 1 mL in respective 1X buffer (diluted 10X B-BUF or 10X Tris+NaCl): 40 $\mu$L beads resuspended in a final volume of 400 $\mu$L 1X buffer (1% solid content)

**Protocol:**

- *E. coli* o/n liquid culture set-up as standard in BacTec PLUS aerobic broth, then following day (approx. 16 hours later) 1.88 μL o/n added to 3 mL broth (equivalent to EC 1E-1 dilution added to broth at 6.25 μL/mL) and 2-hour outgrowth performed at 37°C, 500 rpm.
- Following 2-hour outgrowth, *E. coli* preculture serially diluted (DF10) down to EC 1E-6 in either prewarmed blood-broth (BB), broth only (BO) or 1X buffer (B-BUF or Tris+NaCl).
- 100 μL TVCs performed for all *E. coli* dilutions and NSCs

Manual simulation of Magnitor performed using DynaMag-2 magnet and manual liquid transfers:

- 1 mL samples added to 2 mL tubes containing 112 μL of binding buffer (either B-BUF or Tris+NaCl: 10X for BB and BO sample-sets; and 1X for Buffer sample-sets) + 15 μL beads (prewashed in respective buffer)
- 30 mins shaking (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 50 μL LM added to tubes off magnet (5 μL Polymerase Control (PC) added to each PC sample tube)
- ETGA reaction performed: 5 mins at 1000 rpm, then 55 mins at 800 rpm @26°C
- Manual qPCR set-up for ETGA and Confirm (10 μL reactions)

*Observations:*

- No blood lysis observed for Tris+NaCl, as expected
- Beads more grainy/aggregated in absence of detergents

**Results:**

| 10X Binding Buffer | BB | | BO | | 1X B-BUF | | Tris+NaCl | |
|---|---|---|---|---|---|---|---|---|
| Specimen | TVC | *E cfu/mL | TVC | *E cfu/mL | TVC | *E cfu/mL | TVC | *E cfu/mL |
| E. coli 1E-3 | TNTC | 74800 | TNTC | 52000 | TNTC | 47600 | TNTC | 54700 |
| E. coli 1E-4 | *748 | 7480 | *520 | 5200 | *476 | 4760 | *547 | 5470 |
| E. coli 1E-5 | 88 | 748 | 70 | 520 | 45 | 476 | 57 | 547 |
| E. coli 1E-6 | 6 | 75 | 5 | 52 | 2 | 48 | 5 | 55 |
| NSC | 0 | 0 | 0 | 0 | 0 | 0 | 0 | 0 |
| *Cfu/mL values extrapolated from highest countable TVC ETGA Ct | | | | | | | | |

| 10X Binding | B-BUF | | | Tris+NaCl | | |
|---|---|---|---|---|---|---|
| Specimen | BB | BO | B-BUF | BB | BO | Tris+NaCl |
| E. coli 1E-3 | 22.84 | 21.63 | 18.93 | 24.71 | 17.78 | 14.91 |
| E. coli 1E-4 | 27.32 | 26.16 | 25.08 | 29.70 | 21.58 | 20.14 |
| E. coli 1E-5 | 31.40 | 30.03 | 29.31 | 33.58 | 25.38 | 24.53 |
| E. coli 1E-6 | 36.87 | 32.91 | 33.62 | 34.97 | 29.36 | 31.24 |
| NSC 1 | 44.80 | 35.23 | 34.71 | 36.04 | 38.81 | 33.73 |
| NSC 2 | 43.10 | 34.86 | 35.26 | 35.21 | 38.83 | 34.89 |
| NSC 3 | 44.11 | 34.54 | 36.72 | 35.41 | 38.56 | 34.19 |
| PC | 34.84 | 28.06 | 31.37 | 34.08 | 32.78 | 30.55 |
| Average NSC | 44.00 | 34.87 | 35.56 | 35.55 | 38.74 | 34.27 |

IPC Ct

| 10X Binding Specimen | B-BUF | | | Tris+NaCl | | |
|---|---|---|---|---|---|---|
| | BB | BO | B-BUF | BB | BO | Tris+NaCl |
| E. coli 1E-3 | 33.89 | 31.43 | 32.14 | 33.59 | 32.15 | 33.36 |
| E. coli 1E-4 | 34.41 | 31.22 | 31.67 | 33.63 | 31.66 | 31.68 |
| E. coli 1E-5 | 33.44 | 31.27 | 31.41 | 32.85 | 31.35 | 31.27 |
| E. coli 1E-6 | 33.27 | 31.34 | 31.46 | 33.16 | 31.11 | 31.49 |
| NSC 1 | 34.06 | 31.29 | 31.43 | 33.26 | 31.34 | 31.30 |
| NSC 2 | 35.07 | 31.53 | 31.69 | 33.18 | 31.50 | 31.51 |
| NSC 3 | 33.79 | 31.29 | 31.54 | 33.25 | 31.11 | 31.77 |
| PC | 34.22 | 31.29 | 32.18 | 33.34 | 31.37 | 31.65 |

ETGA ΔCt (average NSC)

| 10X Binding Specimen | B-BUF | | | Tris+NaCl | | |
|---|---|---|---|---|---|---|
| | BB | BO | B-SUF | BB | BO | Tris+NaCl |
| E. coli 1E-3 | 21.16 | 13.25 | 16.63 | 10.85 | 20.95 | 13.35 |
| E. coli 1E-4 | 16.68 | 8.71 | 10.49 | 5.86 | 17.15 | 14.13 |
| E. coli 1E-5 | 12.61 | 4.84 | 6.25 | 1.97 | 13.35 | 9.74 |
| E. coli 1E-6 | 7.14 | 1.97 | 1.94 | 0.58 | 9.38 | 3.03 |
| NSC 1 | N/A | N/A | N/A | N/A | N/A | N/A |
| NSC 2 | N/A | N/A | N/A | N/A | N/A | N/A |
| NSC 3 | N/A | N/A | N/A | N/A | N/A | N/A |
| PC | 9.16 | 6.82 | 4.20 | 1.47 | 5.96 | 3.72 |

Critical Values based on average NSC (cfu/mL)

| 10X Binding Bu Specimen | B-BUF | | | Tris+NaCl | | |
|---|---|---|---|---|---|---|
| | BB | BO | B-BUF | BB | BO | Tris+NaCl |
| E. coil 1E-3 | 0.03 | 5.34 | 0.47 | 40.63 | 0.03 | 0.08 |
| E. coli 1E-4 | 0.07 | 12.40 | 3.32 | 129.20 | 0.04 | 0.31 |
| E. coli 1E-5 | 0.12 | 18.10 | 6.25 | 190.59 | 0.05 | 0.64 |
| E. coli 1E-6 | 0.53 | 13.30 | 12.42 | 50.03 | 0.08 | 6.69 |
| NSC 1 | N/A | N/A | N/A | N/A | N/A | N/A |
| NSC 2 | N/A | N/A | N/A | N/A | N/A | N/A |
| NSC 3 | N/A | N/A | N/A | N/A | N/A | N/A |
| PC | N/A | N/A | N/A | N/A | N/A | N/A |

Confirm GrNeg Ct

| 10X Binding Specimen | B-BUF | | | Tris+NaCl | | |
|---|---|---|---|---|---|---|
| | BB | BO | B-BUF | BB | BO | Tris+NaCl |
| *E. coli* 1E-3 | 31.36 | NoCt | 28.13 | 30.53 | 31.60 | 25.94 |
| *E. coli* 1E-4 | 36.41 | NoCt | 30.86 | 35.72 | 38.16 | 26.69 |
| *E. coli* 1E-5 | 37.67 | NoCt | NoCt | NoCt | 39.26 | 34.62 |
| *E. coli* 1E-6 | NoCt | NoCt | NoCt | NoCt | NoCt | NoCt |
| NSC 1 | NoCt | NoCt | NoCt | 42.97 | NoCt | NoCt |
| NSC 2 | NoCt | NoCt | NoCt | NoCt | 39.64* | NoCt |
| NSC 3 | NoCt | NoCt | NoCt | 49.52 | NoCt | NoCt |
| PC | NoCt | NoCt | NoCt | NoCt | NoCt | NoCt |

Positivity threshold (Pt) $\leq$ 40 Ct; * false positives

**Analysis:**

- Detergents are important for producing good ETGA results in the presence of blood (as indicated by poorer ETGA results for '10X Tris+NaCl with BB'), but microbial capture/detection is still evident in the absence of blood lysis (as demonstrated by results for '10X Tris+NaCl with BB' sample-set).
- Good ETGA results in the absence of blood, indicate that detergents, as components of the binding buffer, are not necessary for binding of *E. coli* to beads
- Good ETGA results in the '10X Tris+NaCl with 1X Tris+NaCl' sample-set demonstrate that biological components in blood and/or broth are not required for microbial binding.
- Interestingly, the B-BUF appears to be slightly inhibitory to ETGA signal in 10X B-BUF with BO and 1X B-BUF sample-sets, but recent work elsewhere has shown that Sodium Deoxycholate could be somewhat inhibitory to the assay, so this observation is not unexpected
- Confirm performed best in the '10X Tris+NaCl with 1X Tris+NaCl' sample-set. All other similar sample-sets produced similar Confirm GrNeg results.
- IPC signal was somewhat inhibited by the presence of blood, as can be expected.
- These results demonstrate that neither detergents or the biological sample are mediators of microbial binding for *E. coli*

**Example 8: In the absence of blood, microbial capture by magnetic beads occurs regardless of any pH buffering or osmotic stabilisation with salt**

**Aim**:

To further investigate the importance of Momentum's binding buffer in mediating microbial binding the effect of pH buffering and salt on binding was investigated in a clean system (i.e. in the absence of any blood or broth).

**10X buffer preparation:**

25 mL of each buffer made fresh:

- BUF-1 500 mM Tris-HCl [pH7.4] + 1.5 M NaCl
- BUF-2 500 mM Tris-HCl [pH8.0] + 1.5 M NaCl
- BUF-3 500 mM Tris-HCl [pH8.5] + 1.5 M NaCl
- BUF-4 500 mM Tris-HCl [pH8.0] ONLY
- BUF-5 1.5 M NaCl ONLY
- BUF-6 Water ONLY

Estapor beads (Merck, Cat M1-30/40) washed 3 x 1 mL in respective 1X buffer (diluted 10X buffers): 30 $\mu$L beads resuspended in a final volume of 300 $\mu$L 1X buffer (1% solid content)

**Protocol:**

- E.coli o/n liquid cultures set-up as standard in BacTec PLUS aerobic broth (containing SPS) and Nutrient Broth (NB containing no SPS), then the following day (approx. 16 hours later) 1.88 $\mu$L o/n added to 3 mL broth (equivalent to EC 1E-1 dilution added to broth at 6.25 $\mu$L/mL) for each broth type (NB in morning and PLUS broth in afternoon), and 2-hour outgrowth incubations performed at 37°C, 500 rpm.

- For each experiment (NB and PLUS), 1E-1 *E. coli* preculture diluted down to *E. coli* 1E-6 (DF10) in each 1X buffer (BUF-1 to BUF-6)

- 100 $\mu$L TVCs performed using a separate EC dilution set performed in relevant broth (NB or PLUS broth) to prevent plate viability inconsistencies resulting from different 1X buffers

Manual simulation of Magnitor performed using DynaMag-2 magnet and manual liquid transfers:

- 1 mL samples added to 2 mL tubes containing 112 $\mu$L of respective 1X buffer + 15 $\mu$L beads (prewashed in respective buffer)
- 30 mins shaking (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 50 $\mu$L LM added to tubes off magnet
- ETGA reaction performed: 5 mins at 1000 rpm, then 55 mins at 800 rpm @26°C
- Manual qPCR set-up for ETGA and Confirm (10 $\mu$L reactions)

**Results:**

**NB *dataset (i.e.* No SP5) - *morning experiment***

|  | Colonies | *E cfu/mL |
| --- | --- | --- |
| *E. coli* 1e-3b | TNTC | 35000 |
| *E. coli* 1e-4 | *350 | 3500 |
| *E. coli* 1e-5 | 12 | 350 |
| *E. coli* 1e-6 | 5 | 35 |

*All buffer (BUF-1 to 6) NC TVCs = 0*

| | Colonies | *E cfu/mL | ETGA Ct | | | | | |
|---|---|---|---|---|---|---|---|---|
| | | | BUF-1 | BUF-2 | BUF-3 | BUF-4 | BUF-5 | BUF-6 |
| *E. coli* 1e-3 | TNTC | 35000 | 19.00 | 18.44 | 17.73 | 17.45 | 19.12 | 16.28 |
| *E. coli* 1e-4 | *350 | 3500 | 22.60 | 21.23 | 21.53 | 21.16 | 22.51 | 26.22 |
| *E. coli* 1e-5 | 12 | 350 | 25.57 | 26.94 | 28.18 | 24.77 | 27.51 | 28.71 |
| *E. coli* 1e-6 | 5 | 35 | 30.41 | 30.47 | 31.37 | 29.92 | 31.36 | 33.81 |
| NSC 1 | 0 | 0 | 40.37 | 39.45 | 39.70 | 40.18 | 39.22 | 38.16 |
| NSC 2 | 0 | 0 | 40.63 | 36.20 | 39.74 | 38.03 | 40.01 | 38.64 |
| NSC 3 | 0 | 0 | 39.76 | 39.36 | 39.09 | 39.67 | 38.47 | 38.17 |
| PC | N/A | N/A | 30.24 | 31.51 | 33.06 | 32.57 | 32.90 | 32.08 |
| NSC Ave. | | | 40.25 | 38.34 | 39.51 | 39.30 | 39.23 | 38.32 |


| | Colonies | *E cfu/mL | IPC Ct | | | | | |
|---|---|---|---|---|---|---|---|---|
| | | | BUF-1 | BUF-2 | BUF-3 | BUF-4 | SUF-5 | BUF-6 |
| *E. coli* 1e-3b | TNTC | 35000 | 32,17 | 32.36 | 32.32 | 32.30 | 32.05 | 32.31 |
| *E. coli* 1e-4 | *350 | 3500 | 31.58 | 31.62 | 31.83 | 31.67 | 31.55 | 31.28 |
| *E. coli* 1e-5 | 12 | 350 | 31.44 | 31.65 | 31.44 | 31.27 | 31.67 | 31.62 |
| *C. coli* 1e-6 | 5 | 35 | 31.37 | 31.47 | 31.48 | 31.31 | 31.64 | 31.50 |
| NSC 1 | 0 | 0 | 31.60 | 31.25 | 31.42 | 31.64 | 31.48 | 31.54 |
| NSC 2 | 0 | 0 | 31.53 | 31.33 | 31.60 | 31.40 | 31.75 | 31.52 |
| NSC 3 | 0 | 0 | 31.42 | 31.95 | 31.37 | 31.43 | 31.76 | 31.67 |
| PC | N/A | N/A | 31.20 | 31.49 | 31.65 | 31.49 | 31.57 | 31.97 |


| | Colonies | *E cfu/mL | ETGA ACt | | | | | |
|---|---|---|---|---|---|---|---|---|
| | | | BUF-1 | BUF-2 | SUF-3 | BUF-4 | BUF-5 | BUF-6 |
| *E. coli* 1e-3b | TNTC | 35000 | 21.26 | 19.89 | 21.78 | 21.85 | 20.11 | 22.05 |
| *E. coli* 1e-4 | *350 | 3500 | 17.65 | 17.10 | 17.38 | 18.13 | 16.73 | 12.10 |
| *E. coli* 1e-5 | 12 | 350 | 14.68 | 11.40 | 11.34 | 14.53 | 11.72 | 9.61 |
| *E. coli* 1e-6 | 5 | 35 | 9.85 | 7.86 | 8.14 | 9.38 | 7.87 | **4.51** |
| NSC 1 | 0 | 0 | N/A | N/A | N/A | N/A | N/A | N/A |
| NSC 2 | 0 | 0 | N/A | N/A | N/A | N/A | N/A | N/A |
| NSC 3 | 0 | 0 | N/A | N/A | N/A | N/A | N/A | N/A |
| PC N/A | N/A | N/A | 10.02 | 6.83 | 6.45 | 6.72 | 6.33 | 6.24 |


| | Colonies | *E cfu/mL | ETGA Critical Values (averageNSC) | | | | | |
|---|---|---|---|---|---|---|---|---|
| | | | BUF-1 | BUF-2 | BUF-3 | BUF-4 | SUF-5 | BUF-6 |
| *E. coli* 1e-3b | TNTC | 35000 | 0.014 | 0.036 | 0.010 | 0.009 | 0.031 | 0.008 |
| *E. coli* 1e-4 | *350 | 3500 | 0.017 | 0.025 | 0.014 | 0.012 | 0.032 | 0.795 |
| *E. coli* 1e-5 | 12 | 350 | 0.013 | 0.130 | 0.135 | 0.015 | 0.104 | 0.448 |
| *E. coli* 1e-6 | 5 | 35 | 0.038 | 0.150 | 0.124 | 0.053 | 0.149 | 1.535 |
| NSC 1 | 0 | 0 | N/A | N/A | N/A | N/A | N/A | N/A |
| NSC 2 | 0 | 0 | N/A | N/A | N/A | N/A | N/A | N/A |
| NSC3 | 0 | 0 | N/A | N/A | N/A | N/A | N/A | N/A |
| PC | N/A | N/A | N/A | N/A | N/A | N/A | N/A | N/A |

|  | Colonies | *E cfu/ml | BUF-1 | BUF-2 | Confirm GrNeg Ct BUF-3 | BUF-4 | BUF-5 | BUF-6 |
|---|---|---|---|---|---|---|---|---|
| *E. coli* 1e-3b | TNTC | 35000 | 25.07 | 25.28 | 25.98 | 25.77 | 25.05 | 27.70 |
| *E. coli* 1e-4 | *350 | 3500 | 28.36 | 28.58 | 28.31 | 30.15 | 30.62 | NoCt |
| *E. coli* 1e-5 | 12 | 350 | 31.75 | 31.16 | 31.82 | 32.25 | 38.38 | NoCt |
| *E. coli* 1e-6 | 5 | 35 | NoCt | NoCt | 34.94 | NoCt | NoCt | NoCt |
| NSC 1 | 0 | 0 | NoCt | NoCt | NoCt | NoCt | NoCt | NoCt |
| NSC 2 | 0 | 0 | NoCt | NoCt | NoCt | NoCt | NoCt | NoCt |
| NSC 3 | 0 | 0 | NoCt | NoCt | NoCt | NoCt | NoCt | NoCt |
| PC | N/A | N/A | NoCt | NoCt | NoCt | NoCt | NoCt | NoCt |
| Positivity threshold (Pt) ≤ 40 Ct | | | | | | | | |

*PLUS dataset (i.e with SPS) - afternoon experiment*

|  | Colonies | *E cfu/mL |
|---|---|---|
| *E. coli* 1e-3b | TNTC | 55600 |
| *E. coil* 1e-4 | *556 | 5560 |
| *E. coli* 1e-5 | 77 | 556 |
| *E. coil* 1e-6 | 7 | 55.6 |

*All buffer (BUF-1 to 6) NC TVCs = 0*

|  | Colonies | *E cfu/mL | BUF-1 | BUF-2 | ETGA Ct BUF-3 | BUF-4 | BUF-5 | BUF-6 |
|---|---|---|---|---|---|---|---|---|
| *E. coli* 1e-3 | TNTC | 55600 | 17.30 | 16.32 | 15.48 | 18.29 | 19.15 | 17.61 |
| *E. coli* 1e-4 | *556 | 5560 | 21.89 | 21.09 | 20.37 | 22.37 | 24.39 | 26.13 |
| *E. coli* 1e-5 | 77 | 556 | 26.82 | 24.63 | 26.19 | 26.30 | 29.27 | 34.26 |
| *E. coli* 1e-6 | 7 | 55.6 | 31.76 | 30.71 | 32.60 | 31.57 | 34.20 | 34.68 |
| NSC 1 | 0 | 0 | 37.74 | 39.10 | 38.95 | 41.09 | 41.03 | 41.46 |
| NSC 2 | 0 | 0 | 38.25 | 38.48 | 38.90 | 39.11 | 40.58 | 40.84 |
| NSC 3 | 0 | 0 | 39.46 | 38.90 | 38.03 | 42.26 | 40.81 | 41.02 |
| PC | N/A | N/A | 33.25 | 32.41 | 32.62 | 33.85 | 33.49 | 33.26 |
| NSC Ave. | | | 38.48 | 38.83 | 38.63 | 40.82 | 40.81 | 41.11 |

|  | Colonies | *E cfu/mL | BUF-1 | BUF-2 | IPC Ct BUF-3 | BUF-4 | BUF-5 | BUF-6 |
|---|---|---|---|---|---|---|---|---|
| *E. coli* 1e-3 | TNTC | 55600 | 32.46 | 33.44 | 32.97 | 33.31 | 31.98 | 33.00 |
| *E. coli* 1e-4 | *556 | 5560 | 31.77 | 32.07 | 32.00 | 32.25 | 31.42 | 31.64 |
| *E. coli* 1e-5 | 77 | 556 | 32.01 | 32.03 | 31.96 | 31.70 | 31.21 | 31.15 |
| *E. coli* 1e-6 | 7 | 55.6 | 31.96 | 31.65 | 31.75 | 31.55 | 31.55 | 31.63 |
| NSC 1 | 0 | 0 | 31.84 | 32.11 | 31.99 | 31.13 | 31.58 | 31.65 |
| NSC 2 | 0 | 0 | 31.90 | 31.51 | 32.08 | 31.25 | 31.46 | 31.64 |
| NSC 3 | 0 | 0 | 31.94 | 31.84 | 31.68 | 31.49 | 31.44 | 31.59 |

(continued)

| | | | IPC Ct | | | | | |
|---|---|---|---|---|---|---|---|---|
| | Colonies | *E cfu/mL | BUF-1 | BUF-2 | BUF-3 | BUF-4 | BUF-5 | BUF-6 |
| PC | N/A | N/A | 32.17 | 32.24 | 32.34 | 31.73 | 31.37 | 31.87 |

| | | | ETGA △Ct | | | | | |
|---|---|---|---|---|---|---|---|---|
| | Colonies | *E cfu/mL | BUF-1 | BUF-2 | BUF-3 | BUF-4 | BUF-5 | BUF-6 |
| *E. coli* 1e-3b | TNTC | 55600 | 21.18 | 22.50 | 23.15 | 22.53 | 21.66 | 23.50 |
| *E. coli* 1e-4 | *556 | 5560 | 16.59 | 17.73 | 18.26 | 18.45 | 16.41 | 14.98 |
| *E. coli* 1e-5 | 77 | 556 | 11.66 | 14.20 | 12.44 | 14.52 | 11.54 | 6.85 |
| *E. coli* 1e-6 | 7 | 55.6 | 6.72 | 8.12 | 6.03 | 9.25 | 6.61 | 6.43 |
| NSC 1 | 0 | 0 | N/A | N/A | N /A | N/A | N/A | N/A |
| NSC 2 | 0 | 0 | N/A | N/A | N/A | N/A | N/A | N/A |
| NSC 3 | 0 | 0 | N/A | N/A | N/A | N/A | N/A | N/A |
| PC | N/A | N/A | 5.23 | 6.41 | 6.01 | 6.97 | 7.31 | 7.85 |

| | | | ETGA Critical Values (averageNSC) | | | | | |
|---|---|---|---|---|---|---|---|---|
| | Colonies | *E cfu/mL | BUF-1 | BUF-2 | BUF-3 | BUF-4 | BUF-5 | BUF-6 |
| *E. coli* 1e-3b | TNTC | 55600 | 0.023 | 0.009 | 0.006 | 0.009 | 0.017 | 0.005 |
| *E. coli* 1e-4 | *556 | 5560 | 0.056 | 0.026 | 0.018 | 0.016 | 0.064 | 0.172 |
| *E. coli* 1e-5 | 77 | 556 | 0.172 | 0.030 | 0.100 | 0.024 | 0.187 | 4.827 |
| *E. coli* 1e-6 | 7 | 55.6 | 0.528 | 0.200 | 0.849 | 0.092 | 0.569 | 0.644 |
| NSC 1 | 0 | 0 | N/A | N/A | N/A | N/A | N/A | N/A |
| NSC 2 | 0 | 0 | N/A | N/A | N/A | N/A | N/A | N/A |
| NSC 3 | 0 | 0 | N/A | N/A | N/A | N/A | N/A | N/A |
| PC | N/A | N/A | N/A | N/A | N/A | N/A | N/A | N/A |

| | | | Confirm GrNeg Ct | | | | | |
|---|---|---|---|---|---|---|---|---|
| | Colonies | *E cfu/mL | BUF-1 | BUF-2 | BUF-3 | BUF-4 | BUF-5 | BUF-6 |
| *E. coli* 1e-3 | TNTC | 55600 | 28.12 | 27.49 | 26.73 | 28.36 | 28.13 | 27.82 |
| *E. coli* 1e-4 | *556 | 5560 | 32.91 | 27.92 | 27.85 | 30.15 | 30.85 | NoCt |
| *E. coli* 1e-5 | 77 | 556 | 34.43 | 34.83 | 38.66 | 43.88 | NoCt | NoCt |
| *E. coli* 1e-6 | 7 | 55.6 | NoCt | NoCt | NoCt | NoCt | NoCt | NoCt |
| NSC 1 | 0 | 0 | NoCt | NoCt | NoCt | NoCt | NoCt | NoCt |
| NSC 2 | 0 | 0 | NoCt | NoCt | NoCt | NoCt | NoCt | NoCt |
| NSC 3 | 0 | 0 | NoCt | NoCt | NoCt | NoCt | NoCt | NoCt |
| PC | N/A | N/A | NoCt | NoCt | NoCt | NoCt | NoCt | NoCt |
| Positivity threshold (Pt) S 40 Ct | | | | | | | | |

**Analysis:**

- All buffers, including water only, demonstrated capture of *E. coli* similarly well (as indicated by similar ETGA and Confirm results) - however, there was some indication of osmotic microbial lysis at lower cell densities in water only (BUF-6)

- Both PLUS broth and NB grown *E. coli* produced very similar Magnitor results for all buffers tested, indicating that SPS plays no obvious role in mediating microbial binding of *E. coli*
- These results indicate that no buffer components are essential for binding of *E. coli* to Estapor (carboxylated) beads

## Example 9: Microbial capture from blood by magnetic beads can be performed using a variety of different blood lysis methods

**Aim:**

To determine whether microbial capture and detection can occur when alternative blood lysis methods are employed.

**Preparation:**

Binding Buffers were prepared as follows:

- **E-BUF** = 500 mM Tris-HCl [pH 8.0] + 1.5 M Sodium Chloride + 10% Igepal + 2.5% Tergitol
- UREA = 83 mM Tris-HCl [pH 8.0] + 10 M Urea
- Tris+NaCl = 500 mM Tris-HCl [pH 8.0] + 1.5 M Sodium Chloride

BioEstapor beads (Merck, Cat# BE-M 08/0.3) were re-suspended prior to use.

Protocol:

- S. *aureus* o/n liquid culture set-up as standard in BacTec PLUS aerobic broth, then following day (approx. 16 hours later) 3.0 μL o/n added to 3 mL blood broth (1E-3 dilution) and 4-hour outgrowth performed at 37°C, 500 rpm.
- Following the 4-hour outgrowth, S. *aureus* pre-culture serially diluted (DF10) down to 1E-6 in prewarmed blood broth.
- 100 μL TVCs performed for all S. *aureus* dilutions and NSCs

Manual sample processing using DynaMag-2 magnet and manual liquid transfers by pipette:

**Initial set-up**

- For samples using Urea, 0.25 mL specimens added to 2 mL tubes containing 0.75 mL of UREA + 15 μL beads
- For samples to be frozen, 1 mL specimens added to 2 mL tubes then snap frozen on dry ice for 5 minutes. The specimens were thawed at 37°C for 5 minutes, then 112 μL Tris+NaCl + 15 μL beads were added
- For samples using E-BUF or Tris+NaCl, 1 mL specimens added to 2 mL tubes containing 112 μL of binding buffer (either E-BUF or Tris+NaCl) + 15 μL beads

**Processing of all samples**

- 30 mins orbital mixing (1000 rpm) @ 37°C
- 5 mins magnetisation on DynaMag-2
- All s/n removed
- 1 mL WB added and tubes mixed for 3 mins @ 37°C (1000 rpm)
- 5 mins magnetisation on Dynmag-2
- All s/n removed
- 50 μL LM added to tubes off magnet
- ETGA reaction performed: 5 mins at 1000 rpm, then 55 mins at 800 rpm @26°C
- Manual qPCR set-up for ETGA and Confirm (10 μL reactions)

*Observations:*

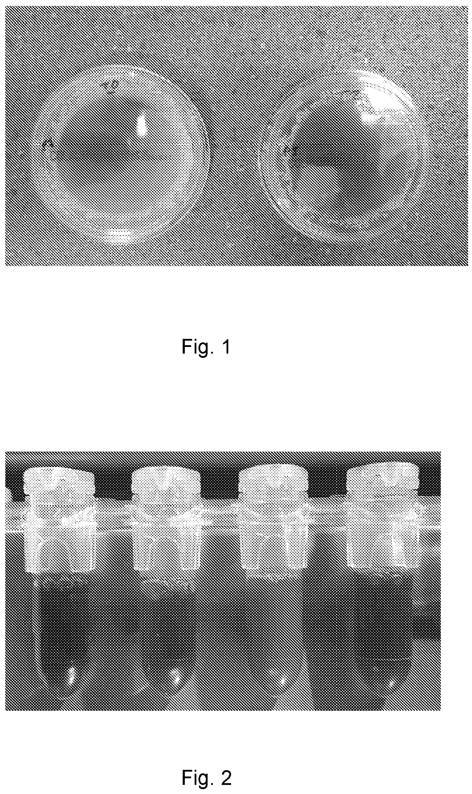

- Blood lysis observed for the frozen samples after thawing (see Figure 2)
- Blood lysis observed to be almost instantaneous with UREA
- Some beads appeared to be lost during processing for the samples using UREA
- No apparent blood lysis observed for Tris+NaCl sample-set (as expected)

**Results:**

| Specimen | TVC | *E cfu/mL |
|---|---|---|
| S. aureus 1E-4 | TNTC | 3,360,000 |
| S. aureus 1E-5 | TNTC | 336,000 |
| S. aureus 1E-6 | *3360 | 33,600 |
| NSC | 0 | 0 |
| *Cfu/mL values extrapolated from highest countable TVC | | |

ETGA Ct

| | BLOOD LYSIS METHOD | | | |
|---|---|---|---|---|
| Specimen | E-BUF | UREA | Tris+NaCl | FREEZING |
| S. aureus 1E-4 | 14.26 | 34.98 | 13.90 | 14.42 |
| S. aureus 1E-5 | 18.50 | 30.26 | 17.79 | 18.99 |
| S. aureus 1E-6 | 22.59 | 35.96 | 22.07 | 22.96 |
| NSC 1 | 42.11 | 37.55 | 32.56 | 35.14 |
| NSC 2 | 41.13 | 36.52 | 32.72 | 34.60 |
| NSC 3 | 43.83 | 36.71 | 32.42 | 34.58 |
| Average NSC | 42.36 | 36.92 | 32.57 | 34.77 |

ETGA ΔCt (average NSC)

| | BLOOD LYSIS METHOD | | | |
|---|---|---|---|---|
| Specimen | E-BUF | UREA | Tris+NaCl | FREEZING |
| S. aureus 1E-4 | 28.10 | 1.94 | 18.67 | 20.35 |
| S. aureus 1E-5 | 23.85 | 6.66 | 14.78 | 15.78 |
| S. aureus 1E-6 | 19.77 | 0.96 | 10.49 | 11.82 |
| NSC 1 | N/A | N/A | N/A | N/A |
| NSC 2 | N/A | N/A | N/A | N/A |
| NSC 3 | N/A | N/A | N/A | N/A |

Critical Values based on average NSC (cfu/mL)

| | BLOOD LYSIS METHOD | | | |
|---|---|---|---|---|
| *Specimen* | **E-BUF** | **UREA** | **Tris+NaCl** | **FREEZING** |
| *S. aureus* 1E-4 | 0.01 | 875527.09 | S.β6 | 2.52 |
| *S. aureus* 1E-5 | 0.02 | 3313.90 | 11.96 | 5.96 |
| *S. aureus* 1E-6 | 0.04 | 17241.36 | 23.34 | 9.31 |
| NSC 1 | N/A | N/A | N/A | N/A |
| NSC 2 | N/A | N/A | N/A | N/A |
| NSC 3 | N/A | N/A | N/A | N/A |

IPC Ct

| | BLOOD LYSIS METHOD | | | |
|---|---|---|---|---|
| *Specimen* | **E-BUF** | **UREA** | **Tris+NaCl** | **FREEZING** |
| *S. aureus* 1E-4 | 35.68 | 36.10 | 37.31 | 38.12 |
| *S. aureus* 1E-5 | 34.49 | 36.18 | 35.39 | 35.54 |
| *S. aureus* 1E.6 | 34.22 | 35.76 | 35.12 | 34.50 |
| NSC 1 | 34.40 | 36.51 | 34.39 | 34.87 |
| NSC 2 | 34.18 | 35.86 | 34.52 | 34.09 |
| NSC 3 | 34.85 | 36.31 | 34.25 | 34.04 |

Confirm GrPos Ct

| | BLOOD LYSIS METHOD | | | |
|---|---|---|---|---|
| *Specimen* | **E-BUF** | **UREA** | **Tris+NaCl** | **FREEZING** |
| S. *aureus* 1E-4 | 22.34 | 23.47 | 18.74 | 19.55 |
| S. *aureus* 1E-5 | 26.03 | 26.33 | 21.19 | 23.03 |
| S. *aureus* 1E-6 | 27.67 | 30.92 | 24.73 | 26.37 |
| NSC 1 | 46.59 | 35.42* | NoCt | 48.06 |
| NSC 2 | 40.33 | NoCt | NoCt | NoCt |
| NSC 3 | NoCt | NoCt | 45.99 | 41.75 |

Positivity threshold (Pt) ≤ 40 Ct; * false positives

Figure 2 shows the extent of blood lysis for each sample-set: E-BUF, UREA, Tris+NaCl, freezing (left to right)

**Analysis:**

- Microbial capture and detection of S. *aureus* by magnetic beads is comparable for alternative lysis methods and no blood lysis, as determined by Confirm. However, microbial detection by ETGA is improved to differing extents by alternative blood lysis methods, due to effects on the reduction of blood-derived ETGA signal.

**Example 10: SPS is needed for optimal bead performance, sample processing and microbial detection in whole blood**

**Aim:**

To determine the optimal SPS concentration for the Magnitor Rapid test using 1 mL whole blood samples. The secondary objective was to assess the effect of SPS on microbial viability in whole blood as determined by TVCs.

**Test conditions:**

2 × 5 mL whole blood or BacTec PLUS aerobic blood broth (1:3 ratio) aliquoted for each sample-set (E. *coli* and NSC sample). Then SPS added as follows:

**Sample-set 10% SPS (μL)**

| | |
|------|------|
| BB | None |
| WB 0% | None |
| WB 0.01% | 5 |
| WB 0.02% | 10 |
| WB 0.04% | 20 |
| WB 0.06% | 30 |
| WB 0.08% | 40 |
| WB 0.10% | 50 |

Then 5 μL of E. *coli* 1E-2 preculture added to each 5-mL specimen tube to recreate standard 1E-5 dilution sample

**Protocol:**

- 1.88 μL neat o/n in Nutrient Broth (NB) added to 3 mL NB; and incubated for 2 hours at 37°C, 500 rpm
- After 2 hours, E. *coli* preculture diluted 10-fold in warm NB; and then 5 μL added to each 5 mL specimen tube (prepared as shown in test conditions)
- Magnitor Test initiated immediately, and TVCs performed as detailed below.

Manual simulation of Magnitor performed using DynaMag-2 magnet and manual liquid transfers:

- 112 μL E-BUF (500 mM Tris-HCl [pH 8.0] + 1.5 M Sodium Chloride + 10% Igepal + 2.5% Tergitol) + 15 μL Beads (BioEstapor, Merck, Cat# BE-M 08/0.3) preloaded into each sample-tube, then 1 mL specimens added to sample tubes
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 μL LM added to tubes off magnet
- ETGA reaction performed: 5 mins at 1000 rpm, then 55 mins at 800 rpm @26°C
- Manual qPCR set-up for ETGA and Confirm (10 μL reactions)

**Results:**

TVC analysis

- 100 µL on COL plates at Time Zero
- Sample bijous, containing approximately 2 mL sample, left at room temperature (20.4°C) on bench (static)
- TVCs performed at time points shown in table: samples mixed thoroughly before plating

| | WB SPS % (colonies) | | | | | | | |
|---|---|---|---|---|---|---|---|---|
| | BB | 0 | 0.01 | 0.02 | 0.04 | 0.06 | 0.08 | 0.1 |
| EC Time ZERO | 263 | 303 | 147 | 96 | 354 | 128 | 322 | 126 |
| EC Time 2hrs | 428 | 256 | 498 | 448 | 1106 | 760 | 491 | 341 |
| EC Time 4hrs | TNTC | 790 | TNTC | TNTC | TNTC | TNTC | TNTC | TNTC |
| EC Time 6hrs | TNTC | TNTC | TNTC | TNTC | TNTC | TNTC | TNTC | TNTC |
| EC Time 21hrs | LAWN | LAWN | LAWN | LAWN | LAWN | LAWN | LAWN | LAWN |
| NSC Time ZERO | 0 | 0 | N/A | N/A | N/A | N/A | N/A | N/A |
| NSC Time 2hrs | 0 | 0 | N/A | N/A | N/A | N/A | N/A | N/A |
| NSC Time 4hrs | 0 | 0 | N/A | N/A | N/A | N/A | N/A | N/A |
| NSC Time 6hrs | 0 | 0 | N/A | N/A | N/A | N/A | N/A | N/A |
| NSC Time 21hrs | 0 | 0 | N/A | N/A | N/A | N/A | N/A | N/A |
| FOLD INCR. 0.2hr | 1.63 | 0.84 | 3.39 | 4.67 | 3.12 | 5.94 | 1.52 | 2.71 |

**Magnitor Rapid test performed at Time ZERO**

ETGA results

| | WB SPS % | | | | | | | |
|---|---|---|---|---|---|---|---|---|
| | BB | 0 | 0.01 | 0.02 | 0.04 | 0.06 | 0.08 | 0.1 |
| EC 1 | 26.94 | *28.72 | 27.90 | 26.41 | 29.31 | 28.70 | 32.31 | 33.76 |
| EC 2 | 27.31 | 45.22 | 29.12 | 25.88 | 29.43 | 28.50 | 31.94 | 34.37 |
| EC 3 | 27.34 | 41.32 | 28.14 | 27.24 | 29.70 | 27.62 | 31.40 | 34.08 |
| NSC 1 | 39.06 | 43.71 | 33.39 | 33.20 | 38.93 | 44.62 | 50.00 | 50.00 |
| NSC 2 | 39.75 | 44.30 | 33.06 | 33.77 | 39.93 | 44.11 | 50.00 | 50.00 |
| NSC 3 | 40.28 | 46.58 | 32.81 | 34.41 | 38.54 | 43.30 | 50.00 | 50.00 |
| Ave. EC | 27.20 | 43.27 | 28.39 | 26.51 | 29.48 | 28.27 | 31.88 | 34.07 |
| Ave. NSC | 39.70 | 44.86 | 33.09 | 33.79 | 39.13 | 44.01 | 50.00 | 50.00 |
| Ave. ΔCt | 12.50 | 1.59 | 4.70 | 7.28 | 9.65 | 15.74 | 18.12 | 15.93 |
| CV (cfu/mL) | 0.45 | 1003.27 | 56.59 | 6.16 | 4.40 | 0.02 | 0.01 | 0.02 |

*Note: NoCt changed to 50 Ct for analysis; *Outlier excluded due to substantial pellet loss during processing*

IPC results

| | WB SPS % | | | | | | |
| | BB | 0 | 0.01 | 0.02 | 0.04 | 0.06 | 0.08 | 0.1 |
|---|---|---|---|---|---|---|---|---|
| EC 1 | 34.03 | 42.50 | 42.19 | 40.42 | 37.87 | 35.81 | 38.14 | 37.06 |
| EC 2 | 34.83 | NoCt | 39.34 | 39.01 | 38.38 | 35.70 | 36.80 | 37.11 |
| EC 3 | 34.47 | NoCt | 40.49 | 37.85 | 38.34 | 36.11 | 36.80 | 37.66 |
| NSC 1 | 34.15 | NoCt | 39.75 | 39.26 | 36.56 | 36.10 | 36.80 | 37.17 |
| NSC 2 | 34.00 | NoCt | 39.12 | 38.40 | 37.81 | 35.98 | 37.87 | 38.60 |
| NSC 3 | 34.18 | 45.91 | 40.58 | 37.32 | 36.90 | 36.36 | 38.09 | 37.46 |
| Ave. NSC | 34.11 | 45.91 | 39.81 | 38.33 | 37.09 | 36.15 | 37.59 | 37.74 |

Confirm GrNeg results

| | WB SPS % | | | | | | |
| | BB | 0 | 0.01 | 0.02 | 0.04 | 0.06 | 0.08 | 0.1 |
|---|---|---|---|---|---|---|---|---|
| EC 1 | 32.33 | 30.05 | 27.94 | 29.15 | 28.71 | 31.58 | 29.20 | 29.27 |
| EC 2 | 31.45 | 34.24 | 29.18 | 28.16 | 28.75 | 29.77 | 29.80 | 28.65 |
| EC 3 | 31.71 | 34.97 | 28.32 | 29.75 | 29.82 | 29.26 | 28.87 | 29.61 |
| NSC 1 | NoCt | NoCt | NoCt | NoCt | NoCt | NoCt | NoCt | 47.82 |
| NSC 2 | 43.07 | NoCt | NoCt | NoCt | NoCt | NoCt | NoCt | NoCt |
| NSC 3 | 47.16 | NoCt | NoCt | NoCt | NoCt | NoCt | NoCt | NoCt |
| Ave. EC | 31.83 | 33.08 | 28.48 | 29.02 | 29.09 | 30.20 | 29.29 | 29.18 |

**Analysis:**

- SPS indicates a benefit of providing microbial protection/viability in whole blood based on TVC assay; but no major issues with *E. coli* viability in whole blood generally.
- Incorporation of SPS improved sample processing efficacy and microbial detection performance for both ETGA and Confirm readouts
- SPS at 0.06% produced the best results for TVC-based viability; ETGA detection (best results taking *E. coli* and NSC sample Cts into account); and PCR inhibition as indicated by IPC Ct values. Confirm GrNeg results were also improved by SPS addition to whole blood, but the exact concentration of SPS was less critical.

**Example 11: Microbial capture from blood by magnetic beads occurs using a variety of commercially available carboxylated bead products of similar size (-300 nm diameter)**

**Aim:**

To compare alternative carboxylated magnetic beads of similar size using Momentum's Magnitor assay

**Test conditions:**

| I.D | Bead | Size ($\mu$m) |
|---|---|---|
| A | PS-MAG-COOH (microparticles GmbH #S2003) | 0.27 |
| B | Sphero Carboxyl magnetic particles (Spherotech #CM-025-10H) | 0.1 - 0.4 |
| C | SuperMag Carboxylic Acid Beads (Ocean Nanotech #SC0201) | 0.2 |

(continued)

| I.D | Bead | Size (μm) |
|---|---|---|
| D | Carboxyl-Adembeads (Ademtech #02120) | 0.2 |
| E | Carboxyl beads (FG Beads #TAS8848N1140) | 0.2 |
| G | Bio-Estapor (Merck #BE-M08/03) - streptavidin-conjugated | 0.3 |

**Protocol:**

*E. coli* and *S. pyogenes* o/n liquid cultures set up as standard in 3 mL broth and blood broth (BacTec PLUS aerobic) respectively, and incubated for 16-20 hours (37°C)

The following day:

- *E. coli* liquid culture diluted to 1E-3 in blood broth and then spiked into blood broth (6.25 μL per mL blood broth); and pre-incubated for 1hr 30mins (37°C)
- *S. pyogenes* liquid culture diluted to 1E-1 in blood broth and then spiked into blood broth (6.25 μL per mL blood broth); and pre-incubated for 2hr 30mins (37°C)

*E. coli experiment performed in morning*

- *E. coli* 1E-3 pre-culture serially diluted in blood broth to produce 5 dilution points (1E-3 to 1E-7)
- Samples set up by adding 1 mL specimen to 2 mL tubes preloaded with 15 μl 1% solid beads + 112 μL Binding Buffer; and Magnitor V4.0 test performed: 5 dilution points + 3 NSCs (8 sample-set) per bead type with three bead types tested on each epMotion 5073m

*S. pyogenes experiment performed in afternoon*

- *S. pyogenes* 1E-3 pre-culture serially diluted in blood broth to produce 5 dilution points (1E-1 to 1E-5)
- Samples set up by adding 1 mL specimen to 2 mL tubes preloaded with 15 μl 1% solid beads + 112 μL Binding Buffer; and Magnitor V4.0 test performed: 5 dilution points + 3 NSCs (8 sample-set) per bead type with three bead types tested on each epMotion 5073m

Magnitor V4.0 protocol (automated sample processing on epMotion 5073m)

- 30 mins orbital mixing (1000 rpm) @ 37°C
- 15 mins magnetisation
- 1 mL s/n removed
- 0.82 mL WB added to tubes whilst beads magnetised
- 1 mL s/n removed
- 50 μL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 μL reactions)

**Results:**

Internal Positivity Thresholds (Pt) calculated using NSCs (n= 6) for each bead type: formula = PERCENTILE.INC(array,0.05)

| | A | B | C | D | E | G |
|---|---|---|---|---|---|---|
| **NSC1** | 35.12 | 37.10 | 41.11 | 45.29 | 43.55 | 40.33 |

(continued)

|        | A     | B     | C     | D     | E     | G     |
|--------|-------|-------|-------|-------|-------|-------|
| NSC2   | 35.00 | 38.85 | 42.73 | 46.18 | 43.15 | 40.37 |
| NSC3   | 34.57 | 37.02 | 40.49 | 50.00 | 42.32 | 41.79 |
| NSC4   | 33.77 | 38.25 | 41.20 | 44.74 | 43.82 | 43.80 |
| NSC5   | 33.72 | 38.22 | 40.52 | 45.25 | 45.08 | 44.12 |
| NSC6   | 35.06 | 41.19 | 41.93 | 44.33 | 43.32 | 42.39 |
| **Pt (5th%)** | **33.74** | **37.04** | **40.49** | **44.44** | **42.53** | **40.34** |

*Note, that Pt (5th%) method will generate one false positive within n=6: highlighted in red font within main results table below*

_E. coli_

_E. coli_

| | | TVCs | *E CFU/ml | ETGA Ct | Result | IPC Ct | GrNeg Ct | GrPos Ct | Cand. Ct | I.D. |
|---|---|---|---|---|---|---|---|---|---|---|
| | | | | **ETGA** | | | | **Confirm** | | |
| A | _E. coli_ 1e-3 | TNTC | 28,800 | 27.20 | Positive | 35.15 | 30.99 | NoCt | NoCt | GrNeg |
| | _E. coli_ 1e-4 | *288 | 2,880 | 31.30 | Positive | 34.91 | NoCt | NoCt | NoCt | No ID |
| | _E. coli_ 1e-5 | 35 | 288 | 33.31 | Positive | 35.08 | NoCt | NoCt | NoCt | No ID |
| | _E. coli_ 1e-6 | 4 | 29 | 35.47 | Negative | 35.37 | NoCt | NoCt | NoCt | No ID |
| | _E. coli_ 1e-7 | 0 | 3 | 34.63 | Negative | 35.09 | NoCt | NoCt | NoCt | No ID |
| | NSC1 | 0 | - | 35.12 | Negative | 35.67 | NoCt | NoCt | NoCt | No ID |
| | NSC2 | 0 | - | 35.00 | Negative | 35.10 | NoCt | NoCt | NoCt | No ID |
| | NSC3 | 0 | - | 34.57 | Negative | 35.27 | NoCt | NoCt | NoCt | No ID |
| B | _E. coli_ 1e-3 | TNTC | 28,800 | 25.67 | Positive | 34.45 | 36.51 | NoCt | NoCt | GrNeg |
| | _E. coli_ 1e-4 | *288 | 2,880 | 31.31 | Positive | 34.02 | NoCt | NoCt | NoCt | No ID |
| | _E. coli_ 1e-5 | 35 | 288 | 32.92 | Positive | 34.74 | NoCt | NoCt | NoCt | No ID |
| | _E. coli_ 1e-6 | 4 | 29 | 36.56 | Positive | 34.26 | NoCt | NoCt | NoCt | No ID |
| | _E. coli_ 1e-7 | 0 | 3 | 38.68 | Negative | 34.46 | NoCt | NoCt | NoCt | No ID |
| | NSC1 | 0 | - | 37.10 | Negative | 33.86 | NoCt | NoCt | NoCt | No ID |
| | NSC2 | 0 | - | 38.85 | Negative | 34.28 | NoCt | NoCt | NoCt | No ID |
| | NSC3 | 0 | - | 37.02[†] | Positive | 33.95 | 40.26 | NoCt | NoCt | No ID |
| C | _E. coli_ 1e-3 | TNTC | 28,800 | 23.59 | Positive | 35.38 | 29.32 | NoCt | NoCt | GrNeg |
| | _E. coli_ 1e-4 | *288 | 2,880 | 27.50 | Positive | 35.16 | 34.29 | NoCt | NoCt | GrNeg |
| | _E. coli_ 1e-5 | 35 | 288 | 31.34 | Positive | 35.16 | 42.48 | NoCt | NoCt | No ID |
| | _E. coli_ 1e-6 | 4 | 29 | 32.91 | Positive | 35.29 | NoCt | NoCt | NoCt | No ID |
| | _E. coli_ 1e-7 | 0 | 3 | 40.91 | Negative | 35.61 | NoCt | NoCt | NoCt | No ID |
| | NSC1 | 0 | - | 41.11 | Negative | 34.63 | NoCt | NoCt | NoCt | No ID |
| | NSC2 | 0 | - | 42.73 | Negative | 35.09 | 49.10 | NoCt | NoCt | No ID |
| | NSC3 | 0 | - | 40.49[†] | Positive | 35.58 | NoCt | NoCt | NoCt | No ID |
| D | _E. coli_ 1e-3 | TNTC | 28,800 | 24.41 | Positive | 35.51 | 31.63 | NoCt | NoCt | GrNeg |
| | _E. coli_ 1e-4 | *288 | 2,880 | 28.00 | Positive | 34.72 | NoCt | NoCt | NoCt | No ID |
| | _E. coli_ 1e-5 | 35 | 288 | 32.33 | Positive | 35.39 | NoCt | NoCt | NoCt | No ID |
| | _E. coli_ 1e-6 | 4 | 29 | 33.46 | Positive | 34.66 | NoCt | NoCt | NoCt | No ID |
| | _E. coli_ 1e-7 | 0 | 3 | 48.92 | Negative | 35.09 | NoCt | NoCt | NoCt | No ID |
| | NSC1 | 0 | - | 45.29 | Negative | 35.40 | NoCt | NoCt | NoCt | No ID |
| | NSC2 | 0 | - | 46.18 | Negative | 35.11 | NoCt | NoCt | NoCt | No ID |
| | NSC3 | 0 | - | 50.00 | Negative | 34.70 | NoCt | NoCt | NoCt | No ID |
| E | _E. coli_ 1e-3 | TNTC | 28,800 | 22.77 | Positive | 35.70 | 30.68 | NoCt | NoCt | GrNeg |
| | _E. coli_ 1e-4 | *288 | 2,880 | 27.16 | Positive | 35.49 | 39.05 | NoCt | NoCt | GrNeg |
| | _E. coli_ 1e-5 | 35 | 288 | 33.84 | Positive | 34.91 | NoCt | NoCt | NoCt | No ID |
| | _E. coli_ 1e-6 | 4 | 29 | 42.64 | Negative | 35.16 | NoCt | NoCt | NoCt | No ID |
| | _E. coli_ 1e-7 | 0 | 3 | 42.40 | Positive | 35.16 | NoCt | NoCt | NoCt | No ID |
| | NSC1 | 0 | - | 43.55 | Negative | 35.30 | NoCt | NoCt | NoCt | No ID |
| | NSC2 | 0 | - | 43.15 | Negative | 34.55 | NoCt | NoCt | NoCt | No ID |
| | NSC3 | 0 | - | 42.32[†] | Positive | 34.19 | NoCt | NoCt | NoCt | No ID |
| G | _E. coli_ 1e-3 | TNTC | 28,800 | 22.91 | Positive | 36.48 | 32.84 | NoCt | NoCt | GrNeg |
| | _E. coli_ 1e-4 | *288 | 2,880 | 27.54 | Positive | 35.61 | NoCt | NoCt | NoCt | No ID |
| | _E. coli_ 1e-5 | 35 | 288 | 31.36 | Positive | 35.86 | 42.94 | NoCt | NoCt | No ID |
| | _E. coli_ 1e-6 | 4 | 29 | 41.03 | Negative | 35.44 | NoCt | NoCt | NoCt | No ID |
| | _E. coli_ 1e-7 | 0 | 3 | 42.24 | Negative | 35.45 | NoCt | NoCt | NoCt | No ID |
| | NSC1 | 0 | - | 40.33[†] | Positive | 35.78 | NoCt | 43.85 | NoCt | No ID |
| | NSC2 | 0 | - | 40.37 | Negative | 35.31 | NoCt | NoCt | NoCt | No ID |
| | NSC3 | 0 | - | 41.79 | Negative | 36.90 | NoCt | 38.72[†] | NoCt | GrPos |

Confirm Positivity threshold (Pt) ≤ 40 Ct; false positives[†]

*S. pyogenes*

| Group | | TVCs | *E CFU/ml | ETGA Ct | Result | IPC Ct | GrNeg Ct | GrPos Ct | Cand. Ct | I.D. |
|---|---|---|---|---|---|---|---|---|---|---|
| | | | | ETGA | | | | Confirm | | |
| A | S. pyogenes 1e-1 | TNTC | 2,210,000 | 26.20 | Positive | 34.95 | NoCt | 27.74 | NoCt | GrPos |
| | S. pyogenes 1e-2 | TNTC | 221,000 | 30.05 | Positive | 34.52 | NoCt | 31.28 | NoCt | GrPos |
| | S. pyogenes 1e-3 | TNTC | 22,100 | 32.49 | Positive | 34.77 | NoCt | 35.19 | NoCt | GrPos |
| | S. pyogenes 1e-4 | *221 | 2,210 | 33.67 | Positive | 35.15 | NoCt | NoCt | NoCt | No ID |
| | S. pyogenes 1e-5 | 3 | 221 | 33.30 | Positive | 34.33 | NoCt | NoCt | NoCt | No ID |
| | NSC1 | 0 | -- | 33.77 | Negative | 35.22 | NoCt | NoCt | NoCt | No ID |
| | NSC2 | 0 | - | 33.72† | Positive | 35.09 | NoCt | NoCt | NoCt | No ID |
| | NSC3 | 0 | - | 35.06 | Negative | 34.93 | NoCt | NoCt | NoCt | No ID |
| B | S. pyogenes 1e-1 | TNTC | 2,210,000 | 30.57 | Positive | 33.56 | NoCt | 32.72 | NoCt | GrPos |
| | S. pyogenes 1e-2 | TNTC | 221,000 | 34.19 | Positive | 33.45 | NoCt | 38.13 | NoCt | GrPos |
| | S. pyogenes 1e-3 | TNTC | 22,100 | 36.31 | Positive | 34.20 | NoCt | NoCt | NoCt | No ID |
| | S. pyogenes 1e-4 | *221 | 2,210 | 37.67 | Negative | 33.58 | NoCt | NoCt | NoCt | No ID |
| | S. pyogenes 1e-5 | 3 | 221 | 36.79 | Positive | 33.44 | NoCt | NoCt | NoCt | No ID |
| | NSC1 | 0 | - | 38.25 | Negative | 34.07 | 43.83 | NoCt | NoCt | No ID |
| | NSC2 | 0 | - | 38.22 | Negative | 33.86 | NoCt | NoCt | NoCt | No ID |
| | NSC3 | 0 | - | 41.19 | Negative | 34.23 | NoCt | NoCt | NoCt | No ID |
| C | S. pyogenes 1e-1 | TNTC | 2,210,000 | 26.21 | Positive | 35.04 | NoCt | 26.56 | NoCt | GrPos |
| | S. pyogenes 1e-2 | TNTC | 221,000 | 30.61 | Positive | 34.96 | NoCt | 30.38 | NoCt | GrPos |
| | S. pyogenes 1e-3 | TNTC | 22,100 | 34.17 | Positive | 35.19 | NoCt | 34.06 | NoCt | GrPos |
| | S. pyogenes 1e-4 | *221 | 2,210 | 38.99 | Positive | 34.78 | NoCt | NoCt | NoCt | No ID |
| | S. pyogenes 1e-5 | 3 | 221 | 41.83 | Negative | 34.25 | NoCt | NoCt | NoCt | No ID |
| | NSC1 | 0 | -- | 41.20 | Negative | 34.91 | NoCt | NoCt | NoCt | No ID |
| | NSC2 | 0 | -- | 40.52 | Negative | 35.07 | 46.11 | NoCt | NoCt | No ID |
| | NSC3 | 0 | - | 41.93 | Negative | 34.18 | 43.77 | NoCt | NoCt | No ID |
| D | S. pyogenes 1e-1 | TNTC | 2,210,000 | 27.51 | Positive | 35.47 | NoCt | 28.26 | NoCt | GrPos |
| | S. pyogenes 1e-2 | TNTC | 221,000 | 31.66 | Positive | 35.00 | NoCt | 30.96 | NoCt | GrPos |
| | S. pyogenes 1e-3 | TNTC | 22,100 | 34.50 | Positive | 35.42 | NoCt | 34.86 | NoCt | GrPos |
| | S. pyogenes 1e-4 | *221 | 2,210 | 38.78 | Positive | 35.53 | NoCt | 42.04 | NoCt | No ID |
| | S. pyogenes 1e-5 | 3 | 221 | 43.26 | Positive | 35.40 | NoCt | NoCt | NoCt | No ID |
| | NSC1 | 0 | - | 44.74 | Negative | 34.96 | NoCt | NoCt | NoCt | No ID |
| | NSC2 | 0 | - | 45.25 | Negative | 34.55 | NoCt | NoCt | NoCt | No ID |
| | NSC3 | 0 | - | 44.33† | Positive | 35.20 | NoCt | NoCt | NoCt | No ID |
| E | S. pyogenes 1e-1 | TNTC | 2,210,000 | 26.32 | Positive | 34.59 | NoCt | 27.87 | NoCt | GrPos |
| | S. pyogenes 1e-2 | TNTC | 221,000 | 30.65 | Positive | 35.46 | NoCt | 30.83 | NoCt | GrPos |
| | S. pyogenes 1e-3 | TNTC | 22,100 | 34.55 | Positive | 35.19 | NoCt | 35.01 | NoCt | GrPos |
| | S. pyogenes 1e-4 | *221 | 2,210 | 39.11 | Positive | 35.15 | NoCt | 41.25 | NoCt | No ID |
| | S. pyogenes 1e-5 | 3 | 221 | 43.96 | Negative | 35.59 | NoCt | 46.72 | NoCt | No ID |
| | NSC1 | 0 | -- | 43.82 | Negative | 36.22 | NoCt | NoCt | NoCt | No ID |
| | NSC2 | 0 | - | 45.08 | Negative | 35.37 | NoCt | 42.20 | NoCt | No ID |
| | NSC3 | 0 | - | 43.32 | Negative | 35.31 | NoCt | NoCt | NoCt | No ID |
| G | S. pyogenes 1e-1 | TNTC | 2,210,000 | 26.36 | Positive | 36.24 | NoCt | 28.73 | NoCt | GrPos |
| | S. pyogenes 1e-2 | TNTC | 221,000 | 30.74 | Positive | 36.09 | NoCt | 32.02 | NoCt | GrPos |
| | S. pyogenes 1e-3 | TNTC | 22,100 | 34.60 | Positive | 35.96 | NoCt | 35.34 | NoCt | GrPos |
| | S. pyogenes 1e-4 | *221 | 2,210 | 39.37 | Positive | 35.53 | NoCt | 42.28 | NoCt | No ID |
| | S. pyogenes 1e-5 | 3 | 221 | 41.78 | Negative | 34.66 | NoCt | 33.29 | NoCt | GrPos |
| | NSC1 | 0 | - | 43.80 | Negative | 35.29 | NoCt | 35.27† | NoCt | GrPos |
| | NSC2 | 0 | - | 44.12 | Negative | 35.37 | NoCt | 38.56† | NoCt | GrPos |
| | NSC3 | 0 | -- | 42.39 | Negative | 35.96 | NoCt | NoCt | NoCt | No ID |

Confirm Positivity threshold (Pt) ≤ 40 Ct; false positives †

**Observations:**

- Bead B difficult to resuspend before diluting down to 1% solid content; and appeared visually more dilute after dilution to 1% solid
- At the end of processing, samples were placed on DynaMag-2 magnetic rack, and all bead types C-G magnetised similarly apart from: Bead A, which appeared to have heavy pellets; and Bead B, which had very small bead pellets

**Analysis:**

All carboxylated magnetic beads tested here demonstrate microbial binding as determined by ETGA and Confirm readouts. However, the sensitivity of microbial detection does vary somewhat, depending on the level of blood-derived ETGA signal and/or assay inhibition.

**Example 12: Microbial capture from blood by magnetic beads occurs using a variety of different bead sizes and functional coatings**

**Aim:**

To compare microbial capture performance for a variety of commercially-available magnetic beads of different size and functional coating using Momentum's Magnitor test (ETGA and Confirm technologies). Two experiments were performed to demonstrate microbial capture for automated (Protocol 1) and manual (Protocol 2) sample processing. Importantly, Protocol 2 included three bead resuspension washes to more convincingly demonstrate that ETGA/Confirm signal is specific to bead-bound microbial cells, rather than sample carryover (as opposed to Protocol 1, which included a single beads-magnetised wash step).

**Test conditions:**

| Heading | Description | Product | Diameter ($\mu$m) | Ferrite % | Polymer |
|---|---|---|---|---|---|
| COOH-0.2 | Very Small Estapor® Carboxylated Nanospheres (-COOH) | Merck #M1-020/50 | 0.160-0.240 | >50 | Polystyrene |
| COOH-1.0 | Original Estapor® Carboxylated Microspheres (-COOH) | Merck #M1-170/40 | 0.700-1.300 | 35-45 | Polystyrene |
| HYDRO-1.0 | Original Estapor® Hydrophobic Microspheres | Merck #MS-070/40 | 0.700-1.300 | 35-50 | Polystyrene |
| NH2-1.5 | Original Estapor® Aminated Microspheres (-NH2) | Merck #M2-070/40 | 1.000-2.000 | 35-45 | Polystyrene |
| Peps6 | Magnetic beads covered with Peps6 | ApoH Technologies Ltd #MP20006 | 0.200 | Unknown | Unknown |
| Speed | SpeedBeads™ magnetic carboxylate modified particles (two layers of magnetite) | GE Healthcare #65152105050250 | 1.000 | 40 | Polystyrene |
| BioEsta | Streptavidin coated Small Estapor® Carboxylated Nanospheres (-COOH) | Merck #BE-M08/03 | 0.251-0.400 | 40-60 | Polystyrene |

All beads washed in 1 mL 1X E-BUF (50 mM Tris-HCl [pH 8.0] + 150 mM Sodium Chloride + 1% Igepal + 0.25% Tergitol) and resuspended to 1% solid in 1X E-BUF

**Sample set-up (performed separately for Protocol 1 and 2 which were performed on different days):**

- *E. coli* o/n liquid cultures set up as standard in 3 mL broth (BacTec PLUS aerobic) and incubated for 16-20 hours (37°C)
- The following day, *E. coli* liquid culture diluted to 1E-3 in blood broth and 2-hour outgrowth incubation performed (37°C @ 500 rpm)

- Following the 2-hour outgrowth incubation, *E. coli* 1E-3 pre-culture serially diluted in blood broth to produce three dilution points (EC 1E-6 to 1E-8)
- 1 mL specimens (three *E. coli* dilutions and three NSC samples: 6 sample-set) added to 2 mL sample tubes preloaded with 112 μL 10X E-BUF (500 mM Tris-HCl [pH 8.0] + 1.5 M Sodium Chloride + 10% Igepal + 2.5% Tergitol) + 15 μL beads (1% solid), then Magnitor test initiated according to either Protocol 1 or Protocol 2 (see below):

**Protocol 1 (automated sample processing on epMotion 5073m):**

- 30 mins orbital mixing (1000 rpm) @ 37°C
- 15 mins magnetisation
- 1 mL s/n removed
- 0.82 mL WB added to tubes whilst beads magnetised
- 1 mL s/n removed
- 50 μL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 μL reactions)

**Protocol 2 (manual sample processing using DynaMag-2 magnet and manual liquid transfers by pipette):**

- 30 mins orbital mixing (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 1 mL WB added and tubes mixed for 2 mins @ RT (1000 rpm)
- 5 mins magnetisation on Dynmag-2
- All s/n removed
- 1 mL WB added and tubes mixed for 2 mins @ RT (1000 rpm)
- 5 mins magnetisation on Dynmag-2
- All s/n removed
- 50 μL LM added to tubes off magnet
- ETGA reaction performed: 5 mins at 1000 rpm, then 55 mins at 800 rpm @26°C
- Manual qPCR set-up for ETGA and Confirm (10 μL reactions)

**Results:**

Protocol 1 (automated sample processing on epMotion 5073m) - performed on 20190221

| | Colonies | *E cfu/mL | COOH-0.2 | COOH-1.0 | HYDRO-1.0 | ETGA Ct NH2-1.5 | Speed | BioEsta | Peps6 |
|---|---|---|---|---|---|---|---|---|---|
| *E. coli* 1E-6 | TNTC | 18500 | 20.97 | 20.13 | 21.01 | 21.36 | 21.98 | 21.01 | 19.85 |
| *E. coli* 1E-7 | *185 | 1850 | 25.28 | 24.23 | 26.43 | 24.61 | 25.06 | 25.46 | 26.27 |
| *E. coli* 1E-8 | 12 | 185 | 27.56 | 28.64 | 30.54 | 27.24 | 30.63 | 30.59 | 28.63 |
| NSC 1 | 0 | 0 | 38.19 | 36.32 | 38.73 | 37.86 | 39.18 | 37.94 | 40.26 |
| NSC 2 | 0 | 0 | 38.33 | 36.16 | 39.41 | 37.19 | 38.90 | 39.22 | 38.82 |
| NSC 3 | 0 | 0 | 34.34 | 35.78 | 37.67 | 36.76 | 40.05 | 39.16 | 39.42 |

| | Colonies | *E cfu/mL | Confirm GrNeg Ct | | | | | | |
| --- | --- | --- | --- | --- | --- | --- | --- | --- | --- |
| | | | COOH-0.2 | COOH-1.0 | HYDRO-1.0 | NH2-1.5 | Speed | BioEsta | Peps6 |
| E. coli 1E-6 | TNTC | 18500 | 32.56 | 30.75 | 33.89 | 32.42 | 30.51 | 32.63 | 46.19 |
| E. coli 1E-7 | *185 | 1850 | 40.82 | 33.67 | NoCt | 31.99 | 33.47 | NoCt | NoCt |
| E. coli 1E-8 | 12 | 185 | 39.21 | NoCt | NoCt | 39.38 | NoCt | NoCt | NoCt |
| NSC 1 | 0 | 0 | NoCt | 42.19 | 40.84 | 44.46 | 40.55 | NoCt | NoCt |
| NSC 2 | 0 | 0 | NoCt | 40.61 | 47.17 | NoCt | NoCt | NoCt | NoCt |
| NSC 3 | 0 | 0 | NoCt | 37.42[†] | 41.04 | NoCt | NoCt | NoCt | NoCt |

Positivity threshold (Pt) ≤ 40 Ct; false positives [†]

| | Colonies | *E cfu/mL | IPC Ct | | | | | | |
| --- | --- | --- | --- | --- | --- | --- | --- | --- | --- |
| | | | COOH-0.2 | COOH-1.0 | HYDRO-1.0 | NH2-1.5 | Speed | BioEsta | Peps6 |
| E. coli 1E-6 | TNTC | 18500 | 35.89 | 35.14 | 35.72 | 36.28 | 36.06 | 36.07 | 34.86 |
| E. coli 1E-7 | *185 | 1850 | 35.98 | 35.14 | 34.74 | 36.15 | 35.08 | 35.59 | 35.63 |
| E. coli 1E-8 | 12 | 185 | 34.56 | 34.83 | 34.93 | 36.15 | 34.72 | 35.40 | 34.82 |
| NSC 1 | 0 | 0 | 34.45 | 34.83 | 34.56 | 35.91 | 35.55 | 35.49 | 34.74 |
| NSC 2 | 0 | 0 | 34.51 | 34.71 | 35.13 | 36.07 | 34.92 | 35.04 | 34.42 |
| NSC 3 | 0 | 0 | 35.04 | 34.85 | 34.45 | 35.62 | 35.62 | 35.34 | 34.85 |

**Protocol 2** (manual sample processing using DynaMag-2 magnet and manual liquid transfers by pipette) -performed on 20190228

| | Colonies | *E cfu/mL | ETGA Ct | | | | | |
| --- | --- | --- | --- | --- | --- | --- | --- | --- |
| | | | COOH-0.2 | COOH-1.0 | HYDRO-1.0 | NH2-1.5 | Speed | BioEsta |
| E. coli 1E-6 | *724 | 7240 | 23.97 | 25.45 | 24.80 | 26.29 | 25.95 | 24.32 |
| E. coli 1E-7 | 76 | 724 | 27.64 | 30.33 | 29.14 | 31.52 | 30.34 | 27.35 |
| E. coli 1E-8 | 7 | 72 | 32.20 | 30.81 | 36.25 | 32.91 | 29.64 | 31.54 |
| NSC 1 | 0 | 0 | 40.47 | 39.30 | 40.27 | 34.36 | 43.39 | 41.61 |
| NSC 2 | 0 | 0 | 42.23 | 37.84 | 41.03 | 33.96 | 43.47 | 41.70 |
| NSC 3 | 0 | 0 | 42.74 | 39.66 | 41.53 | 34.34 | 43.27 | 40.19 |

| | Colonies | *E cfu/mL | Confirm GrNeg Ct | | | | | |
| --- | --- | --- | --- | --- | --- | --- | --- | --- |
| | | | COOH-0.2 | COOH-1.0 | HYDRO-1.0 | NH2-1.5 | Speed | BioEsta |
| E. coli 1E-6 | *724 | 7240 | 27.84 | 28.54 | 27.26 | 27.05 | 27.72 | 26.90 |
| E. coli 1E-7 | 76 | 724 | 31.29 | 32.46 | 37.65 | 33.26 | 32.58 | 29.29 |
| E. coli 1E-8 | 7 | 72 | 36.95 | NoCt | NoCt | 36.41 | 31.61 | 34.06 |
| NSC 1 | 0 | 0 | NoCt | 33.69[†] | NoCt | NoCt | NoCt | NoCt |
| NSC 2 | 0 | 0 | NoCt | 31.78[†] | NoCt | NoCt | 42.70 | NoCt |
| NSC 3 | 0 | 0 | NoCt | 35.24[†] | NoCt | NoCt | NoCt | NoCt |

Positivity threshold (Pt) ≤ 40 Ct; false positives [†]

| | Colonies | *E cfu/mL | COOH-0.2 | COOH-1.0 | HYDRO-1.0 | NH2-1.5 | Speed | BioEsta |
|---|---|---|---|---|---|---|---|---|
| | | | | | IPC Ct | | | |
| E. coli 1E-6 | *724 | 7240 | 33.22 | 33.94 | 33.24 | 37.43 | 34.15 | 33.30 |
| E. coli 1E-7 | 76 | 724 | 32.44 | 32.91 | 32.78 | 36.47 | 33.70 | 32.53 |
| E. coli 1E-8 | 7 | 72 | 33.29 | 33.07 | 32.57 | 35.99 | 33.80 | 33.18 |
| NSC 1 | 0 | 0 | 33.32 | 33.02 | 33.07 | 35.78 | 33.83 | 32.99 |
| NSC 2 | 0 | 0 | 32.95 | 33.44 | 32.95 | 35.99 | 33.88 | 33.54 |
| NSC 3 | 0 | 0 | 33.61 | 33.66 | 33.23 | 35.77 | 34.33 | 33.25 |

**Analysis:**

These results demonstrate that a variety of different bead sizes and functional coatings produce comparable levels of microbial binding as determined by ETGA and Confirm readouts.

**Example 13: Magnetic beads of different size and functional coating can be used to capture a broad range of microbial species (Gram Negative, Gram Positive and Candida) from blood**

**Aim:**

To compare microbial capture performance for a variety of commercially-available magnetic beads of different size and functional coating using Momentum's Magnitor test (ETGA and Confirm technologies). *E. coli* was tested previously (Bead size and coating I: source experiment: 20190221 WP7_Bead-Comparison Analysis and 20190228_WP7_Bead-Comparison-3-wash_Analysis): to expand on this previous work, three additional microbial species were tested (S. *aureus, S. pneumoniae* and C. *albicans).*

**Test conditions:**

| Heading | Description | Product | Diameter ($\mu$m) | Ferrite % |
|---|---|---|---|---|
| COOH-0.2 | Very Small Estapor® Carboxylated Nanospheres | Merck #M1-020/50 | 0.160-0.240 | >50 |
| COOH-1.0 | Original Estapor® Carboxylated Microspheres | Merck #M1-070/40 | 0.700-1.300 | 35-45 |
| HYDRO-1.0 | Original Estapor® Hydrophobic Microspheres | Merck #MS-070/40 | 0.700-1.300 | 35-50 |
| NH2-1.5 | Original Estapor® Aminated Microspheres (-NH2) | Merck #M2-070/40 | 1.000-2.000 | 35-45 |

All beads washed in 1 mL 1X Tris+NaCl and resuspended to 1% solid in 1X Tris+NaCl

**Protocol:**

Sample set-up:

- Microorganism overnight liquid cultures (o/n) set-up in BacTec PLUS aerobic broth (inoculation of 3 mL broth from agar plate). The following day (approx 16 hours later) 300 $\mu$L S. *pneumoniae* and *C. albicans* liquid culture inoculated in 3 mL blood broth (1E-1 dilution), and 3 $\mu$L S. *aureus* liquid culture inoculated in 3 mL blood broth (1E-3 dilution); and 2 hour outgrowth performed at 37°C, 500 rpm.
- Following 2-hour outgrowth, microbial pre-cultures diluted (DF10) in blood broth to create three dilution points per microorganism.
- 100 $\mu$L TVCs performed for each microbial dilution

Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 μL beads (1% solid) and 112 μL E-BUF (500 mM Tris-HCl [pH 8.0] + 1.5 M Sodium Chloride + 10% Igepal + 2.5% Tergitol)
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 μL LM added to tubes off magnet
- ETGA reaction performed: 5 mins at 1000 rpm, then 55 mins at 800 rpm @26°C
- Manual qPCR set-up for ETGA and Confirm (10 μL reactions)

**Results:**

| | Colonies | *E cfu/mL | ETGA Ct | | | |
| | | | COOH-0.2 | COOH-1.0 | HYDRO-1.0 | NH2-1.5 |
|---|---|---|---|---|---|---|
| *S. aureus* 1E-5 | TNTC | 131,000 | 21.48 | 21.37 | 21.00 | 22.19 |
| *S. aureus* 1E-6 | TNTC | 13,100 | 26.12 | 26.13 | 25.73 | 26.58 |
| *S. aureus* 1E-7 | *131 | 1,310 | 30.76 | 30.29 | 30.25 | 30.38 |
| *C. albicans* 1E-2 | LAWN | 956,000 | 26.68 | 26.04 | 26.24 | 26.64 |
| *C. albicans* 1E-3 | TNTC | 95,600 | 28.08 | 26.46 | 27.08 | 26.76 |
| *C. albicans* 1E-4 | *956 | 9,560 | 31.20 | 31.09 | 30.64 | 31.14 |
| *S. pneumoniae* 1E-2 | LAWN | 732,000 | 30.52 | 31.34 | 30.61 | 30.41 |
| *S. pneumoniae* 1E-3 | TNTC | 73,200 | 35.07 | 35.38 | 34.28 | 34.01 |
| *S. pneumoniae* 1E-4 | *732 | 7,320 | 38.73 | 38.02 | 37.75 | 34.93 |
| NSC 1 | 0 | - | 42.44 | 36.99 | 41.11 | 33.84 |
| NSC 2 | 0 | - | 41.52 | 38.95 | 40.56 | 34.52 |
| NSC 3 | 0 | - | 41.64 | 39.35 | 40.17 | 35.27 |

| | | | Confirm Ct | | | | | | | | | | |
| | | | COOH-0.2 | | | COOH-1.0 | | | HYDRO-1.0 | | | NH2-1.5 | | |
| | Colonies | *E cfu/mL | GrNeg | GrPos | Candida | GrNeg | GrPos | Candida | GrNeg | GrPos | Candida | GrNeg | GrPos | Candida |
|---|---|---|---|---|---|---|---|---|---|---|---|---|---|---|
| *S. aureus* 1E-5 | TNTC | 131,000 | NoCt | 29.03 | NoCt | NoCt | 29.05 | NoCt | NoCt | 28.63 | NoCt | NoCt | 26.17 | NoCt |
| *S. aureus* 1E-6 | TNTC | 13,100 | NoCt | 32.30 | NoCt | NoCt | 33.17 | NoCt | NoCt | 32.67 | NoCt | NoCt | 29.46 | NoCt |
| *S. aureus* 1E-7 | *131 | 1,310 | NoCt | 39.23 | NoCt | 40.08 | NoCt | NoCt | NoCt | 41.24 | NoCt | NoCt | 35.84 | NoCt |
| *C. albicans* 1E-2 | LAWN | 956,000 | NoCt | NoCt | 28.35 | NoCt | NoCt | 27.45 | NoCt | NoCt | 26.37 | NoCt | NoCt | 27.24 |
| *C. albicans* 1E-3 | TNTC | 95,600 | NoCt | NoCt | 30.33 | NoCt | 36.10* | 31.54 | NoCt | NoCt | 30.17 | NoCt | NoCt | 29.05 |
| *C. albicans* 1E-4 | *956 | 9,560 | NoCt | NoCt | NoCt | NoCt | NoCt | 38.86 | NoCt | NoCt | 48.57 | NoCt | 39.31* | 44.85 |
| *S. pneumoniae* 1E-2 | LAWN | 732,000 | NoCt | 29.20 | NoCt | NoCt | 26.26 | NoCt | NoCt | 34.58 | NoCt | NoCt | 26.31 | NoCt |
| *S. pneumoniae* 1E-3 | TNTC | 73,200 | NoCt | 29.16 | NoCt | NoCt | 29.85 | NoCt | NoCt | 28.22 | NoCt | NoCt | 31.18 | NoCt |
| *S. pneumoniae* 1E-4 | *732 | 7,320 | NoCt | 32.38 | NoCt | NoCt | 35.47 | NoCt | NoCt | 32.33 | NoCt | NoCt | 33.74 | NoCt |
| NSC 1 | 0 | - | NoCt | NoCt | NoCt | NoCt | NoCt | NoCt | NoCt | NoCt | NoCt | NoCt | 41.24 | NoCt |
| NSC 2 | 0 | - | NoCt | NoCt | NoCt | NoCt | NoCt | NoCt | NoCt | NoCt | NoCt | NoCt | NoCt | NoCt |
| NSC 3 | 0 | - | NoCt | NoCt | NoCt | NoCt | NoCt | NoCt | NoCt | NoCt | NoCt | NoCt | NoCt | NoCt |

Positivity threshold (Pt) ≤ 40 Ct, *false positives

| | Colonies | *E cfu/mL | COOH-0.2 | IPC Ct COOH-1.0 | HYDRO-1.0 | NH2-1.5 |
|---|---|---|---|---|---|---|
| *S. aureus* 1E-5 | TNTC | 131,000 | 33.22 | 33.19 | 33.14 | 34.50 |
| *S. aureus* 1E-6 | TNTC | 13,100 | 33.19 | 32.55 | 33.07 | 34.55 |
| *S. aureus* 1E-7 | *131 | 1,310 | 33.61 | 32.80 | 33.05 | 34.20 |
| *C. albicans* 1E-2 | LAWN | 956,000 | 34.75 | 34.71 | 34.80 | 35.47 |
| *C. albicans* 1E-3 | TNTC | 95,600 | 33.59 | 33.02 | 33.49 | 34.54 |
| *C. albicans* 1E-4 | *956 | 9,560 | 33.14 | 33.02 | 32.81 | 34.06 |
| *S. pneumoniae* 1E-2 | LAWN | 732,000 | 34.02 | 33.22 | 33.56 | 34.17 |
| *S. pneumoniae* 1E-3 | TNTC | 73,200 | 33.29 | 33.05 | 33.44 | 35.28 |
| *S. pneumoniae* 1E-4 | *732 | 7,320 | 33.23 | 32.84 | 33.02 | 33.75 |
| NSC 1 | 0 | - | 33.17 | 33.21 | 33.50 | 33.96 |
| NSC 2 | 0 | - | 33.38 | 33.17 | 33.32 | 34.50 |
| NSC 3 | 0 | - | 33.55 | 33.59 | 33.05 | 34.20 |

**Analysis:**

- These results demonstrate that a variety of different bead sizes and functional coatings produce comparable levels of microbial binding as determined by ETGA and Confirm readouts.

**Example 14: Magnetic beads of different size and functional coating can be used to capture a broad range of microbial species (Gram Negative, Gram Positive and Candida) from a simple Tris+NaCl buffer**

**Aim:**

To compare microbial capture performance for a variety of commercially-available magnetic beads of different size and functional coating using Momentum's Magnitor test (ETGA and Confirm technologies). This experiment was performed using a simple buffer (50 mM Tris-HCl [pH 8.0] + 150 mM NaCl) as the specimen and wash buffer i.e. no detergents used until the addition of microbial lysis mix.

**Test conditions:**

| Heading | Description | Product | Diameter (μm) | Ferrite % |
|---|---|---|---|---|
| COOH-0.2 | Very Small Estapor® Carboxylated Nanospheres | Merck #M1-020/50 | 0.160-0.240 | >50 |
| COOH-1.0 | Original Estapor® Carboxylated Microspheres | Merck #M1-070/40 | 0.700-1.300 | 35-45 |
| HYDRO-1.0 | Original Estapor® Hydrophobic Microspheres | Merck #MS-070/40 | 0.700-1.300 | 35-50 |
| NH2-1.5 | Original Estapor® Aminated Microspheres (-NH2) | Merck #M2-070/40 | 1.000-2.000 | 35-45 |

All beads washed in 1 mL 1X Tris+NaCl (50 mM Tris-HCl [pH 8.0] + 150 mM NaCl) and resuspended to 1% solid in 1X Tris+NaCl

**Protocol:**

Sample set-up:

- Microorganism overnight liquid cultures (o/n) set-up in BacTec PLUS aerobic broth (inoculation of 3 mL broth from agar plate). The following day (approx 16 hours later) 3 $\mu$L *E. coli* and S. *aureus* liquid culture inoculated in 3 mL broth (1E-3 dilution), and 300 $\mu$L *C. albicans* liquid culture inoculated in 3 mL broth (1E-1 dilution); and 2-hour outgrowth performed at 37°C, 500 rpm.
- Following 2-hour outgrowth, microbial pre-cultures diluted (DF10) in 1X Tris+NaCl buffer to create three dilution points per microorganism.
- 100 $\mu$L TVCs performed for each microbial dilution

Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 $\mu$L beads (1% solid)
- 30 mins orbital mixing (1000 rpm) @ 37°C
- 5 mins magnetisation on DynaMag-2
- All s/n removed
- 1 mL WB (1X Tris+NaCl) added and tubes mixed for 3 mins @ RT (1000 rpm)
- 5 mins magnetisation on Dynmag-2
- All s/n removed
- 50 $\mu$L LM added to tubes off magnet
- ETGA reaction performed: 5 mins at 1000 rpm, then 55 mins at 800 rpm @26°C
- Manual qPCR set-up for ETGA and Confirm (10 $\mu$L reactions)

**Results:**

|  | | | | ETGA Ct | | |
| --- | --- | --- | --- | --- | --- | --- |
|  | Colonies | *E cfu/mL | COOH-0.2 | COOH-1.0 | HYDRO-1.0 | NH2-1.5 |
| *E. coli* 1E-6 | *629 | 6,290 | 18.68 | 19.24 | 22.36 | 30.63 |
| *E. coli* 1E-7 | 65 | 629 | 22.43 | 22.29 | 25.24 | 31.25 |
| *E. coli* 1E-8 | 21 | 63 | 28.70 | 28.21 | 29.44 | 31.35 |
| *S. aureus* 1E-5 | *791 | 7,910 | 18.32 | 18.22 | 18.08 | 25.72 |
| *S. aureus* 1E-6 | 102 | 791 | 22.26 | 22.94 | 22.58 | 30.12 |
| *S. aureus* 1E-7 | 5 | 79 | 27.65 | 26.37 | 26.61 | 30.26 |
| *C. albicans* 1E-2 | LAWN | 811,000 | 25.38 | 25.51 | 26.02 | 29.07 |
| *C. albicans* 1E-3 | TNTC | 81,100 | 27.13 | 27.71 | 27.50 | 30.09 |
| *C. albicans* 1E-4 | *811 | 8,110 | 28.93 | 28.78 | 29.03 | 30.59 |
| NSC 1 | 0 | - | 35.04 | 32.14 | 36.86 | 32.12 |
| NSC 2 | 0 | - | 36.37 | 31.65 | 36.47 | 31.61 |
| NSC 3 | 0 | - | 36.54 | 32.51 | 35.46 | 30.97 |

| | Colonies | *E cfu/mL | Confirm Ct | | | | | | | | | | | |
| | | | COOH-0.2 | | | COOH-1.0 | | | HYDRO-1.0 | | | NH2-1.5 | | |
| | | | GrNeg | GrPos | Candida | GrNeg | GrPos | Candida | GrNeg | GrPos | Candida | GrNeg | GrPos | Candida |
| E. coli 1E-6 | *629 | 6,290 | 28.24 | NoCt | NoCt | 26.59 | NoCt | NoCt | 27.42 | NoCt | NoCt | 27.39 | NoCt | NoCt |
| E. coli 1E-7 | 65 | 629 | 38.82 | NoCt | NoCt | 29.58 | NoCt | NoCt | 29.43 | NoCt | NoCt | NoCt | NoCt | NoCt |
| E. coli 1E-8 | 21 | 63 | NoCt | NoCt | NoCt | NoCt | NoCt | NoCt | 34.63 | NoCt | NoCt | NoCt | 46.54 | NoCt |
| S. aureus 1E-5 | *791 | 7,910 | NoCt | 29.56 | NoCt | NoCt | 28.61 | NoCt | NoCt | 28.79 | NoCt | NoCt | NoCt | NoCt |
| S. aureus 1E-6 | 102 | 791 | NoCt | 32.82 | NoCt | NoCt | 31.34 | NoCt | NoCt | 31.52 | NoCt | NoCt | NoCt | NoCt |
| S. aureus 1E-7 | 5 | 79 | NoCt | 36.19 | NoCt | NoCt | 35.89 | NoCt | NoCt | 35.26 | NoCt | NoCt | NoCt | NoCt |
| C. albicans 1E-2 | LAWN | 811,000 | NoCt | 43.68 | 28.74 | NoCt | 41.77 | 27.34 | NoCt | 32.91 | 26.74 | NoCt | NoCt | 31.90 |
| C. albicans 1E-3 | TNTC | 81,100 | NoCt | NoCt | 29.59 | 43.60 | NoCt | 29.14 | NoCt | 36.69 | 29.16 | NoCt | 38.39 | 48.53 |
| C. albicans 1E-4 | *811 | 8,110 | NoCt | NoCt | 31.92 | NoCt | NoCt | 30.81 | NoCt | NoCt | 30.45 | NoCt | 32.56 | 40.26 |
| NSC 1 | 0 | - | NoCt | NoCt | NoCt | NoCt | NoCt | NoCt | NoCt | NoCt | NoCt | NoCt | 41.43 | NoCt |
| NSC 2 | 0 | - | NoCt | NoCt | NoCt | NoCt | NoCt | NoCt | NoCt | NoCt | NoCt | NoCt | NoCt | NoCt |
| NSC 3 | 0 | - | NoCt | 43.20 | NoCt | NoCt | NoCt | NoCt | NoCt | NoCt | NoCt | NoCt | 40.79 | NoCt |

Positivity threshold (Pt) ≤ 40 Ct; false positives shown in red font

| | Colonies | *E cfu/mL | IPC Ct | | | |
| | | | COOH-0.2 | COOH-1.0 | HYDRO-1.0 | NH2-1.5 |
| E. coli 1E-6 | *629 | 6,290 | 31.24 | 30.86 | 30.98 | 36.67 |
| E. coli 1E-7 | 65 | 629 | 31.10 | 30.46 | 30.27 | 35.56 |
| E. coli 1E-8 | 21 | 63 | 31.01 | 30.25 | 30.51 | 37.00 |
| S. aureus 1E-5 | *791 | 7,910 | 31.43 | 31.17 | 31.02 | NoCt |
| S. aureus 1E-6 | 102 | 791 | 30.89 | 31.11 | 30.71 | 37.43 |
| S. aureus 1E-7 | 5 | 79 | 30.84 | 30.56 | 30.69 | 45.23 |
| C. albicans 1E-2 | LAWN | 811,000 | 35.64 | 33.75 | 33.48 | NoCt |
| C. albicans 1E-3 | TNTC | 81,100 | 32.98 | 31.94 | 33.61 | 40.29 |
| C. albicans 1E-4 | *811 | 8,110 | 31.34 | 31.44 | 31.74 | 37.11 |
| NSC 1 | 0 | - | 31.03 | 30.57 | 30.62 | 37.31 |
| NSC 2 | 0 | - | 31.37 | 30.55 | 30.51 | 35.33 |
| NSC 3 | 0 | - | 31.32 | 30.37 | 30.75 | 36.67 |

**Analysis:**

- These results demonstrate that in a clean system (i.e. simple Tris+NaCl buffer instead of a biological specimen) a variety of different bead sizes and functional surfaces (carboxylated and hydrophobic) produce comparable levels of microbial binding as determined by ETGA and Confirm readouts.
- Interestingly, aminated beads (NH2-1.5) produced very poor Magnitor results in specimen type, indicative of little/no microbial binding. This observation differs to the situation in blood broth specimens, where aminated beads produced comparable levels of microbial capture to the other beads tested.

**Example 15: Magnetic beads of different size and functional coating can be used to capture a broad range of microbial species (Gram Negative, Gram Positive and Candida) from non-lysed blood**

**Aim:**

To compare microbial capture performance for a variety of commercially-available magnetic beads of different size and functional coating using Momentum's Magnitor test (ETGA and Confirm technologies) in the absence of blood lysis i.e. no detergents in binding buffer

**Test conditions:**

| Heading | Description | Product | Diameter (μm) | Ferrit e % |
|---|---|---|---|---|
| COOH-0.2 | Very Small Estapor® Carboxylated Nanospheres | Merck #M1-020/50 | 0.160-0.240 | >50 |
| COOH-1.0 | Original Estapor® Carboxylated Microspheres | Merck #M1-070/40 | 0.700-1.300 | 35-45 |
| HYDRO-1.0 | Original Estapor® Hydrophobic Microspheres | Merck #MS-070/40 | 0.700-1.300 | 35-50 |
| NH2-1.5 | Original Estapor® Aminated Microspheres (-NH2) | Merck #M2-070/40 | 1.000-2.000 | 35-45 |

All beads washed in 1 mL 1X Tris+NaCl (50 mM Tris-HCl [pH 8.0] + 150 mM NaCl) and resuspended to 1% solid in 1X Tris+NaCl

**Protocol:**

Sample set-up:

- Microorganism overnight liquid cultures (o/n) set-up in BacTec PLUS aerobic broth (inoculation of 3 mL broth from agar plate). The following day (approx 16 hours later) 3 μL *E. coli* and S. *aureus* liquid culture inoculated in 3 mL broth (1E-3 dilution), and 300 μL C. *albicans* liquid culture inoculated in 3 mL blood broth (1E-1 dilution); and 2-hour outgrowth performed at 37°C, 500 rpm.
- Following 2-hour outgrowth, microbial pre-cultures diluted (DF10) in blood broth to create three dilution points per microorganism.
- 100 μL TVCs performed for each microbial dilution

Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 μL beads (1% solid) and 112 μL Binding Buffer (Tris-HCl + Sodium Chloride)
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 μL LM added to tubes off magnet
- ETGA reaction performed: 5 mins at 1000 rpm, then 55 mins at 800 rpm @26°C
- Manual qPCR set-up for ETGA and Confirm (10 μL reactions)

**Results:**

| | | | | ETGA Ct | | |
|---|---|---|---|---|---|---|
| | Colonies | *E cfu/mL | COOH-0.2 | COOH-1.0 | HYDRO-1.0 | NH2-1.5 |
| *E. coli* 1E-6 | *671 | 6,710 | 28.37 | 28.22 | 28.83 | 28.40 |
| *E. coli* 1E-7 | 69 | 671 | 32.26 | 32.90 | 31.21 | 31.73 |
| *E. coli* 1E-8 | 24 | 67 | 35.02 | 33.23 | 34.05 | 32.73 |
| *S. aureus* 1E-5 | TNTC | 27,600 | 22.23 | 22.86 | 22.45 | 22.57 |
| *S. aureus* 1E-6 | *276 | 2,760 | 26.62 | 26.64 | 26.53 | 26.09 |

(continued)

|  | Colonies | *E cfu/mL | ETGA Ct | | | |
| --- | --- | --- | --- | --- | --- | --- |
|  |  |  | COOH-0.2 | COOH-1.0 | HYDRO-1.0 | NH2-1.5 |
| *S. aureus* 1E-7 | 28 | 276 | 30.37 | 29.44 | 30.72 | 31.01 |
| *C. albicans* 1E-2 | LAWN | 649,000 | 34.01 | 34.00 | 33.52 | 33.48 |
| *C. albicans* 1E-3 | TNTC | 64,900 | 35.00 | 33.89 | 34.23 | 33.66 |
| *C. albicans* 1E-4 | *649 | 6,490 | 35.47 | 35.08 | 34.48 | 33.73 |
| NSC 1 | 0 | - | 33.95 | 35.32 | 32.42 | 31.15 |
| NSC 2 | 0 | - | 32.27 | 34.34 | 32.47 | 30.76 |
| NSC 3 | 0 | - | 33.89 | 33.37 | 32.45 | 31.24 |

| | | | Confirm Ct | | | | | | | | | | |
| --- | --- | --- | --- | --- | --- | --- | --- | --- | --- | --- | --- | --- |
| | | | COOH-0.2 | | | COOH-1.0 | | | HYDRO-1.0 | | | NH2-1.5 | | |
| | Colonies | *E cfu/mL | GrNeg | GrPos | Candida | GrNeg | GrPos | Candida | GrNeg | GrPos | Candida | GrNeg | GrPos | Candida |
| *E. coli* 1E-6 | *671 | 6,710 | 30.24 | No Ct | No Ct | 30.51 | No Ct | No Ct | 31.59 | No Ct | No Ct | 30.47 | No Ct | No Ct |
| *E. coli* 1E-7 | 69 | 671 | 38.27 | No Ct | No Ct | 48.35 | No Ct | No Ct | No Ct | No Ct | No Ct | 35.36 | No Ct | No Ct |
| *E. coli* 1E-8 | 24 | 67 | No Ct | No Ct | No Ct | No Ct | No Ct | No Ct | No Ct | No Ct | No Ct | No Ct | No Ct | No Ct |
| *S. aureus* 1E-5 | TNTC | 27,600 | No Ct | 24.94 | No Ct | No Ct | 24.55 | No Ct | No Ct | 24.34 | No Ct | No Ct | 24.24 | No Ct |
| *S. aureus* 1E-6 | *276 | 2,760 | No Ct | 27.34 | No Ct | No Ct | 27.54 | 48.19 | No Ct | 28.18 | No Ct | No Ct | 27.37 | No Ct |
| *S. aureus* 1E-7 | 28 | 276 | No Ct | 30.39 | No Ct | No Ct | 29.72 | No Ct | No Ct | 31.12 | No Ct | No Ct | 29.65 | No Ct |
| *C. albicans* 1E-2 | LAWN | 649,000 | No Ct | 40.65 | 31.51 | 40.74 | No Ct | 29.89 | No Ct | No Ct | 30.24 | No Ct | No Ct | 28.70 |
| *C. albicans* 1E-3 | TNTC | 64,900 | No Ct | No Ct | No Ct | 42.94 | No Ct | 34.41 | No Ct | No Ct | No Ct | No Ct | No Ct | No Ct |
| *C. albicans* 1E-4 | *649 | 6,490 | No Ct | No Ct | No Ct | No Ct | No Ct | No Ct | No Ct | No Ct | No Ct | No Ct | No Ct | No Ct |
| NSC 1 | 0 | - | No Ct | No Ct | No Ct | No Ct | No Ct | No Ct | No Ct | 45.94 | No Ct | No Ct | No Ct | No Ct |
| NSC 2 | 0 | - | No Ct | No Ct | No Ct | 42.39 | No Ct | No Ct | No Ct | No Ct | No Ct | No Ct | No Ct | No Ct |
| NSC 3 | 0 | - | No Ct | No Ct | No Ct | No Ct | No Ct | No Ct | No Ct | No Ct | No Ct | No Ct | No Ct | No Ct |

Positivity threshold (Pt) ≤ 40 Ct; false positives shown in red font

|  | Colonies | *E cfu/mL | IPC Ct | | | |
| --- | --- | --- | --- | --- | --- | --- |
|  |  |  | COOH-0.2 | COOH-1.0 | HYDRO-1.0 | NH2-1.5 |
| *E. coli* 1E-6 | *671 | 6,710 | 32.75 | 32.82 | 33.21 | 33.42 |
| *E. coli* 1E-7 | 69 | 671 | 33.02 | 33.61 | 32.90 | 33.81 |
| *E. coli* 1E-8 | 24 | 67 | 34.18 | 33.17 | 33.58 | 33.21 |
| *S. aureus* 1E-5 | TNTC | 27,600 | 33.31 | 33.77 | 33.86 | 34.06 |
| *S. aureus* 1E-6 | *276 | 2,760 | 33.46 | 33.20 | 33.74 | 33.07 |
| *S. aureus* 1E-7 | 28 | 276 | 32.96 | 33.06 | 33.93 | 33.71 |
| *C. albicans* 1E-2 | LAWN | 649,000 | 32.94 | 33.30 | 33.81 | 33.95 |
| *C. albicans* 1E-3 | TNTC | 64,900 | 33.82 | 33.25 | 33.71 | 34.42 |
| *C. albicans* 1E-4 | *649 | 6,490 | 33.58 | 33.53 | 33.32 | 34.25 |
| NSC 1 | 0 | - | 34.42 | 34.62 | 33.47 | 33.77 |
| NSC 2 | 0 | - | 33.62 | 33.76 | 33.61 | 33.86 |
| NSC 3 | 0 | - | 33.83 | 33.81 | 33.71 | 33.33 |

**Analysis:**

- These results demonstrate that a variety of different bead sizes and functional coatings produce comparable levels of microbial binding from blood in the absence of blood lysis as determined by ETGA and Confirm readouts
- However, microbial detection by ETGA is substantially reduced by an increase in NSC ETGA signal in the absence of blood lysis, when compared to previous experiments with blood lysis during microbial binding.

**Example 16: Microbial capture by magnetic beads occurs in a variety of complex biological specimen types**

**Aim:**

To investigate whether microbial capture using magnetic beads is possible for other complex biological fluids, in addition to blood

**Test conditions:**

| Specimen type | Description |
|---|---|
| Tris+NaCl | Tris buffer with NaCl as non-biological control sample-set (two identical sample-sets processing in parallel for Magnitor and Regrowth assays) |
| Blood | Whole blood with CPD and SPS anticoagulants |
| Saliva | Saliva diluted to 50% with distilled H2O |
| Urine | Mid-stream urine |
| Milk | Semi-skimmed Pasteurised Cow's milk |
| Note: Tris+NaCl: 50 mM Tris-HCl [pH 8.0] + 150 mM Sodium Chloride | |

**Protocol:**

- *E. coli* o/n liquid cultures set-up as standard in BacTec PLUS aerobic broth, then the following day (approx 16 hours later) 3 $\mu$L o/n added to 3 mL broth (*E. coli* 1E-3), and 2-hour outgrowth incubation performed at 37°C, 500 rpm.
- Following 2-hour outgrowth, *E. coli* 1E-3 preculture diluted to produce five serial dilution points (*E. coli* 1E-6 to 1E-9) in each specimen type. 100 $\mu$L TVCs performed on COL agar plates.

Manual simulation of Magnitor performed using DynaMag-2 magnet and manual liquid transfers:

- 1 mL specimens added to 2 mL sample tubes preloaded with 112 $\mu$L Binding Buffer (500 mM Tris-HCl [pH 8.0] + 1.5 M Sodium Chloride + 10% Igepal + 2.5% Tergitol + 0.5% Sodium Deoxycholate) + 15 $\mu$L beads (BioEstapor beads; Merck #BE-M08/03 (1% solid)) - Note, sample tubes for Tris+NaCl sample-sets not preloaded with 112 $\mu$L binding buffer (to avoid inclusion of detergents, which might inhibit microbial growth for the Regrowth assay)
- 30 mins shaking (1000 rpm) @ 37°C
- 5 mins magnetisation on DynaMag-2
- All s/n removed
- 1 mL WB added and tubes mixed for 3 mins @ RT (1000 rpm) - note, 1 mL Tris+NaCl buffer added instead of wash buffer for Tris+NaCl sample-sets to avoid inclusion of detergents, which might inhibit microbial growth for the Regrowth assay
- 5 mins magnetisation on Dynmag-2
- All s/n removed
- 50 $\mu$L LM added to tubes off magnet - note, beads resuspended in 100 $\mu$L Tris+NaCl buffer for Regrowth assay
- ETGA reaction performed: 5 mins at 1000 rpm, then 55 mins at 800 rpm @26°C
- Manual qPCR set-up for ETGA and Confirm (10 $\mu$L reactions)

**Results:**

TVCs (100 uL on COL plates)

| Specimen | Colonies |
|---|---|
| Tris+NaCl *E. coli* D1 | TNTC |
| Tris+NaCl *E. coli* D2 | 661 |
| Tris+NaCl *E. coli* D3 | 121 |
| Tris+NaCl *E. coli* D4 | 21 |
| Tris+NaCl *E. coli* D5 | 0 |
| Tris+NaCl NSC | 0 |
| Blood NSC | 0 |
| Saliva NSC | LAWN |
| Urine NSC | 5 |
| Milk NSC | 3 |

Regrowth assay on paired Tris+NaCl sample-set

1. 100 µl of Tris+NaCl specimens plated/inoculated - 'Specimen'
2. 100 µl of supernatant plated/inoculated after microbial binding step - 'After binding'
3. 100 µl of supernatant plated/inoculated after wash step - 'After washing'
4. 50 µl of beads resuspended in 100 µL Tris+NaCl buffer and plated/inoculated (i.e. 50% material on plate and 50% material inoculated into liquid culture) - 'Beads'

Plates and Liquid Cultures incubated overnight at 37°C

| | TVC (colonies) | | | | Nutrient Broth (Growth: YES/NO) | | | |
|---|---|---|---|---|---|---|---|---|
| | Specimen | After bind | After wash | Beads | Specimen | After bind | After wash | Beads |
| *E. coli* D1 | TNTC | 555 | 453 | LAWN | YES | YES | YES | YES |
| *E. coli* D2 | 661 | 104 | 5 | TNTC | YES | YES | YES | YES |
| *E. coli* D3 | 121 | 12 | 1 | 366 | YES | YES | NO | YES |
| *E. coli* D4 | 21 | 12 | 0 | 7 | YES | YES | NO | YES |
| *E. coli* D5 | 0 | 2 | 0 | 0 | YES | YES | NO | YES |
| NSC | 0 | 0 | 0 | 0 | NO | NO | NO | NO |

*Tris+NaCl sample-set*

Magnitor Results:

|  | Colonies | *E cfu/mL | ETGA Ct Tris+NaCl | Blood | Saliva | Urine | Milk |
|---|---|---|---|---|---|---|---|
| *E. coli* D1 | TNTC | 66100 | 14.49 | 21.22 | 26.45 | 23.13 | 20.30 |
| *E. coli* D2 | *661 | 6610 | 20.62 | 25.73 | 28.50 | 26.22 | 24.97 |
| *E. coli* D3 | 121 | 661 | 24.09 | 29.41 | 28.14 | 29.39 | 28.22 |
| *E. coli* D4 | 21 | 66 | 29.84 | 33.98 | 28.20 | 30.83 | 30.99 |
| *E. coli* D5 | 0 | 7 | 35.13 | 36.56 | 28.33 | 30.40 | 30.07 |
| NSC 1 | 0 | 0 | 35.74 | 38.41 | 27.32 | 31.05 | 30.70 |
| NSC 2 | 0 | 0 | 34.90 | 39.26 | 28.06 | 31.06 | 30.48 |
| NSC 3 | 0 | 0 | 35.04 | 39.45 | 28.73 | 31.08 | 30.89 |
| Ave. NSC |  |  | 35.23 | 39.04 | 28.04 | 31.06 | 30.69 |
| Pt (5th%) |  |  | 34.91 | 38.49 | 27.39 | 31.05 | 30.50 |

Positivity Thresholds (Pt) calculated using NSCs (n= 3) for each specimen type using formula = PERCENTILE.INC(array,0.05)

|  | Colonies | *E cfu/mL | Confirm GrNeg Tris+NaCl | Blood | Saliva | Urine | Milk | Confirm GrPos Tris+NaCl | Blood | Saliva | Urine | Milk |
|---|---|---|---|---|---|---|---|---|---|---|---|---|
| *E. coli* D1 | TNTC | 66100 | 20.96 | 27.24 | NoCt | 30.16 | 21.25 | NoCt | NoCt | 27.22 | 32.87 | 28.01 |
| *E. coli* D2 | *661 | 6610 | 27.03 | 31.35 | NoCt | NoCt | 25.38 | NoCt | NoCt | 28.52 | 33.07 | 27.43 |
| *E. coli* D3 | 121 | 661 | 27.64 | 37.88 | NoCt | NoCt | 29.58 | NoCt | NoCt | 29.99 | 31.79 | 26.94 |
| *E. coli* D4 | 21 | 66 | NoCt | NoCt | NoCt | NoCt | 43.46 | NoCt | NoCt | 28.12 | 35.25 | 26.91 |
| *E. coli* D5 | 0 | 7 | NoCt | NoCt | NoCt | NoCt | NoCt | 40.65 | NoCt | 28.09 | 35.43 | 27.38 |
| NSC 1 | 0 | 0 | NoCt | NoCt | NoCt | NoCt | NoCt | NoCt | 42.73 | 28.58 | 35.36 | 26.83 |
| NSC 2 | 0 | 0 | NoCt | NoCt | NoCt | NoCt | 45.84 | NoCt | NoCt | 28.83 | 32.96 | 27.28 |
| NSC 3 | 0 | 0 | NoCt | NoCt | NoCt | NoCt | NoCt | 35.55 | 42.95 | 26.77 | 32.95 | 27.40 |

Positivity threshold (Pt) ≤ 40 Ct

*Note, no observable amplification in Candida channel for Confirm*

|  | Colonies | *E cfu/mL | IPC Ct Tris+NaCl | Blood | Saliva | Urine | Milk |
|---|---|---|---|---|---|---|---|
| *E. coli* D1 | TNTC | 66100 | 38.08 | 34.81 | NoCt | 32.46 | 36.65 |
| *E. coli* D2 | *661 | 6610 | 38.79 | 34.37 | NoCt | 32.83 | 37.05 |
| *E. coli* D3 | 121 | 661 | 46.28 | 34.24 | NoCt | 32.76 | 36.44 |
| *E. coli* D4 | 21 | 66 | 43.87 | 34.31 | NoCt | 32.87 | 35.68 |
| *E. coli* D5 | 0 | 7 | 38.34 | 34.61 | NoCt | 32.60 | 35.72 |
| NSC 1 | 0 | 0 | 37.18 | 34.07 | 41.24 | 33.06 | 36.76 |
| NSC 2 | 0 | 0 | 38.20 | 33.79 | NoCt | 32.89 | 35.60 |
| NSC 3 | 0 | 0 | 36.24 | 34.14 | NoCt | 33.10 | 35.97 |

**Analysis:**

- These results demonstrate that magnetic beads can be used to capture microorganisms from a variety of complex biological specimen types as determined by ETGA and Confirm readouts.

- The Regrowth assay demonstrates that *E. coli* can regrow on agar and liquid culture after binding to magnetic beads, as determined by observable growth for 'Beads' sample-set.

**Example 17: Microbial capture and detection is possible from non-blood specimens in the absence of specimen lysis**

**Aim:**

To show microbial capture and detection in non-blood specimens of milk and urine using non-lysing binding buffer and non-lysing wash buffer.

**Preparation:**

- **10X Tris+NaCl binding buffer** = 500 mM Tris-HCl [pH 8.0] + 1.5 M Sodium Chloride
- **1X Tris+NaCl wash buffer** = 1 in 10 dilution of 10 X Tris+NaCl binding buffer
- **Fresh (Human) urine**
- **Semi-skimmed (pasteurised) cow's milk**

BioEstapor beads (Merck, Cat# BE-M 08/0.3) were re-suspended prior to use.

**Protocol:**

- *E. coli, S. aureus, C. albicans* and *S. pneumoniae* o/n liquid cultures set-up as standard in BacTec PLUS aerobic broth
- The following day (approx 16 hours later) 3 μL of *E. coli* and *S. aureus* o/n used to inoculate fresh 3 mL broth cultures (1E-3 dilutions), and 300 μL *C. albicans* and *S. pneumoniae* used to inoculate fresh 3 mL broth cultures (1E-1 dilutions); and 2-hour outgrowth performed at 37°C, 500 rpm.
- Following 2-hour outgrowth, microbial pre-cultures were serially diluted (DF10) in pre-warmed fresh urine and fresh milk to produce five dilution points: *E. coli* 1E-5 to 1E-9; *S. aureus* 1E-5 to 1E-9; *C. albicans* 1E-2 to 1E-6; and *S. pneumoniae* 1E-2 to 1E-6.
- 100 μL TVCs performed for each microbial dilution tested in milk and urine; NSCs for milk and urine were plated on three types of agar plate (SAB, COL and CBA)
- 1 mL specimens (five dilutions of each microbial species and four NSC samples: 24 samples per specimen type) added to 2 mL sample tubes preloaded with 112 μL Binding Buffer + 15 μL beads (1% solid), then automated Magnitor test initiated.

Automated sample processing on epMotion 5073m:

- 30 mins orbital mixing (1000 rpm) @ 37°C
- 15 mins magnetisation
- 1 mL s/n removed
- 0.82 mL WB (1X Tris+NaCl) added to tubes whilst beads magnetised
- 1 mL s/n removed
- 50 μL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 μL reactions)

**Results:**

| | Urine | | Milk | |
|---|---|---|---|---|
| *Specimen* | **TVC** | ***E cfu/mL** | **TVC** | ***E cfu/mL** |
| *E. coli* 1E-5 | TNTC | 141000 | TNTC | 181000 |
| *E. coli* 1E-6 | TNTC | 14100 | TNTC | 18100 |

(continued)

| | Urine | | Milk | |
|---|---|---|---|---|
| *Specimen* | **TVC** | **\*E cfu/mL** | **TVC** | **\*E cfu/mL** |
| *E. coli* 1E-7 | *141 | 1410 | *181 | 1810 |
| *E. coli* 1E-8 | 32 | 141 | 16 | 181 |
| *E. coli* 1E-9 | 3 | 14 | 6 | 18 |
| *S. aureus* 1E-5 | TNTC | 19600 | *812 | 8120 |
| *S. aureus* 1E-6 | *196 | 1960 | 69 | 812 |
| *S. aureus* 1E-7 | 11 | 196 | 15 | 81 |
| *S. aureus* 1E-8 | 2 | 20 | 8 | 8 |
| *S. aureus* 1E-9 | 1 | 2 | 9 | 1 |
| *C. albicans* 1E-2 | TNTC | 564000 | TNTC | 597000 |
| *C. albicans* 1E-3 | TNTC | 56400 | TNTC | 59700 |
| *C. albicans* 1E-4 | *564 | 5640 | *597 | 5970 |
| *C. albicans* 1E-5 | 89 | 564 | 107 | 597 |
| *C. albicans* 1E-6 | 11 | 56 | 6 | 60 |
| *S. pneumoniae* 1E-2 | TNTC | 4080000 | TNTC | 5740000 |
| *S. pneumoniae* 1E-3 | TNTC | 408000 | TNTC | 574000 |
| *S. pneumoniae* 1E-4 | TNTC | 40800 | TNTC | 57400 |
| *S. pneumoniae* 1E-5 | *408 | 4080 | *574 | 5740 |
| *S. pneumoniae* 1E-6 | 55 | 408 | 66 | 574 |
| NSC_SAB plate | 0 | 20 | 2 | 100 |
| NSC_COL plate | 0 | 20 | *10 | 100 |
| NSC_CBA plate | *2 | 20 | 10 | 100 |

*Cfu/mL values extrapolated from highest countable TVC

(NB: Urine is a non-sterile solution, therefore, colonies on the NSC plates are not unexpected)

(NB: pasteurised milk contains microorganisms, therefore, there should be colonies on the NSC plates)

ETGA Ct

IPC Ct

| Specimen | Urine | Milk |
|---|---|---|
| E. Coli 1E-5 | 15.80 | 18.97 |
| E. Coli 1E-6 | 21.69 | 21.38 |
| E. Coli 1E-7 | 26.06 | 22.19 |
| E. Coli 1E-8 | 29.54 | 22.18 |
| E. Coli 1E-9 | 30.48 | 22.51 |
| S. aureus 1E-5 | 19.57 | 21.47 |
| S. aureus 1E-6 | 23.73 | 22.09 |
| S. aureus 1E-7 | 27.82 | 22.44 |
| S. aureus 1E-8 | 28.88 | 22.15 |
| S. aureus 1E-9 | 28.95 | 22.28 |
| C. albicans 1E-2 | 22.46 | 22.62 |
| C. albicans 1E-3 | 24.56 | 22.51 |
| C. albicans 1E-4 | 28.56 | 22.17 |
| C. albicans 1E-5 | 29.82 | 22.39 |
| C. albicans 1E-6 | 29.43 | 22.00 |
| S. pneumoniae 1E-2 | 24.12 | 23.02 |
| S. pneumoniae 1E-3 | 26.07 | 22.22 |
| S. pneumoniae 1E-4 | 29.00 | 22.24 |
| S. pneumoniae 1E-5 | 30.78 | 22.06 |
| S. pneumoniae 1E-6 | 29.27 | 22.32 |
| NSC 1 | 29.48 | 22.15 |
| NSC 2 | 29.51 | 22.17 |
| NSC 3 | 29.99 | 22.13 |
| NSC 4 | 29.99 | 22.48 |
| Average NSC | 29.74 | 22.23 |

| Specimen | Urine | Milk |
|---|---|---|
| E. Coli 1E-5 | 32.36 | 40.12 |
| E. Coli 1E-6 | 31.64 | 37.94 |
| E. Coli 1E-7 | 32.35 | 43.03 |
| E. Coli 1E-8 | 33.06 | 37.06 |
| E. Coli 1E-9 | 33.29 | 41.03 |
| S. aureus 1E-5 | 32.37 | 36.09 |
| S. aureus 1E-6 | 32.41 | 39.29 |
| S. aureus 1E-7 | 32.48 | 38.13 |
| S. aureus 1E-8 | 32.99 | 36.77 |
| S. aureus 1E-9 | 33.11 | 37.21 |
| C. albicans 1E-2 | 38.08 | 46.11 |
| C. albicans 1E-3 | 33.72 | 37.14 |
| C. albicans 1E-4 | 32.20 | 37.10 |
| C. albicans 1E-5 | 32.43 | 36.26 |
| C. albicans 1E-6 | 32.58 | 39.70 |
| S. pneumoniae 1E-2 | 31.78 | 39.47 |
| S. pneumoniae 1E-3 | 31.45 | 41.49 |
| S. pneumoniae 1E-4 | 31.96 | 38.17 |
| S. pneumoniae 1E-5 | 32.71 | 37.63 |
| S. pneumoniae 1E-6 | 32.90 | 48.38 |
| NSC 1 | 33.42 | 42.68 |
| NSC 2 | 33.63 | 44.35 |
| NSC 3 | 33.53 | 38.45 |
| NSC 4 | 33.51 | 37.14 |

NB: Pasteurised milk contains bacteria; these showed as a consistent ETGA Ct~22

Confirm Ct

| Specimen | Urine | | | Milk | | |
|---|---|---|---|---|---|---|
| | GrNeg | GrPos | Candida | GrNeg | GrPos | Candida |
| E. Coli 1E-5 | 28.86 | NoCt | NoCt | 22.71 | 22.56 | NoCt |
| E. Coli 1E-6 | 29.38 | NoCt | NoCt | 29.95 | 23.01 | NoCt |
| E. Coli 1E-7 | NoCt | NoCt | NoCt | NoCt | 22.61 | NoCt |
| E. Coli 1E-8 | NoCt | 45.77 | NoCt | NoCt | 22.73 | NoCt |
| E. Coli 1E-9 | NoCt | NoCt | NoCt | NoCt | 22.78 | NoCt |
| S. aureus 1E-5 | NoCt | 30.03 | NoCt | NoCt | 21.46 | NoCt |
| S. aureus 1E-6 | NoCt | 34.47 | NoCt | NoCt | 22.41 | NoCt |
| S. aureus 1E.7 | NoCt | 38.97 | NoCt | NoCt | 22.33 | NoCt |
| S. aureus 1E-8 | NoCt | 37.85 | NoCt | NoCt | 22.39 | NoCt |

(continued)

| Specimen | Urine | | | Milk | | |
|---|---|---|---|---|---|---|
| | GrNeg | GrPos | Candida | GrNeg | GrPos | Candida |
| *S. aureus* 1E-9 | NoCt | NoCt | NoCt | NoCt | 23.03 | NoCt |
| *C. albicans* 1E-2 | NoCt | 41.75 | 28.06 | NoCt | 21.59 | 31.08 |
| *C. albicans* 1E-3 | NoCt | NoCt | 28.61 | NoCt | 23.00 | 33.04 |
| *C. albicans* 1E-4 | NoCt | NoCt | 36.26 | NoCt | 22.09 | 39.74 |
| *C. albicans* 1E-5 | NoCt | NoCt | NoCt | NoCt | 36.30 | NoCt |
| *C. albicans* 1E-6 | NoCt | NoCt | NoCt | NoCt | 22.07 | NoCt |
| *S. pneumoniae* 1E-2 | NoCt | 27.07 | NoCt | NoCt | 20.58 | NoCt |
| *S. pneumoniae* 1E-3 | NoCt | 31.78 | NoCt | NoCt | 22.08 | NoCt |
| *S. pneumoniae* 1E-4 | NoCt | 42.22 | NoCt | NoCt | 22.47 | NoCt |
| *S. pneumoniae* 1E-5 | NoCt | NoCt | NoCt | NoCt | 23.00 | NoCt |
| *S. pneumoniae* 1E-6 | NoCt | 36.55 | NoCt | NoCt | 23.29 | NoCt |
| NSC 1 | NoCt | NoCt | 43.91 | NoCt | 22.18 | NoCt |
| NSC 2 | NoCt | NoCt | NoCt | NoCt | 22.15 | NoCt |
| NSC 3 | NoCt | 40.96 | NoCt | NoCt | 21.87 | NoCt |
| NSC 4 | NoCt | NoCt | NoCt | NoCt | 22.28 | NoCt |

Positivity threshold (Pt) $\leq$ 40 Ct

**Analysis:**

- These results demonstrate that microbial capture by magnetic beads is possible in alternative specimens to blood (specifically urine and milk) in the absence of specimen lysis, as determined by ETGA and Confirm read-outs.
- The presence of commensal microorganisms in these specimen types (particularly milk), does however, effect the level of background signal for ETGA and Confirm readouts.

**Examples 18-25**

**Purpose**

To assess whether micro-organisms in clinical blood that may or may not contain antimicrobials can be 'rescued' and removed before being inoculated into a fresh medium for incubation. An investigation into whether the rescued organisms grow more efficiently compared to organisms that remain in blood or antibiotic-containing blood. Intended use of method is to enhance recovery and detectability of microbes.

**Introduction**

It is widely known that when blood cultures are taken from patients that have already been given antibiotics the blood cultures often do not grow organisms, even when the patient clearly has an infection. In recent years the main blood culture suppliers have provided bottles containing antibiotic-absorbing resin but this has only partly solved the problem. In this investigation we aim to show that when an organism is inoculated into blood containing an inhibitory concentration of an antibiotic, the organisms can be grown by capturing them using our magnetic capture beads and transferring them to a blood culture bottle with no antibiotic. A panel of organisms and a range of antibiotics were tested.

**Key findings:**

- Two to four hours difference seen in flip times between horse and human blood, but end results are comparable. Subsequent experiments can therefore be carried out using horse blood, of practical importance during this time where human blood is not available (due to the COVID-19 pandemic).

- Sample incubation in the presence of magnetic beads did not help or hinder growth of the organism.
- Flip times between the different capture reagents for *E. coli* and *S. aureus* are very similar showing that this rescue method allows organism recovery and could be performed with or without blood-lysing agents.
- In standard aerobic (SA) bottles containing bacteria in blood with an antimicrobial, the rescue method always flipped positive before the Blood Culture (BC) controls, which were negative at 5 days. For *C. albicans,* the rescue method flip times were as much as 27h faster than the BC controls and for *C. neoformans* they were over 13 hours faster.
- When comparing different magnetic bead types for organism capture (Example 23), all bead rescue samples flipped positive within 18h compared to the no-rescue blood culture control, which was negative after 5 days. This is true for all 5 bead types tested, and for both *E. coli* in blood containing 0.075mg/L Ciprofloxacin and *S. aureus* in blood containing 500mg/L Vancomycin.
- When comparing 5 further different magnetic bead types for organism capture (Example 24), all bead rescue samples flipped positive within 17h for *S. aureus* compared to the no-rescue blood culture controls which were negative. This is true for all 6 bead types tested for *S. aureus* in blood containing 500mg/L Vancomycin. For *E. coli,* the bead rescue samples flipped positive within 30h for all 6 bead types tested in blood containing 0.075mg/L Ciprofloxacin. The blood culture controls (no bead rescue) were negative.
- In high peak serum concentrations of antibiotic-blood (Ceftriaxone), FA+ blood culture bottles fail to recover *S. pneumoniae,* as reported by Flayhart *et al.* 2007. The data presented herein shows that using the methods of the invention, *S. pneumoniae* can be recovered and successfully grown from blood containing high peak serum concentrations of antibiotic.
- In high peak serum concentrations of antibiotic-blood, FA+ blood culture bottles fail to recover organisms, as reported by Chung *et al.* 2019. The data presented herein shows that using the methods of the invention, organisms can be recovered and successfully grown from concentrations as low as 180cfu/mL from blood containing high peak serum concentrations of antibiotic.

## Materials

### Buffer and reagent compositions

| | Volume | Reagent | Cat # |
|---|---|---|---|
| Detergent-free Microbial Binding Buffer -DMBB (10x) | 25.0 mL | 2M Tris-HCl [pH 8.0] | Sigma T3069 |
| | 8.8 g | NaCl | Sigma S7653 |
| | make up to 100 mL with Water | Water, molecular grade | Sigma W4502 |
| Gentle Microbial Binding Buffer - GMBB (10x) | 19.8 mL DMBB 0.2mL detergent | DMBB with 1% detergent | N/A |
| Detergent-free Wash Buffer -DWB (1x) | 10 mL | DMBB | as above |
| | 90 mL | Water, molecular grade | Sigma W4502 |
| E-buff | 10mL | Tris-NaCl buffer with detergent | N/A |
| 0.1N HCL | 1mL | 1.0N HCL | Fluka 318949-500mL |
| | 9m L | Water, molecular grade | Sigma W4502 |
| Momentum Capture Beads | 50μL per 5mL sample | Streptavidin-coated, ~300nm beads, Bio estapor | Merck Cat# BE-M 08/0.3 |
| Culture broth | 1L | Culture broth | N/A |

### Organisms, antimicrobials and other materials

- **Blood:** Horse (defibrinated) from TCS Biosciences Ltd., Buckingham, UK.
- **Test organisms:**

  ◦ **Gram Positive:** *S. aureus* ATCC 25923; *S. pneumoniae* ATCC 49619.

  ◦ **Gram Negative:** *E. coli* ATCC 25922, *Klebsiella pneumoniae* ATCC 13883, *Pseudomonas aeruginosa* ATCC 27853.

  ◦ **Fungi/Yeast:** *Candida albicans* ATCC 10231, *Cryptococcus neoformans* NCPF 8281.

- **Antibiotics:**

  ◦ **Gram Positive:** Vancomycin, Piperacillin, Cefepime, Ceftriaxone for *S. pneumoniae*

  ◦ **Gram Negative:** Ciprofloxacin, Piperacillin, Meropenem, Cefotaxime and Cefepime.

  ◦ **Yeast:** Amphotericin B.

Example 18: S. *aureus* **rescue from blood containing piperacillin**

**Aim:**

The purpose of this repeat experiment is to assess whether horse blood can be a substitute for human blood during a time where human blood is not available.

**Protocol:**

**Day 1:** Prepare overnight culture of test organism in blood/broth medium (50:50 blood:broth) and incubate at 37°C in a shaking incubator.

**Day 2:** Prepare ten-fold serial dilution of overnight culture in blood/broth as required

- Antibiotic added to whole blood (defibrinated horse blood) at required level
- Whole blood as no-antibiotic control
- Test dilution(s) of organism added to blood+antibiotic or blood only at 10 $\mu$L per mL of blood (sufficient prepared for experiment requirements)
- 100 $\mu$L plate count sample taken (T0 sample)
- 2-hour incubation at 37 °C (static), to mimic organism exposure to antimicrobials in the patient
- 100 $\mu$L plate count sample taken (T2 sample)
- 5 mL of spiked blood+abx or blood only added to blood culture bottle (using vial adapter) and incubation started (adapter removed)
- 5 mL of spiked blood+abx or blood only (from same source) treated with Momentum Capture Beads as below:

  1. Momentum Capture Beads diluted in Detergent-free Microbial Binding Buffer (DMBB) - 50 $\mu$L beads:700 $\mu$L DMBB per clean-up
  2. 750 $\mu$L of diluted beads added to 50 mL conical centrifuge tube
  3. 5 mL of transfer medium added to tube
  4. 5 mL of spiked blood+abx or blood only added to tube
  5. Incubate on ITL TherMix (Integrated Technologies Ltd. Kent, UK), 32.5 °C / 30 min /1000rpm - ramped speed protocol
  6. Magnetise on V&P magnet (V&P scientific Inc, CA, US) for 10 min then remove supernatant
  7. Resuspend beads in 1 mL Detergent-free Wash Buffer (DWB) off magnet and transfer to 2 mL Eppendorf flip-top tube on DynaMag-2 magnet (Thermo Fisher Scientific, Life Technologies Ltd. Paisley, UK).
  8. Magnetise for 3 min then remove supernatant
  9. Resuspend beads in 1 mL Detergent-free Wash Buffer (DWB) off magnet then replace on DynaMag-2 magnet
  10. Magnetise for 3 min then remove supernatant

11. Resuspend beads in 1 mL DWB, add to new blood culture bottle (using vial adapter) and start incubation (adapter removed)

**Day 3:** Plate counts recorded.

Record the "Flip" time of each bottle or bottle result at the end of 5 days incubation. The "Flip" time is when a bottle turns positive as determined by the automated blood culture cabinet, which records time to positivity.

**Results:**

| Sample type | Organism | Antibiotic: Piperacillin 96ug/mL blood | Defibrinated horse blood | | CPD human blood | |
|---|---|---|---|---|---|---|
| | | | cfu/5mL | Flip time in Days | cfu/5mL | Flip time in Days |
| Control | *S. aureus* | Y | 50000 | Neg | 5000 | Neg |
| Bead rescue | *S. aureus* | Y | 50000 | 0.65 | 5000 | 0.57 |
| Control | *S. aureus* | Y | 5000 | Neg | 750 | Neg |
| Bead rescue | *S. aureus* | Y | 5000 | 0.78 | 750 | 0.62 |
| Control | *S. aureus* | N | 35000 | 0.45 | 16000 | 0.34 |
| Bead rescue | *S. aureus* | N | 35000 | 0.53 | 16000 | 0.45 |

**Comments:**

- Two to four hours difference seen in flip times between horse and human blood, but end results are comparable.
- Subsequent experiments can therefore be carried out using horse blood during this time where human blood is not available.

**Example 19: Breakpoint experiment detail**

**Aim:**

To determine a starting point in terms of antibiotic concentration and microbial load for subsequent experiments. Antibiotic concentration ranges were tested to find appropriate breakpoints for use in experiments. A range of antibiotic concentrations were tested against a given microbial load to determine the concentration at which growth was inhibited but the microorganisms were not killed. This was determined by plate counts at intervals of 0, 2.5 and 5 hours.

**Protocol:**

1. **Day 1:** Prepare an overnight culture of the test organism in 3mL of blood/broth solution (1.5mL defibrinated Horse blood: 1.5mL transfer Medium).
2. **Day 2:** Dissolve antibiotic in required diluent to prepare a suitable stock solution and prepare a dilution series in Nuclease-free Water to cover a final concentration range of 100 to 0.001 and 0mg/L.
3. Dilute the overnight culture of the microorganism to the required point in pre-warmed blood/broth solution. This may require determining for each organism/antibiotic combination, but as a starting dilution $10^{-4}$ will be used (giving a final concentration in the sample of $10^{-5}$).
4. Aliquot 980$\mu$L of pre-warmed defibrinated horse blood into the required number of 2mL flip-top tube.
5. Add 10$\mu$L of diluted antibiotic to the relevant tube, and 10$\mu$L of diluent to the last tube as a control. Mix.
6. Add 10$\mu$L of the required dilution of the overnight culture of microorganism. Mix
7. Sample 100$\mu$L from each tube and plate out on Columbia Blood Agar base for the Time 0 count.
8. Plate 100$\mu$L of the diluted overnight culture as a growth/no growth control.
9. Incubate the samples at 37°C in a shaking incubator (450 rpm) and sample 100$\mu$L for plate counting at 2.5- and 5-hour intervals.

10. Incubate the plates overnight at 37°C.

11. **Day 3:** Count the plates and tabulate the results. Determine the antibiotic concentration that inhibits growth without total die-off of the microorganisms.

The concentrations determined by this protocol were used in the following examples unless stated otherwise.

## Example 20: Organism growth with and without magnetic beads and/or antibiotics. Aim:

The purpose of this experiment is to assess whether the presence of magnetic beads enables organisms to grow faster because they bind to the beads and like a solid substrate to grow on. This experiment looks at relative rate of growth of organisms in blood broth, with and without antibiotic, with and without magnetic beads, (without bead transfer).

### Protocol:

**Day 1:** Prepare overnight culture of test organism in blood/broth medium (50:50 blood:broth) and incubate at 37°C in a shaking incubator.

**Day 2:** Prepare ten-fold serial dilution of overnight culture in blood/broth as required and incubate at 37°C for 2h for outgrowth.

In sample set 1, the dilutions of test organism in blood will contain beads, a duplicate set of samples (set 2) will not contain beads. Neither of these sets contain antibiotic.

In sample set 3, the dilutions of test organism in blood will contain antibiotic and beads, a duplicate set of samples (set 4) will not contain the beads. Both of these sets contain antibiotic.

Antibiotic added to whole blood (defibrinated horse blood) at required level.

Whole blood as no-antibiotic control.

Using 10mL pre-warmed whole blood per sample, add the Test dilution(s) of organism at 10μL per mL of blood. In a duplicate set, add the relevant antimicrobial to each of the 3 tubes.

Plate 100μL of these samples for T0 TVC.

Remove 5 mL from the 10mL samples to create set 1 and set 3.

Add 50μL of raw magnetic beads per 5mL blood to set 1 and set 3 sample tubes, leaving set 2 and set 4 with no beads.

Add 5mL of spiked blood+beads (set 1) and 5mL of spiked blood/Abx+beads (set 3) to blood culture bottles (BioMerieux SA culture bottle) using a vial adapter (West Pharmaceutical Services, Inc. PA, US).

Then add 5mL of the spiked blood (from same source), no-bead control (set 2 and set 4) to blood culture bottles.

Remove the adaptor and incubate the blood culture bottles in the automated blood culture cabinet (BacT/ALERT BioMerieux) for up to 5 days.

**Day 3:** Count the TVC plates and record.

Record the "Flip" time of each bottle or bottle result at the end of 5 days incubation. The "Flip" time is when a bottle turns positive as determined by the automated blood culture cabinet, which records time to positivity.

### Results:

| Organism | cfu/5mL | Time to positivity in days | | | |
| --- | --- | --- | --- | --- | --- |
| | | No ABX | | Piperacillin 96ug/mL blood | |
| | | Control | With magnetic beads | Control | With magnetic beads |
| *S. aureus* | 900 | 0.58 | 0.57 | Neg | Neg |
| *E. coli* | 1800 | 0.43 | 0.43 | Neg | Neg |

### Comments:

Sample incubation in the presence of magnetic beads did not help or hinder growth of the organism.

**Protocol for organism rescue experiments (Examples 21 - Example 24)**

**Day 1:** Prepare overnight culture of test organism in blood/broth medium (50:50 blood:broth) and incubate at 37°C (or 30°C for *C. neoformans*) in a shaking incubator.
**Day 2:** In the clean room, prepare suitable volumes of the antibiotics for use in the experiment.

- Prepare stock solutions using the formula $1000/P \times V \times C = W$
- where P = potency given by the manufacturer ($\mu$g/mg), V = volume required (mL), C = final concentration of solution (multiples of 1000) (mg/L), and W = weight of antibiotic in mg to be dissolved in volume V (mL).
- Prepare ten-fold serial dilution of overnight culture in blood/broth as required and incubate at 37°C (or 30°C for *C. neoformans*) for 2h for outgrowth.
- Antibiotic added to whole blood (defibrinated horse blood) at required level
- Whole blood as no-antibiotic control
- Test dilution(s) of organism added to blood+antibiotic or blood only at 10 $\mu$L per mL of blood (sufficient prepared for experiment requirements)
- 100 $\mu$L plate count sample taken (T0 sample)
- 2-hour incubation (static) at 37 °C (or 30°C for *C. neoformans*)
- 100 $\mu$L plate count sample taken (T2 sample)
- 5 mL of spiked blood+abx or blood only added to blood culture bottle (using vial adapter) and incubation started (adapter removed)
- 5 mL of spiked blood+abx or blood only (from same source) treated with Momentum Capture Beads as below:

  1. Capture Beads diluted in the relevant Capture Reagent - 50 $\mu$L beads:700 $\mu$L capture reagent per clean-up

  2. 750 $\mu$L of diluted beads added to 50 mL conical centrifuge tube

  3. 5 mL of transfer medium added to tube

  4. 5 mL of spiked blood+abx or blood only added to tube

  5. Incubate on ITL TherMix (32.5 °C / 30 min /1000rpm - ramped speed protocol)

  6. Magnetise on V&P magnet for 10 min then remove supernatant

  7. Resuspend beads in 1 mL Detergent-free Wash Buffer (DWB) off magnet and transfer to 2 mL Eppendorf flip-top tube on DynaMag-2 magnet

  8. Magnetise for 3 min then remove supernatant

  9. Resuspend beads in 1 mL Detergent-free Wash Buffer (DWB) off magnet then replace on DynaMag-2 magnet

  10. Magnetise for 3 min then remove supernatant

  11. Resuspend beads in 1 mL DWB, add to new blood culture bottle (using vial adapter) and start incubation (adapter removed)

**Day 3:** Plate counts recorded.

Record the "Flip" time of each bottle or bottle result at the end of 5 days incubation. The "Flip" time is when a bottle turns positive as determined by the automated blood culture cabinet, which records time to positivity.

**Example 21: Comparison of organism rescue when using detergent-free or blood lysing capture buffers.**

**Aim:**

To compare detergent-free or blood-lysing capture buffers when performing the organism rescue method. Do different capture buffers have an effect on successful organism rescue? Compare DMBB, E-Buff and GMBB as capture reagents on both *E. coli* and *S. aureus* recovery and using DWB for all washes.

**Results:**

| | | | | Flip time in Days | | | |
| | | | | No bead rescue | Capture Reagent for bead rescue | | |
| Sample type | Organism | cfu/5mL | Antibiotic: Piperacillin 96ug/mL blood | Control BC | DMBB | E-Buff | GMBB |
|---|---|---|---|---|---|---|---|
| Control | *E. coli* | 150000 | Y | Neg | 0.5 | 0.45 | 0.47 |
| Control | *E. coli* | 50000 | Y | Neg | 0.54 | 0.52 | 0.5 |
| Control | *E. coli* | 100000 | N | 0.24 | 0.34 | 0.29 | 0.33 |
| Control | *S. aureus* | 50000 | Y | Neg | 0.65 | 0.63 | 0.61 |
| Control | *S. aureus* | 10000 | Y | Neg | 0.71 | 0.67 | 0.69 |
| Control | *S. aureus* | 75000 | N | 0.24 | 0.39 | 0.35 | 0.38 |

**Comments:**

Flip times between the different capture reagents for *E. coli* and *S. aureus* are very similar showing that this rescue method allows organism recovery and could be performed with or without blood-lysing agents.

**Example 22: Application of organism rescue to further microorganisms and antimicrobial agents**

**Aim:**

To demonstrate that the rescue method is applicable to a wide variety of microorganisms and antimicrobial agents.

**Preparation of Vancomycin:**

For Vancomycin (Lot# 058M4009V) with a potency of 1008 $\mu$g/mg, use the following equation to find the weight needed to make 1mL of a 10,000mg/mL stock solution: Vancomycin stock solutions were prepared using the formula $1000/P \times V \times C = W$ where P = potency given by the manufacturer ($\mu$g/mg), V = volume required (mL), C = final concentration of solution (multiples of 1000) (mg/L), and W = weight of antibiotic in mg to be dissolved in volume V (mL).
So,

$$1000 / 1008 \text{ x } 10\text{mL x } 10 = W$$

$$0.992 \text{ x } 10 \text{ x } 10 = W$$

W= 99.2mg in 10mL saline (0.9%) for a 10,000mg/mL stock solution.
Two different strains of *S. aureus* grew in every dilution of Vancomycin at every time point. The decision was made to use a final concentration of 500mg/L for subsequent experiments given that the drug is relatively slow acting and experiment length had to be kept within reasonable timeframes.

Results:

| Organism | Org cfu/5mL blood | Antibiotic | [Antibiotic] mg/L blood | Control Flip time in Days | Bead Rescue Flip time in Days |
|---|---|---|---|---|---|
| E. coli | 1000000 | Ciprofloxacin | 0.075 | Neg | 0.61 |
| E. coli | 100000 | Ciprofloxacin | 0.075 | Neg | 0.68 |
| E. coli | 100000 | Ciprofloxacin | 0 | 0.34 | 0.42 |
| S. aureus | 1000000 | Vancomycin | 500 | Neg | 0.61 |
| S. aureus | 100000 | Vancomycin | 500 | Neg | 0.66 |
| S. aureus | 100000 | Vancomycin | 0 | 0.29 | 0.46 |
| S. aureus | 1000 | Vancomycin | 500 | Neg | 1.15 |
| S. aureus | 1000 | Vancomycin | 0 | 0.47 | 0.67 |
| S. pneumoniae | 100000 | Ceftriaxone | 1 | Neg | 0.68 |
| S. pneumoniae | 10000 | Ceftriaxone | 1 | Neg | 0.71 |
| S. pneumoniae | 10000 | Ceftriaxone | 0 | 0.28 | 0.37 |
| S. pneumoniae | 200 | Ceftriaxone | 1 | Neg | 0.86 |
| S. pneumoniae | 200 | Ceftriaxone | 0 | 0.39 | 0.46 |
| C. albicans | 4000 | Amphotericin B | 1 | 2.22 | 1.27 |
| C. albicans | 4000 | Amphotericin B | 2 | 2.98 | 1.85 |
| C. albicans | 4000 | Amphotericin B | 0 | 0.68 | 0.74 |
| C. neoformans | 102200 | Amphotericin B | 2 | 3.7 | 3.14 |

**Comments:**

For the no antimicrobial control, blood culture always flipped positive before the rescue method.

In standard SA bottles containing bacteria in blood with an antimicrobial, the rescue method always flipped positive before the BC controls, which were negative at 5 days. For *C. albicans,* the rescue method flip times were as much as 27h faster than the BC controls and for *C. neoformans* they were over 13 hours faster.

## Example 23: Comparison of organism rescue using different magnetic bead types I Aims:

To perform the rescue method using five different bead types; hydrophobic, aminated, carboxylated, Speed-Beads and streptavidin coated and comparing the efficiency of capture and recovery.

| Study ID | Bead type | Description | Product # | Diameter (μm) | Ferrite % | Polymer | % solid |
|---|---|---|---|---|---|---|---|
| HY | HYDRO-1.0 | Original Estapor® Hydrophobic Microspheres | Merck #MS-070/40, #M844/3 | 0.700-1.300 | 35-50 | Polystyrene | 1% |
| NH | NH2-1.5 | Original Estapor® Aminated Microspheres (-NH2) | Merck #M2-070/40, #7071/20 | 1.000-2.000 | 35-45 | Polystyrene | 1% |
| CO | COOH-0.3 | Small Estapor® Carboxylated Nanospheres (-COOH) | Merck #M1-030/40, #8062/16 | 0.251-0.400 | 40-60 | Polystyrene | 1% |
| SP | Speed | SpeedBeads™ magnetic carboxylate modified particles (two layers of magnetite) | GE Healthcare #65152105050250, #9651316 | 1.000 | 40 | Polystyrene | 1% |
| BE | BioEsta | Streptavidin coated Small Estapor® Carboxylated Nanospheres (-COOH) | Merck #BE-M08/03, MBL #BE-006 | 0.251-0.400 | 40-60 | Polystyrene | 1% |

Results:

| Sample type | Organism | Cfu/mL | Bead type used | Flip time in Days | | Difference (Days) compared to BE | |
|---|---|---|---|---|---|---|---|
| | | | | Ciprofloxacin 0.075mg/L blood | No Antibiotics | Ciprofloxacin 0.075mg/L blood | No Antibiotics |
| Control | E. coli | 11400 | N/A, BC | Negative | 0.36 | Negative | -0.05 |
| Bead rescue | E. coli | 11400 | HY | 0.99 | 0.42 | 0.36 | 0.01 |
| Bead rescue | E. coli | 11400 | NH | 0.62 | 0.42 | -0.01 | 0.01 |
| Bead rescue | E. coli | 11400 | CO | 0.64 | 0.39 | 0.01 | -0.02 |
| Bead rescue | E. coli | 11400 | SP | 0.65 | 0.41 | 0.02 | 0 |
| Bead rescue | E. coli | 11400 | BE | 0.63 | 0.41 | - | - |
| Control | E. coli | 114000 | N/A, BC | Negative | 0.36 | Negative | |
| Bead rescue | E. coli | 114000 | HY | 0.61 | 0.42 | 0.01 | 0.01 |
| Bead rescue | E. coli | 114000 | NH | 0.60 | 0.42 | 0.00 | 0.01 |
| Bead rescue | E. coli | 114000 | CO | 0.60 | 0.39 | 0.00 | -0.02 |
| Bead rescue | E. coli | 114000 | SP | 0.66 | 0.41 | 0.06 | 0.00 |
| Bead rescue | E. coli | 114000 | BE | 0.60 | 0.41 | - | - |

| Sample type | Organism | Cfu/mL | Bead type used | Flip time in Days | | Difference (Days) compared to BE | |
|---|---|---|---|---|---|---|---|
| | | | | Vancomycin 500mg/L blood | No Antibiotics | Vancomycin 500mg/L blood | No Antibiotics |
| Control | S. aureus | 20000 | N/A, BC | Negative | 0.32 | Negative | -0.17 |
| Bead rescue | S. aureus | 20000 | HY | 0.68 | 0.53 | -0.02 | 0.04 |
| Bead rescue | S. aureus | 20000 | NH | 0.68 | 0.46 | -0.02 | -0.03 |
| Bead rescue | S. aureus | 20000 | CO | 0.60 | 0.41 | -0.1 | -0.08 |
| Bead rescue | S. aureus | 20000 | SP | 0.72 | 0.56 | 0.02 | 0.07 |
| Bead rescue | S. aureus | 20000 | BE | 0.70 | 0.49 | - | - |
| Control | S. aureus | 200000 | N/A, BC | Negative | 0.32 | Negative | -0.17 |
| Bead rescue | S. aureus | 200000 | HY | 0.59 | 0.53 | -0.04 | 0.04 |
| Bead rescue | S. aureus | 200000 | NH | 0.61 | 0.46 | -0.02 | -0.03 |
| Bead rescue | S. aureus | 200000 | CO | 0.53 | 0.41 | -0.1 | -0.08 |
| Bead rescue | S. aureus | 200000 | SP | 0.61 | 0.56 | -0.02 | 0.07 |
| Bead rescue | S. aureus | 200000 | BE | 0.53 | 0.49 | - | - |

**Comments:**

All bead rescue samples flipped positive within 24h (within 18h for *S. aureus*) compared to the no-rescue blood culture control, which was negative after 5 days.

This is true for all 5 bead types tested, for *E. coli* in blood containing 0.075mg/L Ciprofloxacin and *S. aureus* in blood containing 500mg/L Vancomycin.

**Example 24: Comparison of organism rescue using different magnetic bead types II Aim:**

To perform the rescue method using a further five different bead types; citrate-capped, starch-capped, polyacrylamide, mannose-binding lectin and polylysine, and comparing the efficiency of capture and recovery.

| Study ID | Bead type | Description | Product # | Diameter (μm) | % solid |
|---|---|---|---|---|---|
| Raw Fe | citrate capped | Nanomagnets, 250nm, citrate capped, 2.5mg, 1mL, DI water | Nanopartz AM1-250-CIT-DIH-2.5-1 | 0.25 | 1% |
| CHO | starch capped | Nanomagnets, 250nm, starch capped, 2.5mg, 1mL, DI water | Nanopartz CM1-250-STC-DIH-2.5-1 | 0.25 | 1% |
| Alt polymer | polyacrylamide capped | Nanomagnets, 250nm, polyacrylamide capped, 2.5mg,1mL, DI water | Nanopartz CM1-250-PLAC-DIH-2.5-1 | 0.25 | 1% |
| MBL | mannose binding lectin | Nanomagnets, 250nm, mannosebinding lectin capped, 2.5mg,1mL, DI water | Nanopartz CM1-250-MBL-DIH-2.5-1 | 0.25 | 1% |
| P-Ly | Poly-l-lysine capped | Nanomagnets, 250nm, Poly-l-lysine capped, 2.5mg,1mL, DI water | Nanopartz CM1-250-PLL-DIH-2.5-1 | 0.25 | 1% |
| BE | BioEsta | Streptavidin coated Small Estapor® Carboxylated Nanospheres (-COOH) | Merck #BE-M08/03, MBL #BE-006 | 0.251~0.400 | 1% |

**Results:**

| Sample type | Organism | Cfu/mL | Bead type used | Flip time in Days | | Difference (Days) compared to BE | |
|---|---|---|---|---|---|---|---|
| | | | | Ciprofloxacin 0.075mg/L blood | No Antibiotics | Ciprofloxacin 0.075mg/L blood | No Antibiotics |
| Control | E. coli | 360800 | N/A, BC | Negative | 0.27 | Negative | -0.08 |
| Bead rescue | E. coli | 360800 | Raw Fe | 0.73 | 0.3 | -0.12 | -0.05 |
| Bead rescue | E. coli | 360800 | CHO | 0.69 | 0.28 | -0.16 | -0.07 |
| Bead rescue | E. coli | 360800 | Alt polymer | 1.23 | 0.32 | 0.38 | -0.03 |
| Bead rescue | E. coli | 360800 | MBL | 0.84 | 0.29 | -0.01 | -0.06 |
| Bead rescue | E. coli | 360800 | P-Ly | 1.0 | 0.35 | 0.15 | 0.00 |
| Bead rescue | E. coli | 360800 | BE | 0.85 | 0.35 | | |

| Sample type | Organism | Cfu/mL | Bead type used | Flip time in Days | | Difference (Days) compared to BE | |
|---|---|---|---|---|---|---|---|
| | | | | Vancomycin 500mg/L blood | No Antibiotics | Vancomycin 500mg/L blood | No Antibiotics |
| Control | S. aureus | 35760 | N/A, BC | Negative | 0.26 | Negative | -0.19 |
| Bead rescue | S. aureus | 35760 | Raw Fe | 0.68 | 0.49 | 0.07 | 0.04 |
| Bead rescue | S. aureus | 35760 | CHO | 0.67 | 0.47 | 0.06 | 0.02 |
| Bead rescue | S. aureus | 35760 | Alt polymer | 0.69 | 0.49 | 0.08 | 0.04 |
| Bead rescue | S. aureus | 35760 | MBL | 0.65 | 0.51 | 0.04 | 0.06 |
| Bead rescue | S. aureus | 35760 | P-Ly | 0.7 | 0.46 | 0.09 | 0.01 |
| Bead rescue | S. aureus | 35760 | BE | 0.61 | 0.45 | | |
| Control | S. aureus | 357600 | N/A, BC | Negative | 0.26 | Negative | -0.19 |
| Bead rescue | S. aureus | 357600 | Raw Fe | 0.58 | 0.49 | 0.03 | 0.04 |
| Bead rescue | S. aureus | 357600 | CHO | 0.60 | 0.47 | 0.05 | 0.02 |
| Bead rescue | S. aureus | 357600 | Alt polymer | 0.63 | 0.49 | 0.08 | 0.04 |
| Bead rescue | S. aureus | 357600 | MBL | 0.59 | 0.51 | 0.04 | 0.06 |
| Bead rescue | S. aureus | 357600 | P-Ly | 0.63 | 0.46 | 0.08 | 0.01 |
| Bead rescue | S. aureus | 357600 | BE | 0.55 | 0.45 | | |

**Comments:**

All bead rescue samples flipped positive within 17h for *S. aureus* compared to the no-rescue blood culture controls, which were negative. This is true for all 6 bead types tested for *S. aureus* in blood containing 500mg/L Vancomycin.

For *E. coli*, the bead rescue samples flipped positive within 30h for all 6 bead types tested in blood containing 0.075mg/L Ciprofloxacin. The blood culture controls (no bead rescue) were negative.

## Example 25: Comparison of organism rescue of microbes that fail to recover in FA plus BacT/ALERT blood culture bottles in the presence of antibiotics

**Protocol for reproducing work of Flayhart et *al.* and Chung et *al.***

**Day 1:** Prepare overnight culture of test organism in blood/broth medium (50:50 blood:broth) and incubate at 37°C in a shaking incubator.

**Day 2:** In the clean room, prepare suitable volumes of the antibiotics for use in the experiment.

- Prepare stock solutions using the formula $1000/P \times V \times C = W$
- where P = potency given by the manufacturer ($\mu$g/mg), V = volume required (mL), C = final concentration of solution (multiples of 1000) (mg/L), and W = weight of antibiotic in mg to be dissolved in volume V (mL).
- Prepare ten-fold serial dilution of overnight culture in blood/broth as required and incubate at 37°C for 2h for outgrowth.
- Antibiotic added to whole blood (defibrinated horse blood) at required level
- Whole blood as no-antibiotic control
- Test dilution(s) of organism added to blood+antibiotic or blood only at 10 $\mu$L per mL of blood (sufficient prepared for experiment requirements)
- 100 $\mu$L plate count sample taken (T0 sample)
- 5 mL of spiked blood+abx or blood only added to blood culture bottle (BioMerieux FA+ culture bottle), using vial adapter and incubation started (adapter removed)
- 5 mL of spiked blood+abx or blood only (from same source) treated with Momentum Capture Beads as below:

  1. Momentum Capture Beads diluted in the relevant Capture Reagent - 50 $\mu$L beads:700 $\mu$L capture reagent per clean-up

  2. 750 $\mu$L of diluted beads added to 50 mL conical centrifuge tube

  3. 5 mL of transfer medium added to tube

  4. 5 mL of spiked blood+abx or blood only added to tube

  5. Incubate on ITL TherMix (32.5 °C / 30 min /1000rpm - ramped speed protocol)

  6. Magnetise on V&P magnet for 10 min then remove supernatant

  7. Resuspend beads in 1 mL Detergent-free Wash Buffer (DWB) off magnet and transfer to 2 mL Eppendorf flip-top tube on DynaMag-2 magnet

  8. Magnetise for 3 min then remove supernatant

  9. Resuspend beads in 1 mL Detergent-free Wash Buffer (DWB) off magnet then replace on DynaMag-2 magnet

  10. Magnetise for 3 min then remove supernatant

  11. Resuspend beads in 1 mL DWB, add to an unused blood culture bottle (BioMerieux FA+ culture bottle) using vial adapter and start incubation (adapter removed)

**Day 3:** Plate counts recorded.

Record the "Flip" time of each bottle or bottle result at the end of 5 days incubation. The "Flip" time is when a bottle turns positive as determined by the automated blood culture cabinet, which records time to positivity.

***Reproduction of experiment by Flayhart et al. 2007***

**Aim:**

To compare organism capture and rescue from FA plus bottles, on *S. pneumoniae* in Ceftriaxone. Reproduce experiment by Flayhart *et al.* 2007 testing antibiotic concentrations of 250, 125 and 94 mg/L and also include concentrations of 50 and 10 mg/L.

**Results:**

In the table below, "control" represents samples processed using FA plus bottles (also referred to as "BC" in the final column) and "bead rescue" refers to samples processed according to the methods of the invention.

| Sample type | Organism | Org cfu/5mL blood | Antibiotic name | [Antibiotic] mg/L blood | Date incubated | Flip time in Days | Fastest method |
|---|---|---|---|---|---|---|---|
| Control | *S. pneumoniae* | 12600 | Ceftriaxone | 250 | 19/05/2020 | Neg | Rescue |
| Bead rescue | *S. pneumoniae* | 12600 | Ceftriaxone | 250 | 19/05/2020 | 0.51 | Rescue |
| Control | *S. pneumoniae* | 12600 | Ceftriaxone | 125 | 19/05/2020 | Neg | Rescue |
| Bead rescue | *S. pneumoniae* | 12600 | Ceftriaxone | 125 | 19/05/2020 | 0.49 | Rescue |
| Control | *S. pneumoniae* | 12600 | Ceftriaxone | 94 | 19/05/2020 | Neg | Rescue |
| Bead rescue | *S. pneumoniae* | 12600 | Ceftriaxone | 94 | 19/05/2020 | 0.52 | Rescue |
| Control | *S. pneumoniae* | 12600 | Ceftriaxone | 50 | 19/05/2020 | Neg | Rescue |
| Bead rescue | *S. pneumoniae* | 12600 | Ceftriaxone | 50 | 19/05/2020 | 0.47 | Rescue |
| Control | *S. pneumoniae* | 12600 | Ceftriaxone | 10 | 19/05/2020 | Neg | Rescue |
| Bead rescue | *S. pneumoniae* | 12600 | Ceftriaxone | 10 | 19/05/2020 | 0.46 | Rescue |
| Control | *S. pneumoniae* | 12600 | None | 0 | 19/05/2020 | 0.31 | BC |
| Bead rescue | *S. pneumoniae* | 12600 | None | 0 | 19/05/2020 | 0.36 | BC |

**Comments:**

In high peak serum concentrations of antibiotic-blood (Ceftriaxone), FA+ blood culture bottles fail to recover *S. pneumoniae,* as reported by Flayhart *et al.* 2007. This data shows that using the methods of the invention, *S. pneumoniae* can be recovered and successfully grown from blood containing high peak serum concentrations of antibiotic. This experiment shows the methods of the invention are an improvement over the FA plus bottles as tested by Flayhart.

***Reproduction of experiment by Chung et al. 2019***

**Aim:**

To compare organism capture and rescue from FA plus bottles, on the organisms in the table below with their respective antibiotics. As reported in the paper by Chung *et al.* 2019, further antibiotic concentrations were tested (164mg/L, 100mg/L and 49mg/L for Cefepime, Cefotaxime and Meropenem respectively).

| Drug | MIC (mg/L) | | | | |
|---|---|---|---|---|---|
| | *S. aureus* | *C. coli* | *K. pneumoniae* | *P. aeruginosa* | Test conc. mg/L |
| **Cefepime** | ≤4 | ≤1 | ≤1 | ≤1 | 164 |
| **Cefotaxime** | | ≤1 | | | 100 |
| **Meropenem** | | ≤1 | ≤1 | ≤1 | 49 |

**Results:**

In the table below, "control" represents samples processed using FA plus bottles (also referred to as "BC" in the final column) and "bead rescue" refers to samples processed according to the methods of the invention.

EP 4 041 908 B1

| Sample type | Organism | Org cfu/5mL blood | Antibiotic | [Antibiotic] mg/L blood | Flip time in Days | Fastest method |
|---|---|---|---|---|---|---|
| Control | S. aureus | 750000 | Cefepime | 164 | 2.49 | Rescue |
| Bead rescue | S. aureus | 750000 | Cefepime | 164 | 0.39 | Rescue |
| Control | S. aureus | 125000 | Cefepime | 164 | 2.69 | Rescue |
| Bead rescue | S. aureus | 125000 | Cefepime | 164 | 0.46 | Rescue |
| Control | S. aureus | 125000 | None | 0 | 0.31 | BC |
| Bead rescue | S. aureus | 125000 | None | 0 | 0.39 | BC |
| Control | K. pneumoniae | 125000 | Cefepime | 164 | Neg | Rescue |
| Bead rescue | K. pneumoniae | 125000 | Cefepime | 164 | 0.44 | Rescue |
| Control | K. pneumoniae | 12500 | Cefepime | 164 | Neg | Rescue |
| Bead rescue | K. pneumoniae | 12500 | Cefepime | 164 | 0.49 | Rescue |
| Control | K. pneumoniae | 12500 | None | 0 | 0.26 | BC |
| Bead rescue | K. pneumoniae | 12500 | None | 0 | 0.34 | BC |
| Control | P. aeruginosa | 1500000 | Cefepime | 164 | 0.79 | Rescue |
| Bead rescue | P. aeruginosa | 1500000 | Cefepime | 164 | 0.32 | Rescue |
| Control | P. aeruginosa | 150000 | Cefepime | 164 | 0.83 | Rescue |
| Bead rescue | P. aeruginosa | 150000 | Cefepime | 164 | 0.41 | Rescue |
| Control | P. aeruginosa | 150000 | None | 0 | 0.37 | BC |
| Bead rescue | P. aeruginosa | 150000 | None | 0 | 0.4 | BC |
| Control | P. aeruginosa | 1250 | Cefepime | 164 | Neg | Rescue |
| Bead rescue | P. aeruginosa | 1250 | Cefepime | 164 | 0.58 | Rescue |
| Control | P. aeruginosa | 1250 | None | 0 | 0.61 | BC |
| Bead rescue | P. aeruginosa | 1250 | None | 0 | 0.79 | BC |
| Control | E. coli | 1250000 | Cefepime | 164 | Neg | Rescue |
| Bead rescue | E. coli | 1250000 | Cefepime | 164 | 0.38 | Rescue |
| Control | E. coli | 125000 | Cefepime | 164 | Neg | Rescue |
| Bead rescue | E. coli | 125000 | Cefepime | 164 | 0.47 | Rescue |
| Control | E. coli | 125000 | None | 0 | 0.29 | BC |
| Bead rescue | E. coli | 125000 | None | 0 | 0.35 | BC |
| Control | E. coli | 508000 | Cefotaxime | 100 | Neg | Rescue |
| Bead rescue | E. coli | 508000 | Cefotaxime | 100 | 0.31 | Rescue |
| Control | E. coli | 50800 | Cefotaxime | 100 | Neg | Rescue |
| Bead rescue | E. coli | 50800 | Cefotaxime | 100 | 0.39 | Rescue |
| Control | E. coli | 50800 | None | 0 | 0.31 | BC |
| Bead rescue | E. coli | 50800 | None | 0 | 0.37 | BC |
| Control | E. coli | 1500000 | Meropenem | 49 | Neg | Rescue |
| Bead rescue | E. coli | 1500000 | Meropenem | 49 | 0.43 | Rescue |
| Control | E. coli | 150000 | Meropenem | 49 | Neg | Rescue |
| Bead rescue | E. coli | 150000 | Meropenem | 49 | 0.46 | Rescue |
| Control | E. coli | 150000 | None | 0 | 0.27 | BC |
| Bead rescue | E. coli | 150000 | None | 0 | 0.31 | BC |
| Control | E. coli | 900 | Meropenem | 49 | Neg | Rescue |
| Bead rescue | E. coli | 900 | Meropenem | 49 | 0.56 | Rescue |
| Control | E. coli | 900 | None | 0 | 0.39 | BC |
| Bead rescue | E. coli | 900 | None | 0 | 0.5 | BC |
| Control | K. pneumoniae | 1250000 | Meropenem | 49 | Neg | Rescue |
| Bead rescue | K. pneumoniae | 1250000 | Meropenem | 49 | 0.38 | Rescue |
| Control | K. pneumoniae | 125000 | Meropenem | 49 | Neg | Rescue |
| Bead rescue | K. pneumoniae | 125000 | Meropenem | 49 | 0.43 | Rescue |
| Control | K. pneumoniae | 125000 | None | 0 | 0.25 | Rescue |
| Bead rescue | K. pneumoniae | 125000 | None | 0 | 0.24 | Rescue |
| Control | P. aeruginosa | 2000000 | Meropenem | 49 | 0.81 | Rescue |
| Bead rescue | P. aeruginosa | 2000000 | Meropenem | 49 | 0.46 | Rescue |
| Control | P. aeruginosa | 200000 | Meropenem | 49 | 0.9 | Rescue |
| Bead rescue | P. aeruginosa | 200000 | Meropenem | 49 | 0.54 | Rescue |
| Control | P. aeruginosa | 200000 | None | 0 | 0.33 | BC |
| Bead rescue | P. aeruginosa | 200000 | None | 0 | 0.34 | BC |
| Control | P. aeruginosa | 1250 | Meropenem | 49 | Neg | Rescue |
| Bead rescue | P. aeruginosa | 1250 | Meropenem | 49 | 0.81 | Rescue |
| Control | P. aeruginosa | 1250 | None | 0 | 0.59 | BC |
| Bead rescue | P. aeruginosa | 1250 | None | 0 | 0.7 | BC |

**Comments:**

In high peak serum concentrations of antibiotic-blood, FA+ blood culture bottles fail to recover organisms, as reported by Chung *et al.* 2019. This data shows that using the methods of the invention, organisms can be recovered and successfully grown from blood containing high peak serum concentrations of antibiotic. This experiment shows the methods of the invention are an improvement over the FA plus bottles as tested by Chung.

**References:**

Flayhart D, Borek AP, Wakefield T, Dick J, Carroll KC. Comparison of BACTEC PLUS blood culture media to BacT/Alert FA blood culture media for detection of bacterial pathogens in samples containing therapeutic levels of antibiotics. J Clin Microbiol. 2007 Mar;45(3):816-21. doi: 10.1128/JCM.02064-06. Epub 2006 Dec 13. PMID: 17166960; PMCID: PMC1829095.

Chung Y, Kim IH, Han M, Kim HS, Kim HS, Song W, Kim JS. A comparative evaluation of BACT/ALERT FA PLUS and FN PLUS blood culture bottles and BD BACTEC Plus Aerobic and Anaerobic blood culture bottles for anti-microbial neutralization. Eur J Clin Microbiol Infect Dis. 2019 Dec;38(12):2229-2233. doi: 10.1007/s10096-019-03663-3. Epub 2019 Aug 2. PMID: 31375943.

## Claims

1. A method of recovering viable microorganisms from a sample comprising microorganism cells and an antimicrobial agent, the method comprising:

    a) incubating the sample with coated particles to form particle-microorganism complexes; and
    b) separating the particle-microorganism complexes from the antimicrobial agent; thereby recovering viable microorganisms from the sample; and
    c) incubating and/or culturing the viable microorganisms recovered in step b),

    wherein the sample is from a subject who has been treated with the antimicrobial agent, wherein the microorganisms in the sample are or are thought to be susceptible to the antimicrobial agent and wherein the sample comprises a bodily fluid.

2. The method of claim 1 further comprising detecting and/or characterising the recovered viable microorganisms.

3. The method of claim 1 or claim 2, wherein the method is a method of detecting the absence or presence of a viable microorganism in a sample comprising an antimicrobial agent and suspected of containing a microorganism, the method further comprising:
    d) detecting the absence or presence of the recovered microorganisms, wherein the sample is from a subject who has been treated with the antimicrobial agent, and wherein the microorganisms in the sample are or are thought to be susceptible to the antimicrobial agent.

4. The method of any one of claims 1 to 3, wherein the sample also comprises non-microorganism cells and step b) separates the particle-microorganism complexes from the antimicrobial agent and the non-microorganism cells; optionally wherein the non-microorganism cells comprise blood cells.

5. The method of any one of claims 1 to 4, wherein step b) is performed in the absence of a detergent.

6. The method of any one of claims 1 to 4, wherein step b) is performed in the presence of sodium polyanethol sulfonate and/or a reagent that selectively lyses non-microorganism cells in the sample whilst retaining intact microorganisms present in the sample.

7. The method of any one of claims 1 to 6, wherein step b) comprises washing the separated particle-microorganism complexes; optionally wherein the separated particle-microorganism complexes are washed with a solution that does not contain detergent.

8. The method of any one of claims 1 to 7, wherein step b) comprises using a magnetic field or centrifugation.

**9.** The method of any one of claims 1 to 8, wherein the microorganism is a bacterium or fungus.

**10.** The method of any one of claims 1 to 9, wherein the antimicrobial agent is an antibiotic or antifungal.

**11.** The method of any one of claims 1 to 10, wherein the sample is selected from blood, cerebrospinal fluid (CSF), joint fluid, urine and broncheoalveolar lavage (BAL).

**12.** The method of any one of claims 1 to 11, wherein the sample comprises blood or a blood culture sample; optionally wherein the sample comprises whole blood.

**13.** The method of any one of claims 1 to 12, wherein the coated particles are magnetic.

**14.** The method of any one of claims 1 to 13, wherein the coated particles comprise a polymeric surface; optionally wherein the polymeric surface comprises a carbon-based polymer.

**15.** The method of any one of claims 1 to 14, wherein the coated particles comprise, on the outer surface, any one or more of:

    i) carboxylic acid groups
    ii) amino groups
    iii) hydrophobic groups; and
    iv) streptavidin.

**Patentansprüche**

**1.** Verfahren zur Gewinnung lebensfähiger Mikroorganismen aus einer Probe, die Mikroorganismuszellen und ein antimikrobielles Mittel umfasst, wobei das Verfahren umfasst:

    a) Inkubieren der Probe mit beschichteten Partikeln, um Partikel-Mikroorganismen-Komplexe zu bilden; und
    b) Abtrennen der Partikel-Mikroorganismen-Komplexe von dem antimikrobiellen Mittel, wodurch lebensfähige Mikroorganismen aus der Probe gewonnen werden; und
    c) Inkubieren und/oder Kultivieren der in Schritt b) gewonnenen lebensfähigen Mikroorganismen,

wobei die Probe von einer Person stammt, die mit dem antimikrobiellen Mittel behandelt wurde, wobei die Mikroorganismen in der Probe für das antimikrobielle Mittel empfindlich sind oder vermutlich empfindlich sind, und wobei die Probe eine Körperflüssigkeit umfasst.

**2.** Verfahren nach Anspruch 1, das ferner den Nachweis und/oder die Charakterisierung der gewonnenen lebensfähigen Mikroorganismen umfasst.

**3.** Verfahren nach Anspruch 1 oder Anspruch 2, wobei das Verfahren ein Verfahren zum Nachweis des Fehlens oder Vorhandenseins eines lebensfähigen Mikroorganismus in einer Probe ist, die ein antimikrobielles Mittel enthält und bei der der Verdacht besteht, dass sie einen Mikroorganismus enthält, wobei das Verfahren ferner umfasst:
d) Nachweis der Abwesenheit oder Anwesenheit der gewonnenen Mikroorganismen,
wobei die Probe von einer Person stammt, die mit dem antimikrobiellen Mittel behandelt wurde, und wobei die Mikroorganismen in der Probe für das antimikrobielle Mittel empfindlich sind oder vermutlich empfindlich sind.

**4.** Verfahren nach einem der Ansprüche 1 bis 3, bei dem die Probe auch Nicht-Mikroorganismus-Zellen umfasst und Schritt b) die Partikel-Mikroorganismen-Komplexe von dem antimikrobiellen Mittel und den Nicht-Mikroorganismus-Zellen trennt; optional, wobei die Nicht-Mikroorganismus-Zellen Blutzellen umfassen.

**5.** Verfahren nach einem der Ansprüche 1 bis 4, bei dem Schritt b) in Abwesenheit eines Detergens durchgeführt wird.

**6.** Verfahren nach einem der Ansprüche 1 bis 4, bei dem Schritt b) in Gegenwart von Natriumpolyanetholsulfonat und/oder einem Reagenz durchgeführt wird, das selektiv Nicht-Mikroorganismen-Zellen in der Probe lysiert, während die in der Probe vorhandenen Mikroorganismen unversehrt erhalten bleiben.

**7.** Verfahren nach einem der Ansprüche 1 bis 6, bei dem Schritt b) das Waschen der abgetrennten Partikel-Mikroorganismen-Komplexe umfasst; optional, wobei die abgetrennten Partikel-Mikroorganismen-Komplexe mit einer Lösung gewaschen werden, die kein Detergens enthält.

**8.** Verfahren nach einem der Ansprüche 1 bis 7, bei dem Schritt b) die Verwendung eines Magnetfeldes oder von Zentrifugation umfasst.

**9.** Verfahren nach einem der Ansprüche 1 bis 8, bei dem der Mikroorganismus ein Bakterium oder ein Pilz ist.

**10.** Verfahren nach einem der Ansprüche 1 bis 9, bei dem das antimikrobielle Mittel ein Antibiotikum oder ein Antimykotikum ist.

**11.** Verfahren nach einem der Ansprüche 1 bis 10, bei dem die Probe aus Blut, Zerebrospinalflüssigkeit (CSF), Gelenkflüssigkeit, Urin und broncheoalveolärer Lavage (BAL) ausgewählt wird.

**12.** Verfahren nach einem der Ansprüche 1 bis 11, bei dem die Probe Blut oder eine Blutkulturprobe umfasst; optional, bei dem die Probe Vollblut umfasst.

**13.** Verfahren nach einem der Ansprüche 1 bis 12, bei dem die beschichteten Partikel magnetisch sind.

**14.** Verfahren nach einem der Ansprüche 1 bis 13, bei dem die beschichteten Partikel eine Polymeroberfläche umfassen; optional, bei dem die Polymeroberfläche ein Polymer auf Kohlenstoffbasis umfasst.

**15.** Verfahren nach einem der Ansprüche 1 bis 14, bei dem die beschichteten Teilchen auf der äußeren Oberfläche eines oder mehrere von

    i) Carboxylsäuregruppen,
    ii) Aminogruppen,
    iii) hydrophoben Gruppen, und
    iv) Streptavidin

umfassen.

**Revendications**

**1.** Procédé de récupération de micro-organismes viables à partir d'un échantillon comprenant des cellules de micro-organisme et un agent antimicrobien, le procédé comprenant les étapes consistant à :

    a) incuber l'échantillon avec des particules enrobées pour former des complexes particules/micro-organismes ; et
    b) séparer les complexes particules/micro-organismes de l'agent antimicrobien ; récupérer ainsi les micro-organismes viables à partir de l'échantillon ; et
    c) incuber et/ou cultiver les micro-organismes viables récupérés à l'étape b),

dans lequel l'échantillon provient d'un sujet qui a été traité avec l'agent antimicrobien, dans lequel les micro-organismes dans l'échantillon sont ou sont supposés être sensibles à l'agent antimicrobien, et dans lequel l'échantillon comprend un fluide corporel.

**2.** Procédé selon la revendication 1, comprenant en outre la détection et/ou la caractérisation des micro-organismes viables récupérés.

**3.** Procédé selon la revendication 1 ou la revendication 2, dans lequel le procédé est un procédé de détection de l'absence ou de la présence d'un micro-organisme viable dans un échantillon comprenant un agent antimicrobien et suspecté de contenir un micro-organisme, le procédé comprenant en outre les étapes consistant à :
d) détecter l'absence ou la présence des micro-organismes récupérés, dans lequel l'échantillon provient d'un sujet qui a été traité avec l'agent antimicrobien, et dans lequel les micro-organismes dans l'échantillon sont ou sont supposés être sensibles à l'agent antimicrobien.

4. Procédé selon l'une quelconque des revendications 1 à 3, dans lequel l'échantillon comprend également des cellules non de micro-organisme et l'étape b) sépare les complexes particules/micro-organismes de l'agent antimicrobien et des cellules non de micro-organisme ; facultativement dans lequel les cellules non de micro-organisme comprennent des cellules sanguines.

5. Procédé selon l'une quelconque des revendications 1 à 4, dans lequel l'étape b) est réalisée en l'absence d'un détergent.

6. Procédé selon l'une quelconque des revendications 1 à 4, dans lequel l'étape b) est réalisée en présence de polyanéthol sulfonate de sodium et/ou d'un réactif qui lyse sélectivement les cellules non de micro-organisme dans l'échantillon tout en conservant les micro-organismes intacts présents dans l'échantillon.

7. Procédé selon l'une quelconque des revendications 1 à 6, dans lequel l'étape b) comprend le lavage des complexes particules/micro-organismes séparés ; facultativement dans lequel les complexes particules/micro-organismes séparés sont lavés avec une solution qui ne contient pas de détergent.

8. Procédé selon l'une quelconque des revendications 1 à 7, dans lequel l'étape b) comprend l'utilisation d'un champ magnétique ou d'une centrifugation.

9. Procédé selon l'une quelconque des revendications 1 à 8, dans lequel le micro-organisme est une bactérie ou un champignon.

10. Procédé selon l'une quelconque des revendications 1 à 9, dans lequel l'agent antimicrobien est un antibiotique ou un antifongique.

11. Procédé selon l'une quelconque des revendications 1 à 10, dans lequel l'échantillon est choisi parmi le sang, le liquide céphalo-rachidien (LCR), le liquide articulaire, l'urine et le lavage broncho-alvéolaire (BAL).

12. Procédé selon l'une quelconque des revendications 1 à 11, dans lequel l'échantillon comprend du sang ou un échantillon de culture sanguine ; facultativement dans lequel l'échantillon comprend du sang total.

13. Procédé selon l'une quelconque des revendications 1 à 12, dans lequel les particules enrobées sont magnétiques.

14. Procédé selon l'une quelconque des revendications 1 à 13, dans lequel les particules enrobées comprennent une surface polymère ; facultativement dans lequel la surface polymère comprend un polymère à base de carbone.

15. Procédé selon l'une quelconque des revendications 1 à 14, dans lequel les particules enrobées comprennent, sur la surface extérieure, un ou plusieurs parmi :

      i) des groupes acide carboxylique ;
      ii) des groupes amino ;
      iii) des groupes hydrophobes ; et
      iv) de la streptavidine.

Fig. 1

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009007719 A
- WO 2011130584 A
- WO 2010119270 A
- WO 2011070507 A
- WO 2017182775 A
- WO 03102184 A
- WO 0153525 A
- WO 2018044966 A
- EP 1118676 A
- CN 109929763

- CN 109741896
- WO 2006123154 A
- US 6261846 B, Barringer
- US 6410276 B
- WO 9006995 A
- US 4437975 A
- WO 2004067726 A
- WO 2018189502 A
- WO 2013103744 A
- WO 2016005768 A

**Non-patent literature cited in the description**

- **SCERBO et al.** *Surg Infect (Larchmt),* June 2016, vol. 17 (3), 294-302
- **SINHA et al.** *Clin Microbiol Rev.,* 28 February 2018, vol. 31 (2), e00089-17
- **SCHEER et al.** *Clin Microbiol Infect.,* March 2019, vol. 25 (3), 326-331
- **FLAYHART et al.** *J. Clin. Microbiol.,* 2007, 816-821
- **CHUNG et al.** *Eur J Clin Microbiol Infect Dis.,* 2019, vol. 38 (12), 2229-2233
- **CARTWRIGHT et al.** *EBioMedicine,* 2016, vol. 9, 217-227
- **ZWEITZIG et al.** Characterization of a novel DNA polymerase activity assay enabling sensitive, quantitative and universal detection of viable microbes. *Nucleic Acids Research,* 2012, vol. 40, 14, , e109, , 1-12
- **FAHY et al.** Rolling circle replication. *Transcription Mediated Amplification (TMA), strand displacement amplification (SDA) Clinical Chemistry,* 1991, vol. 45, 777-784
- **HOLLAND et al.** Detection of specific polymerase chain reaction product by utilising the 5'-3' exonuclease activity of Thermus aquaticus DNA polymerase. *Proc. Natl. Acad. Sci. USA,* 1991, vol. 88, 7276-7280
- **GELMINI et al.** Quantitative polymerase chain reaction-based homogeneous assay with flurogenic probes to measure C-Erb-2 oncogene amplification. *Clin. Chem.,* 1997, vol. 43, 752-758

- **LIVAK et al.** Towards fully automated genome wide polymorphism screening. *Nat. Genet.,* vol. 9, 341-342
- **TYAGI ; KRAMER.** Molecular beacons - probes that fluoresce upon hybridization. *Nat. Biotechnol.,* 1996, vol. 14, 303-308
- **TYAGI et al.** Multicolor molecular beacons for allele discrimination. *Nat. Biotechnol.,* 1998, vol. 16, 49-53
- **WHITCOMBE et al.** *Nature Biotechnology,* 01 August 1999, vol. 17, 804-807
- **TODD et al.** *Clinical Chemistry,* 2000, vol. 46 (5), 625-630
- **ZWEITZIG et al.** Characterization of a novel DNA polymerase activity assay enabling sensitive, quantitative and universal detection of viable microbes. *Nucleic Acids Research,* 2012, vol. 40, 14, , e109, , 1-12
- **FLAYHART D ; BOREK AP ; WAKEFIELD T ; DICK J ; CARROLL KC.** Comparison of BACTEC PLUS blood culture media to BacT/Alert FA blood culture media for detection of bacterial pathogens in samples containing therapeutic levels of antibiotics. *J Clin Microbiol.,* 13 December 2006, vol. 45 (3), 816-21
- **CHUNG Y ; KIM IH ; HAN M ; KIM HS ; KIM HS ; SONG W ; KIM JS.** A comparative evaluation of BACT/ALERT FA PLUS and FN PLUS blood culture bottles and BD BACTEC Plus Aerobic and Anaerobic blood culture bottles for antimicrobial neutralization. *Eur J Clin Microbiol Infect Dis.,* 02 August 2019, vol. 38 (12), 2229-2233